# EUROPÄISCHE PATENTSCHRIFT

(11) **EP 2 285 809 B1**
(45) Veröffentlichungstag und Bekanntmachung des Hinweises auf die Patenterteilung: **27.03.2013**
(21) Anmeldenummer: 09741818.0
(22) Anmeldetag: 24.04.2009
(51) Int. Cl.: C07D 487/04, A61K 31/519, A61P 11/00

(54) **1,4-DIARYL-PYRIMIDOPYRIDAZIN-2,5-DIONE UND IHRE VERWENDUNG**
1,4-DIARYL-PYRIMIDOPYRIDAZINE-2,5-DIONES AND THEIR USE
1,4-DIARYL-PYRIMIDOPYRIDAZIN-2,5-DIONES ET LEUR UTILISATION

(30) Priorität: 07.05.2008 DE 102008022521
(43) Veröffentlichungstag der Anmeldung: 23.02.2011
(73) Patentinhaber: Bayer Intellectual Property GmbH, 40789 Monheim (DE)
(72) Erfinder: VON NUSSBAUM, Franz, 40219 Düsseldorf (DE); KARTHAUS, Dagmar, 42697 Solingen (DE); DELBECK, Martina, 42579 Heiligenhaus (DE); LI, Volkhart Min-Jian, 42553 Velbert (DE); MEIBOM, Daniel, 51373 Leverkusen (DE); LUSTIG, Klemens, 42113 Wuppertal (DE)
(74) Vertreter: BIP Patents
(86) Internationale Anmeldenummer: PCT/EP2009/003006
(87) Internationale Veröffentlichungsnummer: WO 2009/135599

(56) Entgegenhaltungen:
- WO-A-2004/024700
- WO-A-2005/082863
- WO-A-2005/082864

## Beschreibung

Die vorliegende Anmeldung betrifft neue 1,4-Diaryl-pyrimido[4,5-d]pyridazin-2,5-dion-Derivate, Verfahren zu ihrer Herstellung, ihre Verwendung allein oder in Kombinationen zur Behandlung und/oder Prävention von Krankheiten sowie ihre Verwendung zur Herstellung von Arzneimitteln zur Behandlung und/oder Prävention von Krankheiten, insbesondere zur Behandlung und/oder Prävention von Erkrankungen der Lunge und des Herz-Kreislauf-Systems.

Die Humane Leukozyten-Elastase (HLE, EC 3.4.21.37), auch Humane Neutrophile Elastase (HNE, hNE) genannt, gehört zur Familie der Serinproteasen. Das proteolytische Enzym findet sich in den azurophilen Granula der polymorphkernigen Leukozyten (engl. *polymorphonuclear leukocytes, PMN leukocytes*). Die intrazelluläre Elastase nimmt eine wichtige Funktion in der Pathogenabwehr wahr, indem über Phagozytose aufgenommene Fremdpartikel abgebaut werden. Aktivierte neutrophile Zellen setzen die HNE aus den Granula in den Extrazellulärraum frei (extrazelluläre HNE), wobei ein Teil der freigesetzten HNE an der Außenseite der neutrophilen Zellmembran verbleibt (membranständige HNE). Das hochaktive Enzym ist in der Lage, eine Vielzahl von Bindegewebsproteinen abzubauen, z.B. die Proteine Elastin, Kollagen und Fibronektin. Elastin kommt in hohen Konzentrationen in allen Gewebetypen vor, die eine hohe Elastizität zeigen, z.B. in der Lunge und in Arterien. Bei einer Vielzahl von pathologischen Prozessen (z.B. Gewebeverletzungen) spielt die HNE eine Rolle beim Gewebeab- und -umbau (engl. *tissue remodeling*). Darüber hinaus ist die HNE ein wichtiger Modulator bei entzündlichen Prozessen. HNE induziert beispielsweise eine erhöhte Genexpression von Interleukin-8 (IL-8).

Es wird daher angenommen, dass die HNE bei vielen Erkrankungen, Verletzungen und pathologischen Veränderungen, deren Entstehung und/oder Progression mit einem entzündlichen Geschehen und/oder einem proliferativen und hypertrophen Gewebe- und Gefäßumbau in Zusammenhang steht, eine wichtige Rolle spielt. Dies können insbesondere Erkrankungen und/oder Schädigungen der Lunge oder des Herz-Kreislauf-Systems sein, oder es kann sich hierbei um eine Sepsis, um Krebs-Erkrankungen oder um andere entzündliche Erkrankungen handeln.

In diesem Zusammenhang zu nennende Erkrankungen und Schädigungen der Lunge sind insbesondere die chronisch-obstruktive Lungenerkrankung (engl. *chronic obstructive pulmonary disease,* COPD), das akute Atemwegssyndrom (engl. *acute respiratory distress syndrome,* ARDS), die zystische Fibrose (engl. *cystic fibrosis,* CF; auch Mukoviszidose genannt), das Lungenemphysem (engl. *lung emphysema*) und die akute Lungenschädigung (engl. *acute lung injury,* ALI). Erkrankungen und Schädigungen des Herz-Kreislauf-Systems, in denen die HNE involviert ist, sind zum Beispiel Gewebeveränderungen bei einer Herzinsuffizienz und Reperfusionsschäden nach einem Myokardinfarkt (engl. *acute myocardial infarct,* AMI), der kardiogene Schock, das akute Koronarsyndrom (engl. *acute coronary syndrome,* ACS) sowie Aneurysmen. Erkrankungen in Zusammenhang mit einer Sepsis sind beispielsweise eine systemische entzündliche Reaktion (engl. *systemic inflammatory response syndrome,* SIRS), die schwere Sepsis, der septische Schock und das multiple Organversagen (engl. *multi-organ failure,* MOF; *multi-organ dysfunction,* MODS) sowie die intravaskuläre Gerinnung (engl. *disseminated intravascular coagulation,* DIC). Beispiele für einen Gewebeab- und -umbau bei Krebsprozessen sind das Einwandern von Krebszellen in das gesunde Gewebe (Metastasenbildung) und die Neuausbildung von versorgenden Blutgefäßen (Neo-Angiogenese). Andere entzündliche Krankheiten, bei denen die HNE eine Rolle spielt, sind rheumatoide Erkrankungen, zum Beispiel die rheumatoide Arthritis, chronische Darmentzündungen (engl. *inflammatory bowel disease,* IBD; Morbus Crohn, engl. *Crohn's disease,* CD; Colitis ulcerosa, engl. *ulcerative colitis,* UC) und die Arteriosklerose.

Im Allgemeinen geht man davon aus, dass Elastase-vermittelten pathologischen Prozessen ein verschobenes Gleichgewicht zugrunde liegt zwischen der freien Elastase und dem körpereigenen Elastase-Inhibitorprotein (hauptsächlich das alpha-1-Antitrypsin, AAT) [Neutrophils and protease/antiprotease imbalance, Stockley, Am. J. Respir. Crit. Care Med. 160, 49-52 (1999)]. AAT liegt im Plasma im hohen Überschuss vor und neutralisiert so sehr schnell freie HNE. In verschiedenen pathologischen Prozessen ist die Konzentration an freier Elastase erhöht, so dass lokal die Balance zwischen Protease und Protease-Inhibitor zu Gunsten der Protease verschoben ist. Zudem ist die membranständige Elastase der aktivierten PMN-Zellen vor einer Inhibition durch AAT weitestgehend geschützt. Gleiches gilt für die freie Elastase, die sich in einem erschwert zugänglichen Mikrokompartiment zwischen der neutrophilen Zelle und der angrenzenden Gewebezelle (z.B. Endothelzelle) befindet. Zusätzlich herrschen stark oxidierende Bedingungen im Umfeld von aktivierten Leukozyten (engl. *oxidative burst*), wodurch AAT oxidiert wird und in der inhibitorischen Wirkung mehrere Größenordnungen verliert.

Neue Elastase-inhibierende Wirkstoffe (exogen verabreichte Inhibitoren der HNE) sollten demnach ein niedriges Molekulargewicht aufweisen, um in der Lage zu sein, auch die membranständige HNE und die im geschützten Mikrokompartiment befindliche HNE (s.o.) zu erreichen und zu inhibieren. Hierzu ist auch eine gute Stabilität der Substanzen *in vivo* notwendig (geringe *in vivo*-Clearance). Außerdem sollten diese Verbindungen stabil sein unter oxidativen Bedingungen, um im Krankheitsgeschehen nicht an inhibitorischer Potenz zu verlieren.

Die Pulmonale Arterielle Hypertonie (PAH) ist eine progrediente Lungenerkrankung, die unbehandelt durchschnittlich innerhalb von 2.8 Jahren nach Diagnosestellung zum Tode führt. Eine zunehmende Verengung der Lungenstrombahn führt zu einer Mehrbelastung des rechten Herzens, die bis zum Rechtsherzversagen gehen kann. Definitionsgemäß liegt bei einer chronischen pulmonalen Hypertonie ein pulmonal-arterieller Mitteldruck (mPAP) von > 25 mmHg in Ruhe oder > 30 mmHg unter Belastung vor (Normalwert < 20 mmHg). Die Pathophysiologie der pulmonal-arteriellen Hypertonie ist gekennzeichnet durch Vasokonstriktion und Remodeling der Pulmonalgefäße. Bei der chronischen PAH kommt es zu einer Neomuskularisierung primär nicht muskularisierter Lungengefäße, und die Gefäßmuskulatur der bereits muskularisierten Gefäße nimmt an Umfang zu. Durch diese zunehmende Obliteration der Lungenstrombahn kommt es zu einer progredienten Belastung des rechten Herzens, die zu einer verminderten Auswurfleistung des rechten Herzens führt und letztlich in einem Rechtsherzversagen endet (M. Humbert et al., J. Am. Coll. Cardiol. 2004, 43, 13S-24S). Mit einer Prävalenz von 1-2 pro einer Million handelt es sich bei PAH um eine äußerst seltene Erkrankung. Das mittlere Alter der Patienten wurde auf 36 Jahre geschätzt, nur 10% der Patenten waren über 60 Jahre alt. Deutlich mehr Frauen als Männer sind betroffen (G.E. D'Alonzo et al., Ann. Intern. Med. 1991, 115, 343-349).

Trotz aller Fortschritte in der Therapie der pulmonal-arteriellen Hypertonie gibt es bisher keine Aussicht auf Heilung dieser schwerwiegenden Erkrankung. Auf dem Markt befindliche Standardtherapien (z.B. Prostacyclin-Analoga, Endothelinrezeptor-Antagonisten, Phosphodiesterase-Inhibitoren) sind in der Lage, die Lebensqualität, die körperliche Belastbarkeit und die Prognose der Patienten zu verbessern. Hierbei handelt es sich um primär hämodynamische Therapieprinzipien, die den Gefäßtonus beeinflussen, jedoch keinen direkten Einfluss auf die pathogenen Remodeling-Prozesse haben. Darüber hinaus ist die Anwendbarkeit dieser Medikamente durch die z.T. gravierenden Nebenwirkungen und/oder aufwendigen Applikationsformen eingeschränkt. Der Zeitraum, über den unter einer spezifischen Monotherapie die klinische Situation der Patienten verbessert oder stabilisiert werden kann, ist begrenzt (z.B. aufgrund einer Toleranzentwicklung). Es erfolgt schließlich eine Therapieeskalation und somit eine Kombinationstherapie, bei der mehrere Medikamente gleichzeitig gegeben werden müssen.

Neue Kombinationstherapien sind eine der aussichtsreichsten zukünftigen Therapieoptionen zur Behandlung der pulmonalen arteriellen Hypertonie. In diesem Zusammenhang ist die Erkundung neuer pharmakologischer Mechanismen zur Behandlung der PAH von besonderem Interesse (Ghofrani et al., Herz 2005, 30, 296-302; E.B. Rosenzweig, Expert Opin. Emerging Drugs 2006, 11, 609-619; T. Ito et al., Curr. Med. Chem. 2007, 14, 719-733). Vor allem solche Therapieoptionen, die direkt in das Remodeling-Geschehen eingreifen (anti-Remodeling-Mechanismen, reverse-Remodeling-Mechanismen), könnten Grundlage einer mehr ursächlichen Behandlung sein und somit einen großen Vorteil für die Patienten bringen. Neue Therapien sollten hierbei mit den bekannten kombinierbar sein. Um in einer solchen Kombinationstherapie das Risiko für störende Wechselwirkungen zwischen den Medikamenten zu minimieren, sollten diese neuen Wirkstoffe metabolisierende P450 CYP-Enzyme nicht oder in nur sehr geringem Maße hemmen.

Heute geht man davon aus, dass die Elastase beim pathologischen Remodeling eine zentrale Rolle spielt. In Tiermodellen und in Patienten mit einem erhöhten arteriellen Lungenblutdruck (pulmonale arterielle Hypertension) konnte eine Fragmentierung des Bindegewebes (interne elastische Lamina) festgestellt werden [Rabinovitch et al., Lab. Invest. 55, 632-653 (1986)], und in Tiermodellen für die pulmonale arterielle Hypertension (hypoxisches Ratten- und Mausmodell, Monocrotalin-Rattenmodell) konnte eine erhöhte Elastase-Aktivität gezeigt werden, die mit der Fragmentierung des Bindegewebes einherging [Todorovich-Hunter et al., Am. Rev. Respir. Dis. 146, 213-223 (1992)]. Man vermutet, dass der zu beobachtende Gewebeumbau im Verlauf des Krankheitsgeschehens der pulmonalen arteriellen Hypertonie durch ein Elastase-vermitteltes Freisetzen von bindegewebsständigen Wachstumsfaktoren induziert wird, z.B. des basischen Fibroblasten-Wachstumsfaktors (engl. *basic fibroblast growth factor,* bFGF) [Rabinovitch, Am. J. Physiol. 277, L5-L12 (1999)]. Im hypoxischen Mausmodell für die pulmonale arterielle Hypertension konnte ein positiver Effekt mit einem überexprimierten Elastase-Inhibitorprotein gezeigt werden [Zaidi et al., Circulation 105, 516-521 (2002)]. Im Monocrotalin-Rattenmodell für die pulmonale arterielle Hypertension konnte eine positive Wirkung mit synthetischen, niedermolekularen Elastase-Inhibitoren gezeigt werden; hierbei war auch ein günstiger Effekt beim Gewebeumbau zu verzeichnen [Cowan et al., Nature Med. 6, 698-702 (2000)]. Alle bisher bekannten niedermolekularen Elastase-Inhibitoren sind jedoch wenig selektiv, chemisch reaktiv und/oder nur begrenzt oral verfügbar, was eine klinische Entwicklung eines oralen Elastase-Inhibitors in diesen Indikationen bisher vereitelte.

Der Begriff "pulmonale arterielle Hypertonie" umfasst bestimmte Formen der pulmonalen Hypertonie, wie sie z.B. von der Weltgesundheitsorganisation (WHO) festgelegt worden sind (Clinical Classification of Pulmonary Hypertension, Venedig 2003; G. Simonneau et al., J. Am. Coll. Cardiol. 2004, 43, 5S-12S).

Die pulmonale arterielle Hypertonie beinhaltet nach dieser Einteilung die Idiopathische Pulmonale Arterielle Hypertonie (IPAH, früher auch als primäre pulmonale Hypertonie, PPH, bezeichnet), die Familiär bedingte Pulmonale Arterielle Hypertonie (FPAH), die persistierende pulmonale Hypertonie der Neugeborenen sowie die Assoziierte Pulmonal-Arterielle Hypertonie (APAH), welche assoziiert ist mit Kollagenosen, kongenitalen systemisch-pulmonalen Shuntvitien, portaler Hypertension, HIV-Infektionen, der Einnahme bestimmter Drogen und Medikamente (z.B. von Appetitzüglern), mit Erkrankungen mit einer signifikanten venösen/kapillären Beteiligung wie der pulmonal-venookklusiven Erkrankung und der pulmonal-kapillären Hämangiomatose, oder mit anderen Erkrankungen wie Schilddrüsenerkrankungen, Glykogenspeicherkrankheiten, Morbus Gaucher, hereditäre Teleangiektasie, Hämoglobinopathien, myeloproliferative Erkrankungen und Splenektomie.

Andere Formen der pulmonalen Hypertonie umfassen beispielsweise die mit Linksherzerkrankungen assoziierte pulmonale Hypertonie, z.B. bei ventrikulären oder valvulären Erkrankungen, die mit Erkrankungen der Atemwege und/oder der Lunge assoziierte pulmonale Hypertonie, z.B. bei chronisch-obstruktiver Lungenerkrankung, interstitieller Lungenkrankheit oder Lungenfibrose, die auf chronische thrombotische und/oder embolische Erkrankungen zurückzuführende pulmonale Hypertonie, z.B. bei thromboembolischer Obstruktion von Lungenarterien, sowie die durch allgemein entzündliche Krankheitsprozesse oder durch spezielle Ursachen hervorgerufene pulmonale Hypertonie (z.B. bei Schistosomiasis, Sarkoidose und Tumorerkrankungen).

Die chronisch-obstruktive Lungenerkrankung (COPD) ist eine langsam fortschreitende Lungenerkrankung, die durch eine Behinderung der Atemströmung charakterisiert ist, welche durch ein Lungenemphysem und/oder eine chronische Bronchitis hervorgerufen wird. Die ersten Symptome der Erkrankung zeigen sich in der Regel ab dem vierten bis fünften Lebensjahrzehnt. In den darauffolgenden Lebensjahren verschlimmert sich häufig die Kurzatmigkeit und es manifestiert sich Husten, verbunden mit einem ausgiebigen und stellenweise eitrigen Auswurf und einer Stenose-Atmung bis hin zu einer Atemnot (Dyspnoe). COPD ist in erster Linie eine Krankheit von Rauchern: Rauchen ist verantwortlich für 90% aller COPD-Fälle und 80-90% aller COPD-Todesfälle. COPD ist ein großes medizinisches Problem und stellt weltweit die sechsthäufigste Todesursache dar. Von den über 45-jährigen Menschen sind ca. 4-6% betroffen.

Obwohl die Behinderung der Atemströmung nur partiell und zeitlich befristet sein kann, ist COPD nicht heilbar. Behandlungsziel ist folglich eine Verbesserung der Lebensqualität, die Linderung der Symptome, die Verhinderung akuter Verschlechterungen und die Verlangsamung der fortschreitenden Beeinträchtigung der Lungenfunktion. Bestehende Pharmakotherapien, die sich seit den letzten zwei bis drei Jahrzehnten kaum geändert haben, sind das Verwenden von Bronchodilatoren, um blockierte Atemwege zu öffnen, und in bestimmten Situationen Kortikosteroide, um die Entzündung der Lunge einzudämmen [P.J. Barnes, N. Engl. J. Med. 343, 269-280 (2000)]. Die chronische Entzündung der Lunge, hervorgerufen durch Zigarettenrauch oder andere Reizstoffe, ist die treibende Kraft der Krankheitsentwicklung. Der zugrunde liegende Mechanismus beinhaltet Immunzellen, die im Zuge der inflammatorischen Reaktion der Lunge verschiedene Chemokine ausschütten. Hierdurch werden neutrophile Zellen und im weiteren Verlauf alveolare Makrophagen zum Lungenbindegewebe und Lumen gelockt. Neutrophile Zellen sezernieren einen Protease-Cocktail, der hauptsächlich HNE und Proteinase 3 enthält. Hierdurch wird lokal die Protease/Antiprotease-Balance zu Gunsten der Proteasen verschoben, was u.a. zu einer unkontrollierten Elasase-Aktivität und in Folge hiervon zu einem überschießenden Abbau des Elastins der Alveolaren führt [J.E. Gadek et al., J. Clin. Invest. 68, 889-898 (1981); Z. Werb et al., J. Invest. Dermatol. 79, 154-159 (1982); A. Janoff, Am. Rev. Respir. Dis. 132, 417-433 (1985); P.J. Barnes, N. Engl. J. Med. 343, 269-280 (2000)]. Dieser Gewebeabbau verursacht einen Kollaps der Bronchien. Dies geht einher mit einer verminderten Elastizität der Lunge, was zu einer Behinderung der Atemströmung und beeinträchtigter Atmung führt. Darüber hinaus kann eine häufige und andauernde Entzündung der Lunge zu einem Remodeling der Bronchien und in der Folge zu einer Ausbildung von Läsionen führen. Solche Läsionen tragen zum Auftreten des chronischen Hustens bei, der eine chronische Bronchitis kennzeichnet.

Alpha-1-Antitrypsin (AAT) ist ein kleines körpereigenes Protein und stellt, wie oben erwähnt, den wichtigsten endogenen Elastase-Hemmer dar. Bei Patienten mit einer genetisch bedingten Defizienz dieses Proteins (AATD) ist die Protease/Antiprotease-Balance verschoben. Der Wirkradius und die Wirkdauer der HNE ist in AATD-Patienten entsprechend um einen Faktor 2.5 bzw. 6.5 erhöht [T.G. Liou und E.J. Campbell, Biochemistry 1995, 16171-16177]. AATD-Patienten haben ein erhöhtes Risiko, ein Lungenemphysem oder COPD zu entwickeln, und bei vielen AATD-Patienten ist eine Lungentransplantation indiziert.

Unter einer Bronchiektasie versteht man eine abnorme Ausweitung des Bronchialbaums. Zwei Formen können unterschieden werden: sackförmige, lokalisierte Bronchiektasen und generalisierte, zylindrische Bronchiektasen. Bronchiektasen können in angeborener Form auftreten, sind aber meist erworben und finden sich insbesondere bei Rauchern. Durch die Ausweitung ist die Entleerung des Bronchialsekrets erschwert, und durch das retinierte Bronchialsekret werden Infektionen begünstigt. Bronchiektasen finden sich oft auch bei angeborenen Schleimhauterkrankungen wie der Mukoviszidose mit abnormer Viskosität des Bronchialsekrets und beim Syndrom der ziliären Dyskinesie. Bei diesem Syndrom (Kartagener-Syndrom) sind die Zilienarchitektur und -funktion und dadurch die Sekretdrainage gestört. Andere Ursachen von Bronchiektasen können Obstruktionen proximal der Ektasie sein, z.B. durch Tumore oder Fremdkörper. Als ursächlich werden auch rezidivierende und persistierende Infekte angenommen, die die Bronchuswände schwächen. Ferner gibt es Bronchiektasien, die nicht eindeutig mit Infektionszuständen oder exogenen Noxen in Zusammenhang gebracht werden können (idiopathische Bronchiektasien).

Die Bronchiektasie ist charakterisiert durch eine Einwanderung von Neutrophilen in das Lungengewebe. Die Patienten zeigen ein starkes Ungleichgewicht zwischen Neutrophilenaktivität und schützenden Inhibitor-Proteinen, was zu einer Schädigung des Lungengewebes durch die von den Neutrophilen sezernierten Proteasen (hauptsächlich HNE) führt [Schaaf et al., Respiration 67, 52-59 (2000)].

Die Bronchiolitis obliterans ist eine in den Bronchioli angreifende Entzündung mit Epitheldestruktion und Bildung eines fibrinreichen Exsudats in den Bronchioli und den angrenzenden Alveolen. Durch Organisation des Exsudats entstehen Bindegewebspfröpfe, die aus den Bronchioli in die Alveolen hineinreichen. Die Erkrankung ist gekennzeichnet durch eine erhöhte Anzahl von Neutrophilen in den Atemwegen und ein Ungleichgewicht zwischen der freien Elastase und dem körpereigenen Elastase-Inhibitorprotein [Elssner et al., Transpl. Infect. Dis. 3, 168-176 (2001)]. Als Ursachen werden vorangegangene Infektionen sowie Medikamente diskutiert. Die Erkrankung kann auch im Rahmen einer Abstoßungsreaktion eines Transplantats vorkommen.

Die akute Lungenschädigung (ALI) sowie die ausgeprägtere Form hiervon, das akute Lungenversagen (ARDS), sind schwerwiegende Erkrankungen, die mit einer Mortalität von 50-60% einhergehen. Nach der Definition der North American-European Consensus Conference (NAECC) von 1994 sind ALI und ARDS definiert durch eine akute auslösende Erkrankung, bilaterale radiologisch sichtbare Infiltrate, einen PaO₂/FiO₂-Index von ≤ 300 mmHg (ALI) bzw. ≤ 200 mmHg (ARDS), einen pulmonal-kapillären Verschlussdruck von < 18 mmHg bzw. fehlende klinische Hinweise auf linksatriale Hypertension.

Der Entstehung einer akuten Lungenschädigung können sowohl pulmonale als auch extrapulmonale Erkrankungen vorausgehen. Als lungenspezifische prädisponierende Faktoren gelten Aspiration von Mageninhalt, Pneumonien, Rauchgasvergiftung, Lungenkontusion sowie ein Beinahe-Ertrinken. Vor allem Magensaftaspiration und Pneumonien werden häufig als Ausgangserkrankung für ALI/ARDS pulmonalen Ursprungs gesehen. Als indirekte Ereignisse kommen vor allem Polytrauma, Sepsis, mehrfache Bluttransfusionen, akute Pankreatitis und Verbrennungen vor. Die Inzidenz liegt bei 17.9 Fällen für ALI bzw. 13.5 Fällen für ARDS pro 100 000 Einwohner und Jahr [Luhr et al., Am. J. Respir. Crit. Care Med. 159, 1849-1861 (1999)].

Eine zentrale Rolle für die Entstehung dieser Erkrankungen stellen die massiven entzündlichen Veränderungen in der Lunge dar, die durch ein weitverzweigtes System von Mediatoren ausgelöst werden. Eine wichtige Rolle bei der Entwicklung der Lungenschädigung spielen auch die neutrophilen Granulozyten, deren Anzahl sich mit dem Andauern des entzündlichen Prozesses ständig erhöht [Chollet-Martin et al., Am. J. Respir. Crit. Care Med. 154, 594-601 (1996)]. Die Wirkung der Mediatoren bedingt eine Schädigung der alveolokapillären Membranen, hieraus resultiert eine Permeabilitätserhöhung der alveolär-kapillären Barriere. Durch die Permeabilitätserhöhung kann proteinreiche Flüssigkeit in die Alveolen und auch in das Interstitium eindringen; es bildet sich ein pulmonales Niederdrucködem aus. Charakteristisch für ALI/ARDS ist, dass es sich hierbei um ein nicht-kardiogen induziertes Ödem handelt. Die Ödemflüssigkeit enthält vor allem Fibrin, Erythrozyten, Leukozyten, hyaline Membranen und andere Proteine. Das proteinreiche Exsudat führt zusammen mit den Produkten von aktivierten Neutrophilen zu einer Dysfunktion des Surfactants. Die inflammatorischen Prozesse führen zur Schädigung und zum Verlust von Pneumozyten des Typs II, die Surfactant bilden, so dass ein Herabsinken der Surfactant-Produktion resultiert. Durch den Surfactant-Mangel erhöht sich die Oberflächenspannung in den Alveolen; Alveolen kollabieren, es bilden sich Atelektasen aus. Bei weiter bestehender Perfusion entsteht somit eine Ventilations-Perfusions-Störung, die in einer Erhöhung des pulmonalen Rechts-Links-Shunts mündet. Des Weiteren sinkt die Compliance herab, der alveoläre Totraum hingegen nimmt zu, denn es gibt auch Areale, die zwar belüftet, aber aufgrund einer pulmonalen Hypertension nicht mehr ausreichend perfundiert werden.

In der bronchoalveolaren Waschflüssigkeit von ARDS-Patienten (BALF) konnte eine erhöhte Elastase-Aktivität gemessen werden, die mit dem Schweregrad der Lungenschädigung einhergeht. In Tiermodellen, in denen die Lunge geschädigt wird (z.B. durch Gabe von LPS), kann dieser Effekt nachgestellt werden. Eine Behandlung mit Elastase-Hemmern (z.B. Sivelestat oder Elafin, s.u.) vermindert hier deutlich die Elastase-Aktivität in der BALF und verbessert die Lungenfunktion.

Zur Behandlung einer akuten Lungenschädigung, die mit SIRS assoziiert ist, ist ein Elastase-Hemmer in Japan und Südkorea zugelassen (Sivelestat, Elaspol^{®}). Die reversible, aber reaktive Verbindung besitzt nur eine relativ schwache Wirksamkeit gegenüber der HNE (Kᵢ 200 nM) und wirkt ebenfalls gegenüber der Elastase des Pankreas (IC₅₀ 5.6 µM). Der Wirkstoff wird intravenös verabreicht, eine orale Applikation ist nicht möglich.

Auch Elafin und strukturelle Analoga werden als therapeutisch nutzbare Elastase-Inhibitoren untersucht. Elafin ist ein körpereigenes kleines Protein, welches sowohl Elastase als auch Proteinase 3 hemmt. Aufgrund des proteinergen Charakters ist jedoch eine orale Verabreichung von Elafin nicht möglich.

Die Aufgabe der vorliegenden Erfindung liegt in der Bereitstellung neuer Substanzen, die als niedermolekulare, nicht-reaktive und selektive Inhibitoren der humanen neutrophilen Elastase (HNE) wirken und sich als solche zur Behandlung und/oder Prävention insbesondere von Erkrankungen der Lunge und des Herz-Kreislauf-Systems eignen.

In WO 2004/024700, WO 2004/024701, WO 2005/082863, WO 2005/082864 und WO 2008/003412 werden verschiedene 1,4-Diaryl-dihydropyrimidin-2-on-Derivate als HNE-Inhibitoren zur Behandlung von chronisch-obstruktiven Lungenerkrankungen, akutem Koronarsyndrom, Myokardinfarkt und Herzinsuffizienz offenbart. Di- und Multimere solcher Verbindungen zur Behandlung von Atemwegserkrankungen werden in WO 2006/082412, WO 2006/136857, WO 2007/042815 und WO 2008/030158 beansprucht. 4-Aryl-dihydropyrimidin-2-on-Derivate als Inhibitoren der Calciumkanal-Funktion zur Behandlung von Hypertonie werden in WO 2005/009392 beschrieben. In WO 2007/129060 und WO 2008/135537 werden Tetrahydropyrrolopyrimidindione und Multimere hiervon als HNE-Inhibitoren offenbart.

Es wurde nun überraschend gefunden, dass sich bestimmte 1,4-Diaryl-pyrimido[4,5-d]pyridazin-2,5-dion-Derivate in besonderem Maße für die Behandlung und/oder Prävention von Erkrankungen eignen. Diese im Folgenden beschriebenen Verbindungen sind niedermolekulare, nicht-reaktive und selektive Inhibitoren der humanen neutrophilen Elastase (HNE), die darüber hinaus vorteilhafte pharmakokinetische Eigenschaften bezüglich ihrer Bioverfügbarkeit, Halbwertszeit und/oder Proteinbindung aufweisen. Diese Substanzen stellen somit vielversprechende Ausgangspunkte für neue Arzneimittel zur Behandlung und/oder Prävention insbesondere von Erkrankungen der Lunge und des Herz-Kreislauf-Systems dar.

Gegenstand der vorliegenden Erfindung sind Verbindungen der allgemeinen Formel (I) 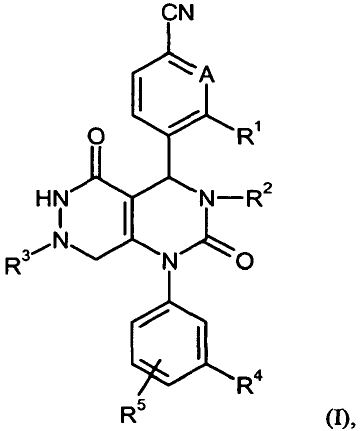 in welcher
- A: für CH oder N steht,
- R¹: für Wasserstoff, Halogen, Cyano, Nitro, (C₁-C₆)-Alkyl, Difluormethyl, Trifluormethyl, (C₁-C₆)-Alkoxy, Difluormethoxy, Trifluormethoxy, Amino, Mono- oder Di-(C₁-C₆)-alkylamino
oder
für eine Gruppe der Formel -NH-C(=O)-R⁶, -NH-C(=O)-NHR⁶, -NH-SO₂-R⁷ oder -S(O)ₙ-R⁸ steht, worin
R⁶ Wasserstoff oder (C₁-C₆)-Alkyl bedeutet,
R⁷ (C₁-C₆)-Alkyl bedeutet,
R⁸ (C₁-C₆)-Alkyl, das mit Hydroxy, (C₁-C₄)-Alkoxy, Amino, Mono- oder Di-(C₁-C₄)-alkylamino, Hydroxycarbonyl, Aminocarbonyl, (C₃-C₆)-Cycloalkyl oder Phenyl substituiert sein kann, oder (C₂-C₆)-Alkenyl, (C₃-C₆)-Cycloalkyl oder Phenyl bedeutet,
wobei die genannten (C₃-C₆)-Cycloalkyl-Gruppen bis zu zweifach, gleich oder verschieden, mit (C₁-C₄)-Alkyl, Hydroxy und (C₁-C₄)-Alkoxy substituiert sein können
und
die genannten Phenyl-Gruppen bis zu zweifach, gleich oder verschieden, mit Fluor, Chlor, Cyano, (C₁-C₄)-Alkyl, Difluormethyl, Trifluormethyl, (C₁-C₄)-Alkoxy, Difluormethoxy und Trifluormethoxy substituiert sein können, und
n die Zahl 0, 1 oder 2 bedeutet,
- R²: für Wasserstoff, für (C₁-C₆)-Alkyl oder (C₂-C₆)-Alkenyl, welche jeweils bis zu dreifach mit Fluor substituiert sein können, oder für Phenyl, Pyridyl oder Pyrimidinyl steht,
wobei Phenyl, Pyridyl und Pyrimidinyl ihrerseits bis zu zweifach, gleich oder verschieden, mit Fluor, Chlor, Cyano, (C₁-C₄)-Alkyl, Trifluormethyl, (C₁-C₄)-Alkoxy und Trifluormethoxy substituiert sein können,
oder
- R²: für eine Gruppe der Formel -C(=O)-O-R⁹, -L¹-C(=O)-O-R¹⁰, -L²-C(=O)-NR¹¹R¹², -L²-SO₂-NR¹¹R¹², -L²-C(=O)-NR¹³-NR¹¹R¹² oder -L²-SO₂-R¹⁴ steht, worin
L¹ (C₁-C₆)-Alkandiyl bedeutet,
L² eine Bindung oder (C₁-C₆)-Alkandiyl bedeutet,
R⁹ (C₁-C₆)-Alkyl bedeutet,
R¹⁰ Wasserstoff oder (C₁-C₆)-Alkyl bedeutet,
R¹¹ und R¹² gleich oder verschieden sind und unabhängig voneinander Wasserstoff, (C₁-C₆)-Alkyl, (C₃-C₆)-Cycloalkyl oder 4- bis 6-gliedriges Heterocyclyl bedeuten,
wobei (C₁-C₆)-Alkyl, (C₃-C₆)-Cycloalkyl und 4- bis 6-gliedriges Heterocyclyl ihrerseits bis zu zweifach, gleich oder verschieden, mit Fluor, Hydroxy, (C₁-C₄)-Alkoxy, Oxo, Amino, Mono- oder Di-(C₁-C₄)-alkylamino, Hydroxycarbonyl, (C₁-C₄)-Alkoxycarbonyl und Aminocarbonyl substituiert sein können und wobei in (C₁-C₆)-Alkyl eine CH₂-Gruppe, soweit in einer chemisch stabilen Verbindung resultierend, gegen ein O-Atom ausgetauscht sein kann, oder
R¹¹ und R¹² zusammen mit dem Stickstoffatom, an das sie gebunden sind, einen 4- bis 6-gliedrigen Heterocyclus bilden, der ein weiteres Ring-Heteroatom aus der Reihe N, O, S, SO oder SO₂ enthalten und bis zu zweifach, gleich oder verschieden, mit (C₁-C₄)-Alkyl, Hydroxy, (C₁-C₄)-Alkoxy, Oxo, Amino, Mono- und Di-(C₁-C₄)-alkylamino substituiert sein kann,
wobei (C₁-C₄)-Alkyl seinerseits mit Hydroxy oder (C₁-C₄)-Alkoxy substituiert sein kann,
R¹³ Wasserstoff oder (C₁-C₄)-Alkyl bedeutet und
R¹⁴ (C₁-C₆)-Alkyl, (C₃-C₆)-Cycloalkyl, Phenyl oder 5- oder 6-gliedriges Heteroaryl bedeutet,
wobei (C₁-C₆)-Alkyl mit Fluor, Chlor, Hydroxy, (C₁-C₄)-Alkoxy, Mono- oder Di-(C₁-C₄)-alkylamino substituiert sein kann
und
Phenyl und 5- oder 6-gliedriges Heteroaryl ihrerseits bis zu zweifach, gleich oder verschieden, mit Fluor, Chlor, Cyano, (C₁-C₄)-Alkyl, Trifluormethyl, (C₁-C₄)-Alkoxy und Trifluormethoxy substituiert sein können,
- R³: für (C₁-C₆)-Alkyl oder (C₂-C₆)-Alkenyl, welche jeweils mit Hydroxy, (C₁-C₄)-Alkoxy, Amino, Mono- oder Di-(C₁-C₄)-alkylamino, Hydroxycarbonyl, (C₁-C₄)-Alkoxycarbonyl, Aminocarbonyl oder Mono- oder Di-(C₁-C₄)-alkylaminocarbonyl substituiert sein können,
oder
für eine Gruppe der Formel -L³-R¹⁵ steht, worin
L³ eine Bindung oder (C₁-C₄)-Alkandiyl bedeutet und
R¹⁵ (C₃-C₇)-Cycloalkyl, 4- bis 7-gliedriges Heterocyclyl, Phenyl oder 5- oder 6-gliedriges Heteroaryl bedeutet,
wobei (C₃-C₇)-Cycloalkyl und 4- bis 7-gliedriges Heterocyclyl ihrerseits bis zu zweifach, gleich oder verschieden, mit (C₁-C₄)-Alkyl, Oxo, Hydroxy und (C₁-C₄)-Alkoxy substituiert sein können
und
Phenyl und 5- oder 6-gliedriges Heteroaryl ihrerseits bis zu zweifach, gleich oder verschieden, mit Fluor, Chlor, Cyano, (C₁-C₄)-Alkyl, Trifluormethyl, (C₁-C₄)-Alkoxy, Trifluormethoxy und Amino substituiert sein können,
- R⁴: für Nitro oder Trifluormethyl steht
und
- R⁵: für Wasserstoff, Fluor oder Chlor steht,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachstehend aufgeführten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren und Diastereomeren sowie ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, *N*-Methylmorpholin, Arginin, Lysin, Ethylendiamin und *N*-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Außerdem umfasst die vorliegende Erfindung auch Prodrugs der erfmdungsgemäßen Verbindungen. Der Begriff "Prodrugs" umfasst Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
(C₁-C₆)-Alkyl und (C₁-C₄)-Alkyl stehen im Rahmen der Erfindung für einen geradkettigen oder verzweigten Alkylrest mit 1 bis 6 bzw. 1 bis 4 Kohlenstoffatomen. Bevorzugt ist ein geradkettiger oder verzweigter Alkylrest mit 1 bis 4 Kohlenstoffatomen. Beispielhaft und vorzugsweise seien genannt: Methyl, Ethyl, *n*-Propyl, Isopropyl, *n*-Butyl, *iso*-Butyl, *sec*.-Butyl, *tert*.-Butyl, 1-Ethylpropyl, *n*-Pentyl, Neopentyl und *n*-Hexyl.
(C₁-C₆)-Alkandiyl und (C₁-C₄)-Alkandiyl stehen im Rahmen der Erfindung für einen geradkettigen oder verzweigten divalenten Alkylrest mit 1 bis 6 bzw. 1 bis 4 Kohlenstoffatomen. Bevorzugt ist ein geradkettiger oder verzweigter Alkandiylrest mit 1 bis 4 Kohlenstoffatomen. Beispielhaft und vorzugsweise seien genannt: Methylen, Ethan-1,2-diyl (1,2-Ethylen), Ethan-1,1-diyl, Propan-1,3-diyl (1,3-Propylen), Propan-1,1-diyl, Propan-1,2-diyl, Propan-2,2-diyl, Butan-1,4-diyl (1,4-Butylen), Butan-1,2-diyl, Butan-1,3-diyl, Butan-2,3-diyl, Pentan-1,5-diyl (1,5-Pentylen), Pentan-2,4-diyl, 3-Methylpentan-2,4-diyl und Hexan-1,6-diyl (1,6-Hexylen).
(C₂-C₆)-Alkenyl und (C₂-C₄)-Alkenyl stehen im Rahmen der Erfindung für einen geradkettigen oder verzweigten Alkenylrest mit 2 bis 6 bzw. 2 bis 4 Kohlenstoffatomen und einer Doppelbindung. Bevorzugt ist ein geradkettiger oder verzweigter Alkenylrest mit 2 bis 4 Kohlenstoffatomen. Beispielhaft und vorzugsweise seien genannt: Vinyl, Allyl, Isopropenyl, *n*-But-2-en-1-yl, *n*-But-3-en-1-yl, *n*-Pent-2-en-1-yl, *n*-Pent-3-en-1-yl, *n*-Pent-4-en-1-yl, 3-Methylbut-2-en-1-yl und 4-Methyl-pent-3-en-1-yl.
(C₁-C₆)-Alkoxy und (C₁-C₄)-Alkoxy stehen im Rahmen der Erfindung für einen geradkettigen oder verzweigten Alkoxyrest mit 1 bis 6 bzw. 1 bis 4 Kohlenstoffatomen. Bevorzugt ist ein geradkettiger oder verzweigter Alkoxyrest mit 1 bis 4 Kohlenstoffatomen Beispielhaft und vorzugsweise seien genannt: Methoxy, Ethoxy, *n*-Propoxy, Isopropoxy, *n*-Butoxy, *iso*-Butoxy, *sec*.-Butoxy, *tert.-*Butoxy, 1-Ethylpropoxy, *n*-Pentoxy, Neopentoxy und *n*-Hexoxy.
(C₁-C₄)-Alkoxycarbonyl steht im Rahmen der Erfindung für einen geradkettigen oder verzweigten Alkoxyrest mit 1 bis 4 Kohlenstoffatomen, der über eine Carbonylgruppe verknüpft ist. Beispielhaft und vorzugsweise seien genannt: Methoxycarbonyl, Ethoxycarbonyl, *n*-Propoxycarbonyl, Isopropoxycarbonyl, *n*-Butoxycarbonyl und *tert*.-Butoxycarbonyl.
Mono-(C₁-C₆)-alkylamino und Mono-(C₁-C₄)-alkylamino stehen im Rahmen der Erfindung für eine Amino-Gruppe mit einem geradkettigen oder verzweigten Alkylsubstituenten, der 1 bis 6 bzw. 1 bis 4 Kohlenstoffatome aufweist. Bevorzugt ist ein geradkettiger oder verzweigter Monoalkylamino-Rest mit 1 bis 4 Kohlenstoffatomen. Beispielhaft und vorzugsweise seien genannt: Methylamino, Ethylamino, *n*-Propylamino, Isopropylamino, *n*-Butylamino, *tert*.-Butylamino, *n*-Pentylamino und *n*-Hexylamino.
Di-(C₁-C₆)-alkylamino und Di-(C₁-C₄)-alkylamino stehen im Rahmen der Erfindung für eine Amino-Gruppe mit zwei gleichen oder verschiedenen geradkettigen oder verzweigten Alkylsubstituenten, die jeweils 1 bis 6 bzw. 1 bis 4 Kohlenstoffatome aufweisen. Bevorzugt ist ein geradkettiger oder verzweigter Dialkylamino-Rest mit jeweils 1 bis 4 Kohlenstoffatomen. Beispielhaft und vorzugsweise seien genannt: *N,N*-Dimethylamino, *N,N*-Diethylamino, *N*-Ethyl-*N*-methylamino, *N*-Methyl-*N*-*n*-propylamino, *N*-Isopropyl-*N*-methylamino, *N*-Isopropyl-*N*-*n*-propylamino, *N,N*-Diisopropylamino, *N*-*n*-Butyl-N-methylamino, *N*-*tert*.-Butyl-*N*-methylamino, *N*-Methyl-*N-n-*pentylamino und *N*-*n*-Hexyl-*N*-methylamino.
Mono- bzw. Di-(C₁-C₄)-alkylaminocarbonyl steht im Rahmen der Erfindung für eine Amino-Gruppe, die über eine Carbonylgruppe verknüpft ist und die einen geradkettigen oder verzweigten bzw. zwei gleiche oder verschiedene geradkettige oder verzweigte Alkylsubstituenten mit jeweils 1 bis 4 Kohlenstoffatomen aufweist. Beispielhaft und vorzugsweise seien genannt: Methylaminocarbonyl, Ethylaminocarbonyl, *n*-Propylaminocarbonyl, Isopropylaminocarbonyl, *n*-Butylaminocarbonyl, *tert*.-Butylaminocarbonyl, *N,N-*Dimethylaminocarbonyl, *N,N*-Diethylaminocarbonyl, *N-*Ethyl-*N*-methylaminocarbonyl, *N*-Methyl-*N*-*n*-propylaminocarbonyl, *N*-Isopropyl-*N*-methylaminocarbonyl, *N-n*-Butyl-*N*-methylaminocarbonyl und *N*-tert.-Butyl-*N*-methylaminocarbonyl .
(C₃-C₇)-Cycloalkyl und (C₃-C₆)-Cycloalkyl stehen im Rahmen der Erfindung für eine monocyclische, gesättigte Cycloalkylgruppe mit 3 bis 7 bzw. 3 bis 6 Ring-Kohlenstoffatomen. Beispielhaft und vorzugsweise seien genannt: Cyclopropyl, Cyclobutyl, Cyclopentyl, Cyclohexyl und Cycloheptyl.

Ein 4- bis 7-gliedriger Heterocyclus steht im Rahmen der Erfindung für einen monocyclischen, gesättigten Heterocyclus mit insgesamt 4 bis 7 Ringatomen, der ein oder zwei Ring-Heteroatome aus der Reihe N, O, S, SO und/oder SO₂ enthält und über ein Ring-Kohlenstoffatom oder gegebenenfalls ein Ring-Stickstoffatom verknüpft ist. Bevorzugt ist ein 4- bis 6-gliedriger Heterocyclus mit ein oder zwei Ring-Heteroatomen aus der Reihe N, O und/oder S, besonders bevorzugt ist ein 5- oder 6-gliedriger Heterocyclus mit ein oder zwei Ring-Heteroatomen aus der Reihe N und/oder O. Beispielhaft seien genannt: Azetidinyl, Oxetanyl, Pyrrolidinyl, Pyrazolidinyl, Tetrahydrofuranyl, Thiolanyl, Piperidinyl, Piperazinyl, Tetrahydropyranyl, Tetrahydrothiopyranyl, Morpholinyl, Thiomorpholinyl, Hexahydroazepinyl und Hexahydro-1,4-diazepinyl. Bevorzugt sind Azetidinyl, Pyrrolidinyl, Tetrahydrofuranyl, Piperidinyl, Piperazinyl, Tetrahydropyranyl und Morpholinyl, besonders bevorzugt Pyrrolidinyl, Tetrahydrofuranyl, Piperidinyl und Morpholinyl.

Ein Pyrrolidino-, Piperidino- oder Morpholino-Rest steht im Rahmen der Erfindung für einen über das jeweilige Ring-Stickstoffatom verknüpften Pyrrolidin-, Piperidin- bzw. Morpholin-Ring.

5- oder 6-gliedriges Heteroaryl steht im Rahmen der Erfindung für einen aromatischen Heterocyclus (Heteroaromaten) mit insgesamt 5 oder 6 Ringatomen, der ein oder zwei Ring-Heteroatome aus der Reihe N, O und/oder S enthält und über ein Ring-Kohlenstoffatom oder gegebenenfalls ein Ring-Stickstoffatom verknüpft ist. Beispielhaft seien genannt: Furyl, Pyrrolyl, Thienyl, Pyrazolyl, Imidazolyl, Oxazolyl, Thiazolyl, Isoxazolyl, Isothiazolyl, Pyridyl, Pyrimidinyl, Pyridazinyl und Pyrazinyl. Bevorzugt sind Thienyl, Pyridyl, Pyrimidinyl, Pyridazinyl und Pyrazinyl.

Halogen schließt im Rahmen der Erfindung Fluor, Chlor, Brom und Iod ein. Bevorzugt sind Chlor, Fluor oder Brom, besonders bevorzugt Fluor oder Chlor.

Ein Oxo-Substituent steht im Rahmen der Erfindung für ein Sauerstoffatom, das über eine Doppelbindung an ein Kohlenstoffatom gebunden ist.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit einem oder zwei gleichen oder verschiedenen Substituenten ist bevorzugt. Ganz besonders bevorzugt ist die Substitution mit einem Substituenten.

Gegenstand der vorliegenden Erfindung sind insbesondere solche Verbindungen der Formel (I), in welcher
- A: für CH steht,
sowie ihre Salze, Solvate und Solvate der Salze.

Gegenstand der vorliegenden Erfindung sind weiterhin insbesondere solche Verbindungen der Formel (I), in welcher
- R⁴: für Trifluormethyl steht
und
- R⁵: für Wasserstoff oder Fluor steht,
sowie ihre Salze, Solvate und Solvate der Salze.

Bevorzugt im Rahmen der vorliegenden Erfindung sind Verbindungen der Formel (I), in welcher
- A: für CH steht,
- R¹: für Wasserstoff, Fluor, Chlor, Cyano, Nitro, (C₁-C₄)-Alkyl, Difluormethyl, Trifluormethyl, (C₁-C₄)-Alkoxy, Difluormethoxy, Trifluormethoxy, Amino, Mono- oder Di-(C₁-C₄)-alkylamino
oder
für eine Gruppe der Formel -NH-C(=O)-R⁶, -NH-SO₂-R⁷ oder -SO₂-R⁸ steht, worin
R⁶ und R⁷ jeweils (C₁-C₄)-Alkyl bedeuten
und
R⁸ (C₁-C₄)-Alkyl, das mit Hydroxy, (C₁-C₄)-Alkoxy, Hydroxycarbonyl, Aminocarbonyl, (C₃-C₆)-Cycloalkyl oder Phenyl substituiert sein kann, oder (C₃-C₆)-Cycloalkyl oder Phenyl bedeutet,
wobei die genannten Phenyl-Gruppen bis zu zweifach, gleich oder verschieden, mit Fluor, Chlor, Cyano, Methyl, Trifluormethyl, Methoxy und Trifluormethoxy substituiert sein können,
- R²: für Wasserstoff, (C₁-C₄)-Alkyl oder (C₂-C₄)-Alkenyl
oder
für eine Gruppe der Formel -L¹-C(=O)-O-R¹⁰, -L²-C(=O)-NR¹¹R¹² oder -L²-SO₂-R¹⁴ steht, worin
L¹ Methylen oder Ethan-1,2-diyl bedeutet,
L² eine Bindung, Methylen, Ethan-1,1-diyl oder Ethan-1,2-diyl bedeutet,
R¹⁰ Wasserstoff oder (C₁-C₄)-Alkyl bedeutet,
R¹¹ Wasserstoff oder (C₁-C₄)-Alkyl, das mit Hydroxy oder (C₁-C₄)-Alkoxy substituiert sein kann, bedeutet,
R¹² Wasserstoff, (C₁-C₆)-Alkyl oder (C₃-C₆)-Cycloalkyl bedeutet,
wobei (C₁-C₆)-Alkyl bis zu zweifach, gleich oder verschieden, mit Hydroxy, (C₁-C₄)-Alkoxy, Hydroxycarbonyl, (C₁-C₄)-Alkoxycarbonyl und Aminocarbonyl substituiert sein kann und in (C₁-C₆)-Alkyl eine CH₂-Gruppe, soweit in einer chemisch stabilen Verbindung resultierend, gegen ein O-Atom ausgetauscht sein kann, oder
R¹¹ und R¹² zusammen mit dem Stickstoffatom, an das sie gebunden sind, einen 5- oder 6-gliedrigen Heterocyclus bilden, der ein weiteres Ring-Heteroatom aus der Reihe N, O oder S enthalten und mit (C₁-C₄)-Alkyl, Hydroxy, (C₁-C₄)-Alkoxy oder Oxo substituiert sein kann,
wobei (C₁-C₄)-Alkyl seinerseits mit Hydroxy oder (C₁-C₄)-Alkoxy substituiert sein kann, und
R¹⁴ (C₁-C₄)-Alkyl, (C₃-C₆)-Cycloalkyl oder Phenyl bedeutet,
wobei Phenyl bis zu zweifach, gleich oder verschieden, mit Fluor, Chlor, Cyano, Methyl, Trifluormethyl, Methoxy und Trifluormethoxy substituiert sein kann,
- R³: für (C₁-C₄)-Alkyl, das mit Hydroxy, (C₁-C₄)-Alkoxy, Hydroxycarbonyl, (C₁-C₄)-Alkoxycarbonyl, Aminocarbonyl, Mono- oder Di-(C₁-C₄)-alkylaminocarbonyl oder 5- oder 6-gliedrigem Heteroaryl substituiert sein kann, für (C₂-C₄)-Alkenyl
oder
für eine Gruppe der Formel -L³-R¹⁵ steht, worin
L³ eine Bindung oder (C₁-C₄)-Alkandiyl bedeutet und
R¹⁵ (C₃-C₇)-Cycloalkyl, 4- bis 6-gliedriges Heterocyclyl oder Phenyl bedeutet, wobei 4- bis 6-gliedriges Heterocyclyl seinerseits mit Oxo substituiert sein kann
und
Phenyl seinerseits bis zu zweifach, gleich oder verschieden, mit Fluor, Chlor, Cyano, Methyl, Trifluormethyl, Methoxy und Trifluormethoxy substituiert sein kann,
- R⁴: für Trifluormethyl steht
und
- R⁵: für Wasserstoff oder Fluor steht,
sowie ihre Salze, Solvate und Solvate der Salze.

Besonders bevorzugt im Rahmen der vorliegenden Erfindung sind Verbindungen der Formel (I), in welcher
- A: für CH steht,
- R¹: für Wasserstoff, Fluor, Chlor, Nitro, Methyl, Difluormethyl, Trifluormethyl, Methoxy, Trifluormethoxy oder eine Gruppe der Formel -SO₂-R⁸ steht, worin
R⁸ (C₁-C₄)-Alkyl, das mit Hydroxy, Methoxy oder Ethoxy substituiert sein kann, bedeutet,
- R²: für Wasserstoff, (C₁-C₄)-Alkyl oder eine Gruppe der Formel -CH₂-C(=O)-O-R¹⁰ oder -CH₂-C(=O)-NR¹¹R¹² steht, worin
R¹⁰ (C₁-C₄)-Alkyl bedeutet,
R¹¹ Wasserstoff oder Methyl bedeutet,
R¹² Wasserstoff oder (C₁-C₄)-Alkyl, das mit Hydroxy, Methoxy oder Ethoxy substituiert sein kann, bedeutet oder
R¹¹ und R¹² zusammen mit dem Stickstoffatom, an das sie gebunden sind, einen Pyrrolidino-, Piperidino- oder Morpholino-Ring bilden,
- R³: für (C₁-C₄)-Alkyl, das mit Hydroxy, Pyrrolidino, Piperidino, Morpholino oder Pyridyl substituiert sein kann, für Allyl oder für eine Gruppe der Formel -L³-R¹⁵ steht, worin
L³ eine Bindung, Methylen oder Ethan-1,2-diyl bedeutet und
R¹⁵ (C₃-C₇)-Cycloalkyl oder Phenyl bedeutet,
wobei Phenyl seinerseits bis zu zweifach, gleich oder verschieden, mit Fluor, Chlor, Cyano, Methyl oder Trifluormethyl substituiert sein kann,
- R⁴: für Trifluormethyl steht
und
- R⁵: für Wasserstoff steht,
sowie ihre Salze, Solvate und Solvate der Salze.

Ganz besonders bevorzugt im Rahmen der vorliegenden Erfindung sind Verbindungen der Formel (I), in welcher
- A: für CH steht,
- R¹: für Wasserstoff, Trifluormethyl oder Methylsulfonyl steht,
- R²: für Wasserstoff oder eine Gruppe der Formel -CH₂-C(=O)-NR¹¹R¹² steht, worin
R¹¹ und R¹² unabhängig voneinander Wasserstoff oder Methyl bedeuten oder
R¹¹ und R¹² zusammen mit dem Stickstoffatom, an das sie gebunden sind, einen Pyrrolidino-Ring bilden,
- R³: für Methyl, Ethyl, 2-Hydroxyethyl oder 2-(Morpholin-4-yl)ethyl steht,
- R⁴: für Trifluormethyl steht
und
- R⁵: für Wasserstoff steht,
sowie ihre Salze, Solvate und Solvate der Salze.

Von besonderer Bedeutung sind Verbindungen gemäß Formel (I) mit der in Formel (I-*ent*) wiedergegebenen Konfiguration an der 4-Position des Dihydropyrimidinon-Rings 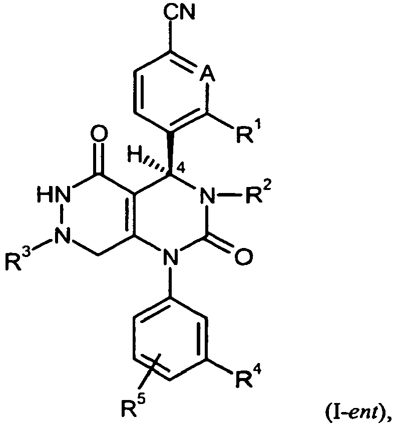 worin A, R¹, R², R³, R⁴ und R⁵ jeweils die oben angegebenen Bedeutungen haben,
und ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Die erfindungsgemäßen 1,4-Diaryl-pyrimido[4,5-d]pyridazin-2,5-dione der Formel (I) können in verschiedenen tautomeren Formen vorliegen (vgl. nachfolgendes Schema 1); sämtliche tautomeren Formen werden von der vorliegenden Erfindung ausdrücklich umfasst.

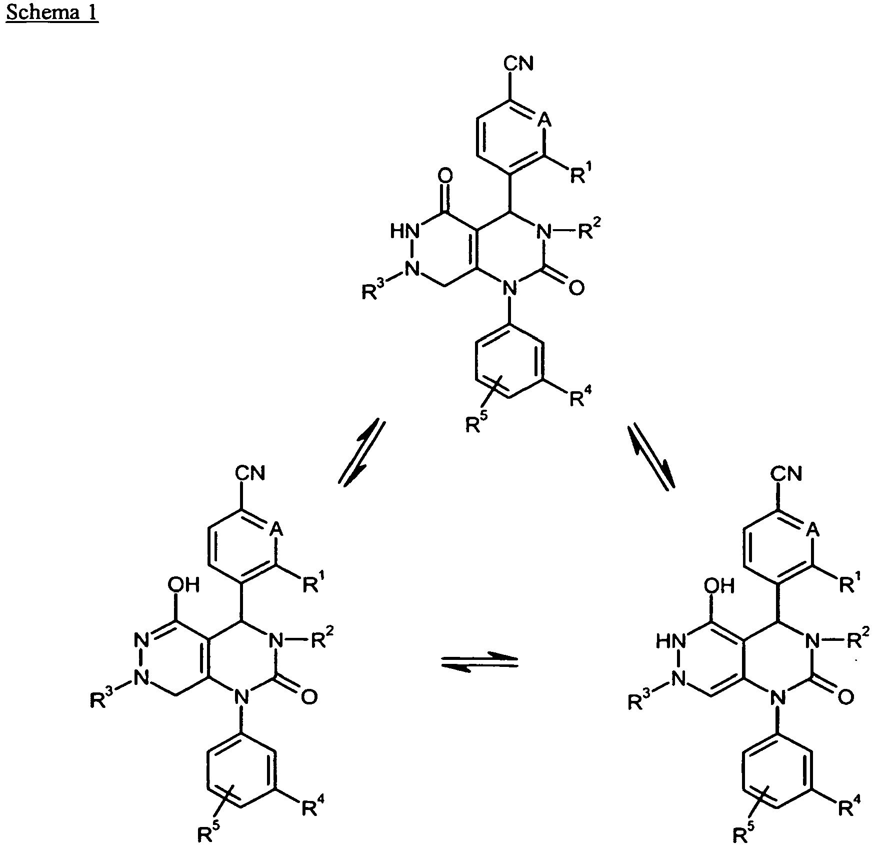

Weiterer Gegenstand der Erfindung ist ein Verfahren zur Herstellung der erfindungsgemäßen Verbindungen der Formel (I), dadurch gekennzeichnet, dass man zunächst eine Verbindung der Formel (II) 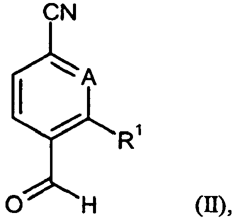 in welcher A und R¹ die oben angegebenen Bedeutungen haben,
in Gegenwart einer Säure oder eines Säureanhydrids in einer 3-Komponenten-Eintopf Reaktion oder sequentiell mit einem Acetessigsäureester der Formel (III) 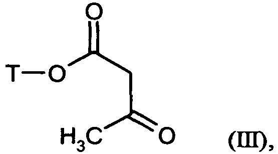 in welcher
- T: für Methyl oder Ethyl steht,
und einem Phenylharnstoff-Derivat der Formel (IV) 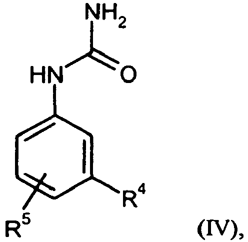 in welcher R⁴ und R⁵ die oben angegebenen Bedeutungen haben,
zu einer Verbindung der Formel (V-A) 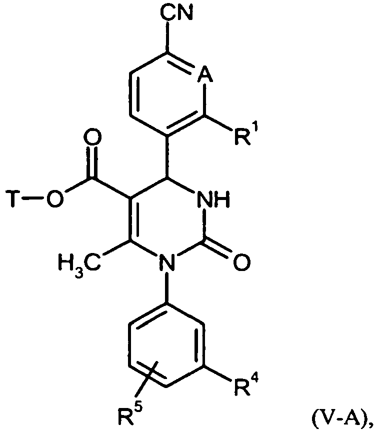 in welcher A, T, R¹, R⁴ und R⁵ jeweils die oben angegebenen Bedeutungen haben,
kondensiert und diese dann
[A] im Falle, dass R² in Formel (I) für Wasserstoff steht, in einem inerten Lösungsmittel zu einer Verbindung der Formel (VI-A) 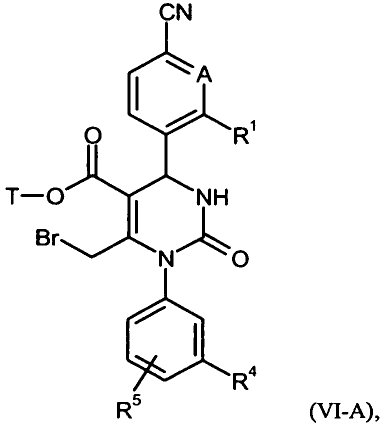 in welcher A, T, R¹, R⁴ und R⁵ jeweils die oben angegebenen Bedeutungen haben,
   bromiert und nachfolgend mit einem Hydrazin-Derivat der Formel (VII)

   R³-NH-NH₂ (VII),

   in welcher R³ die oben angegebene Bedeutung hat,
   unter Ausbildung eines Sechsrings zu einer Verbindung der Formel (I-A) 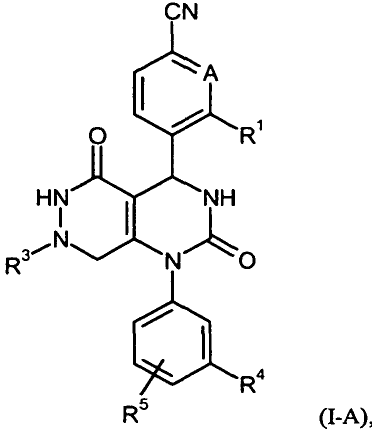 in welcher A, R¹, R³, R⁴ und R⁵ jeweils die oben angegebenen Bedeutungen haben,
   umsetzt
   oder
[B] im Falle, dass R² in Formel (I) von Wasserstoff verschieden ist, zunächst mit einer Verbindung der Formel (VIII)

   R^{2A}-X (VIII),

   in welcher
   - R^{2A}: die oben angegebene Bedeutung von R² hat, jedoch nicht für Wasserstoff steht,
   und
   - X: für eine Abgangsgruppe wie beispielsweise Halogen, Mesylat, Tosylat oder Triflat steht,
   in Gegenwart einer Base zu einer Verbindung der Formel (V-B) 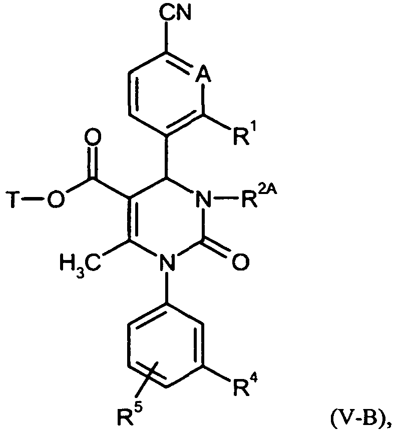 in welcher A, T, R¹, R^{2A}, R⁴ und R⁵ jeweils die oben angegebenen Bedeutungen haben,
   reagiert, danach in einem inerten Lösungsmittel zu einer Verbindung der Formel (VI-B) 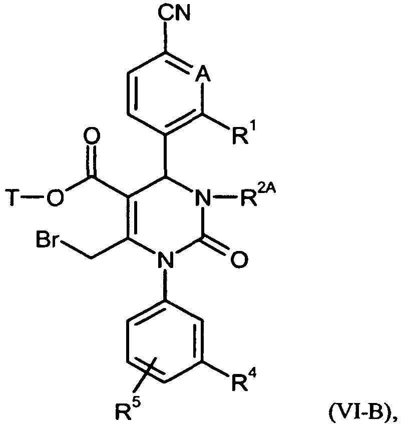 in welcher A, T, R¹, R^{2A}, R⁴ und R⁵ jeweils die oben angegebenen Bedeutungen haben,
   bromiert und nachfolgend mit einem Hydrazin-Derivat der Formel (VII)

   R³-NH-NH₂ (VII),

   in welcher R³ die oben angegebene Bedeutung hat,
   unter Cyclisierung zu einer Verbindung der Formel (I-B) 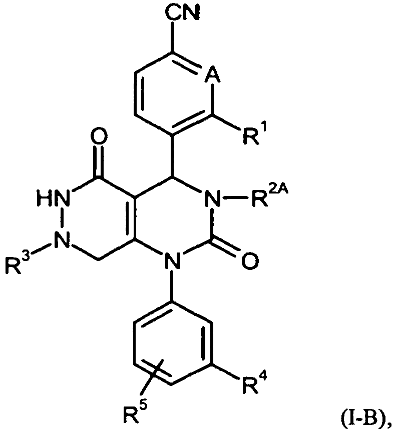 in welcher A, R¹, R^{2A}, R³, R⁴ und R⁵ jeweils die oben angegebenen Bedeutungen haben,
   umsetzt
und gegebenenfalls die so erhaltenen Verbindungen der Formel (I-A) bzw. (I-B) nach dem Fachmann bekannten Methoden in ihre Enantiomere und/oder Diastereomere trennt und/oder mit den entsprechenden (i) Lösungsmitteln und/oder (ii) Basen oder Säuren in ihre Solvate, Salze und/oder Solvate der Salze überführt.

Für den Verfahrensschritt (II) + (III) + (IV) → (V-A) geeignete Lösungsmittel sind übliche organische Lösungsmittel, die sich unter den Reaktionsbedingungen nicht verändern. Dazu zählen beispielsweise Ether wie Diethylether, Diisopropylether, Methyl-*tert*.-butylether, 1,2-Dimethoxyethan, Dioxan oder Tetrahydrofuran, Alkohole wie Methanol, Ethanol, *n*-Propanol, Isopropanol, *n*-Butanol oder *tert*.-Butanol, Kohlenwasserstoffe wie Pentan, Hexan, Cyclohexan, Benzol, Toluol oder Xylol, Halogenkohlenwasserstoffe wie Dichlormethan, 1,2-Dichlorethan, Trichlormethan oder Chlorbenzol, oder andere Lösungsmittel wie Ethylacetat, Acetonitril, Dimethylsulfoxid oder *N,N*-Dimethylformamid. Ebenso ist es möglich, Gemische der genannten Lösungsmittel einzusetzen. Bevorzugt wird Methyl-*tert*.-butylether, Tetrahydrofuran oder Dioxan verwendet.

Als Säure für den Verfahrensschritt (II) + (III) + (IV) → (V-A) eignen sich übliche anorganische oder organische Säuren oder Säureanhydride. Hierzu gehören bevorzugt Carbonsäuren wie beispielsweise Essigsäure oder Trifluoressigsäure, Sulfonsäuren wie Methansulfonsäure, Trifluormethansulfonsäure oder p-Toluolsulfonsäure, Salzsäure, Schwefelsäure, Phosphorsäure, Phosphonsäuren, oder Phosphorsäure- oder Phosphonsäureanhydride oder -ester wie Polyphosphorsäure, Phosphorsäuretriethylester, Polyphosphorsäureethylester, Phosphorpentoxid oder Propanphosphonsäureanhydrid. Bevorzugt wird Phosphorsäuretriethylester in Kombination mit Phosphorpentoxid verwendet. Die Säure wird im Allgemeinen in einer Menge von 0.25 Mol bis 100 Mol, bezogen auf 1 Mol der Verbindung (III), eingesetzt.

Der Verfahrensschritt (II) + (III) + (IV) → (V-A) wird im Allgemeinen in einem Temperaturbereich von +20°C bis +150°C, bevorzugt bei +50°C bis +100°C durchgeführt. Die Umsetzung kann bei normalem, erhöhtem oder erniedrigtem Druck erfolgen (z.B. von 0.5 bis 5 bar). Im Allgemeinen arbeitet man bei Normaldruck.

Die Bromierung im Verfahrensschritt (V-A) → (VI-A) bzw. (V-B) → (VI-B) wird bevorzugt mit Hilfe von elementarem Brom in einem üblichen inerten Lösungsmittel wie Chloroform bei einer Temperatur von -20°C bis +40°C durchgeführt.

Die Dihydropyridazinon-Bildung im Verfahrensschritt (VI-A) + (VII) → (I-A) bzw. (VI-B) + (VII) → (I-B) erfolgt vorzugsweise in einem Ether wie Tetrahydrofuran, 1,2-Dimethoxyethan oder Dioxan als inertem Lösungsmittel bei einer Temperatur von +20°C bis +120°C. Das Hydrazin-Derivat der Formel (VII) kann hierbei auch in Form eines Salzes, zum Beispiel als Hydrochlorid, eingesetzt werden; in diesem Falle wird die Reaktion in Gegenwart einer tertiären Amin-Base, wie beispielsweise Triethylamin, *N*-Methylmorpholin, *N*-Methylpiperidin oder *N,N*-Diisopropylethylamin, oder einer Carbonat-Base, wie beispielsweise Natrium-, Kalium- oder Cäsiumcarbonat oder polymer gebundenes Carbonat, durchgeführt.

Für den Verfahrensschritt (V-A) + (VIII) → (V-B) geeignete Lösungsmittel sind übliche organische Lösungsmittel, die sich unter den Reaktionsbedingungen nicht verändern. Dazu zählen beispielsweise Ether wie Diethylether, Diisopropylether, Methyl-*tert*.-butylether, 1,2-Dimethoxyethan, Dioxan oder Tetrahydrofuran, Kohlenwasserstoffe wie Pentan, Hexan, Cyclohexan, Benzol, Toluol oder Xylol, Halogenkohlenwasserstoffe wie Dichlormethan, 1,2-Dichlorethan, Trichlormethan oder Chlorbenzol, oder andere Lösungsmittel wie Ethylacetat, Aceton, Methylethylketon, Methyl-*tert*.-butylketon, Acetonitril, Dimethylsulfoxid, *N,N*-Dimethylformamid, *N,N'*-Dimethylpropylenharnstoff (DMPU) oder *N*-Methylpyrrolidon (NMP). Ebenso ist es möglich, Gemische der genannten Lösungsmittel einzusetzen. Bevorzugt wird Tetrahydrofuran, Acetonitril oder Dimethylformamid verwendet.

Als Base für den Verfahrensschritt (V-A) + (VIII) → (V-B) eignen sich übliche anorganische oder organische Basen. Hierzu gehören insbesondere Alkali- oder Erdalkalicarbonate wie Lithium-, Natrium-, Kalium-, Calcium- oder Cäsiumcarbonat, Alkali-Alkoholate wie Natrium- oder Kalium-*tert*.-butylat, Alkalihydride wie Natrium- oder Kaliumhydrid, Amide wie Lithium- oder Kalium-bis(trimethylsilyl)amid oder Lithiumdiisopropylamid (LDA), organische Amine wie Triethylamin, *N*-Methylmorpholin, *N*-Methylpiperidin, *N,N*-Diisopropylethylamin, 1,5-Diazabicyclo[4.3.0]non-5-en (DBN), 1,8-Diazabicyclo[5.4.0]undec-7-en (DBU), Pyridin oder 4-*N,N*-Dimethylaminopyridin, oder Phosphazen-Basen (so genannte "Schwesinger-Basen") wie beispielsweise P1-t-Bu, P2-t-Bu oder P4-t-Bu. Bevorzugt wird Kaliumcarbonat, Cäsiumcarbonat, Natriumhydrid, Triethylamin oder *N,N*-Diisopropylethylamin verwendet; besonders bevorzugt sind Kaliumcarbonat und Natriumhydrid. Die Base wird im Allgemeinen in einer Menge von 0.1 Mol bis 10 Mol, bevorzugt von 1 Mol bis 3 Mol, bezogen auf 1 Mol der Verbindung (V-A), eingesetzt.

Der Verfahrensschritt (V-A) + (VIII) → (V-B) wird im Allgemeinen in einem Temperaturbereich von -20°C bis +100°C, bevorzugt bei 0°C bis +80°C durchgeführt. Die Umsetzung kann bei normalem, erhöhtem oder erniedrigtem Druck erfolgen (z.B. von 0.5 bis 5 bar). Im Allgemeinen arbeitet man bei Normaldruck.

Weitere erfindungsgemäße Verbindungen der Formel (I) können, falls zweckmäßig, auch durch Umwandlungen von funktionellen Gruppen einzelner Substituenten, insbesondere den unter R¹, R² und R³ aufgeführten, ausgehend von anderen, nach obigem Verfahren erhaltenen Verbindungen der Formel (I) hergestellt werden. Diese Umwandlungen werden nach üblichen, dem Fachmann bekannten Methoden durchgeführt und umfassen beispielsweise Reaktionen wie nukleophile oder elektrophile Substitutionsreaktionen, Übergangsmetall-vermittelte Kupplungsreaktionen (z.B. Suzuki- oder Heck-Reaktion), Oxidation, Reduktion, Hydrierung, Alkylierung, Acylierung, Aminierung, Hydroxylierung, Veretherung, Veresterung, Esterspaltung und -hydrolyse, Bildung von Nitrilen, Carbonamiden, Sulfonamiden, Carbamaten und Harnstoffen, sowie die Einführung und Entfernung temporärer Schutzgruppen [vgl. auch nachfolgende Reaktionsschemata 2-4 sowie die Ausführungsbeispiele].

Eine Trennung der erfindungsgemäßen Verbindungen in die entsprechenden Enantiomere und/oder Diastereomere kann, je nach Zweckmäßigkeit, auf der Stufe der Verbindungen (I-A) bzw. (I-B) oder auch auf der Stufe der Verbindungen (V-A) bzw. (V-B) erfolgen, wobei letztere dann in separierter Form entsprechend den zuvor beschriebenen Verfahrensschritten weiter umgesetzt werden. Eine solche Auftrennung der Stereoisomeren läßt sich nach üblichen, dem Fachmann bekannten Methoden durchführen; vorzugsweise werden chromatographische Verfahren, insbesondere die HPLC-Chromatographie an chiraler Phase, verwendet.

Die Verbindungen der Formeln (III), (IV), (VII) und (VIII) sind kommerziell erhältlich oder als solche literaturbekannt oder sie können nach gängigen, in der Literatur beschriebenen Methoden hergestellt werden.

Die Verbindungen der Formel (II) sind zum Teil literaturbekannt oder sie können in Analogie zu in der Literatur beschriebenen Verfahren hergestellt werden [vgl. auch nachfolgende Reaktionsschemata 5 und 6 sowie dort angegebene Literatur].

Bei dem zuvor beschriebenen Verfahren kann es gegebenenfalls synthetisch zweckmäßig sein, statt der Verbindung der Formel (II) zunächst eine Verbindung der Formel (II-A) 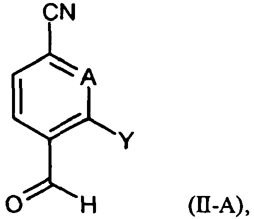 in welcher A die oben angegebene Bedeutung hat
und
- Y: für eine austauschbare Gruppe wie beispielsweise Fluor, Chlor, Brom, Iod, Nitro oder Amino steht,
in der geschilderten Reaktionssequenz einzusetzen und den gewünschten Aryl-Substituenten R¹ dann auf der Stufe des - der Verbindung (V-A) oder (V-B) entsprechenden - Dihydropyrimidinons im Austausch gegen den Rest Y einzuführen. Die Verbindungen der Formel (II-A) sind gleichfalls zum Teil literaturbekannt oder können analog literaturbekannter Methoden hergestellt werden.

Die oben beschriebenen Verfahren können durch die folgenden Reaktionsschemata beispielhaft veranschaulicht werden: 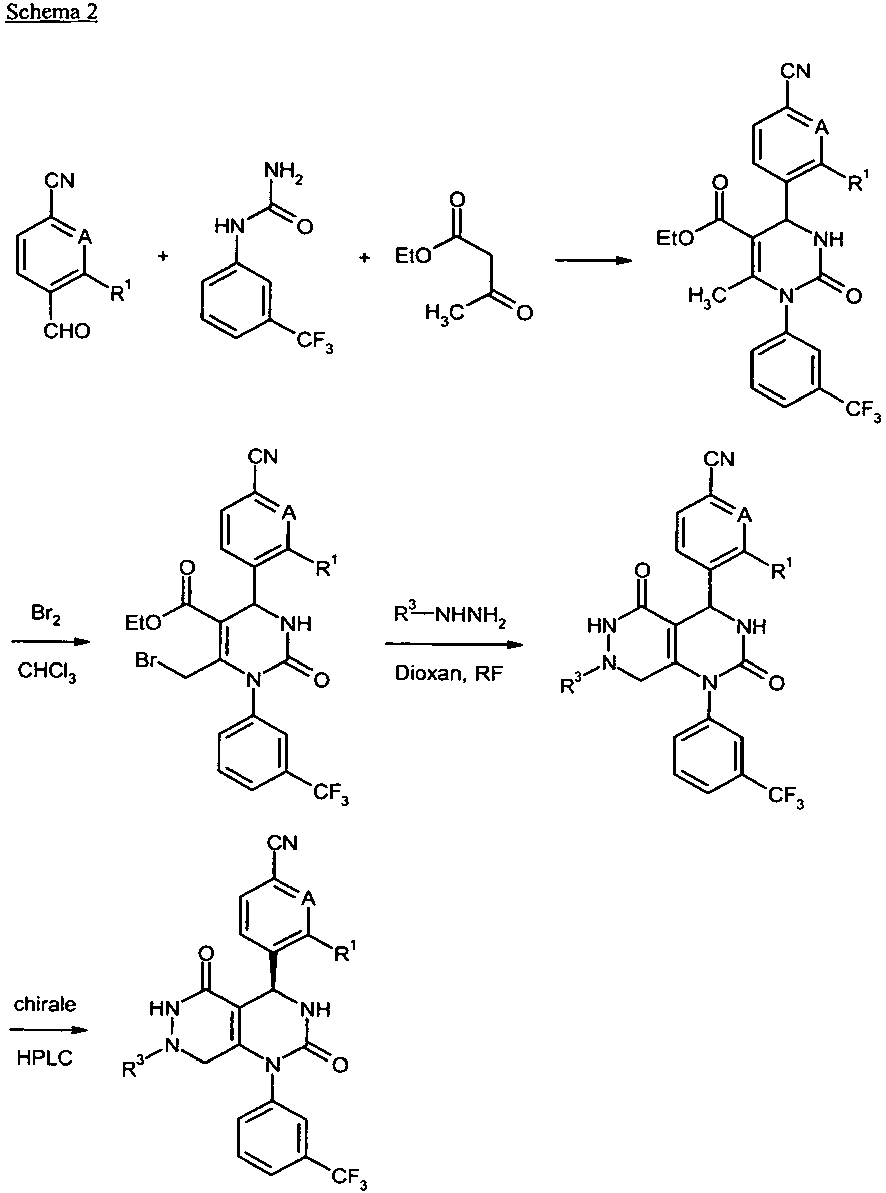 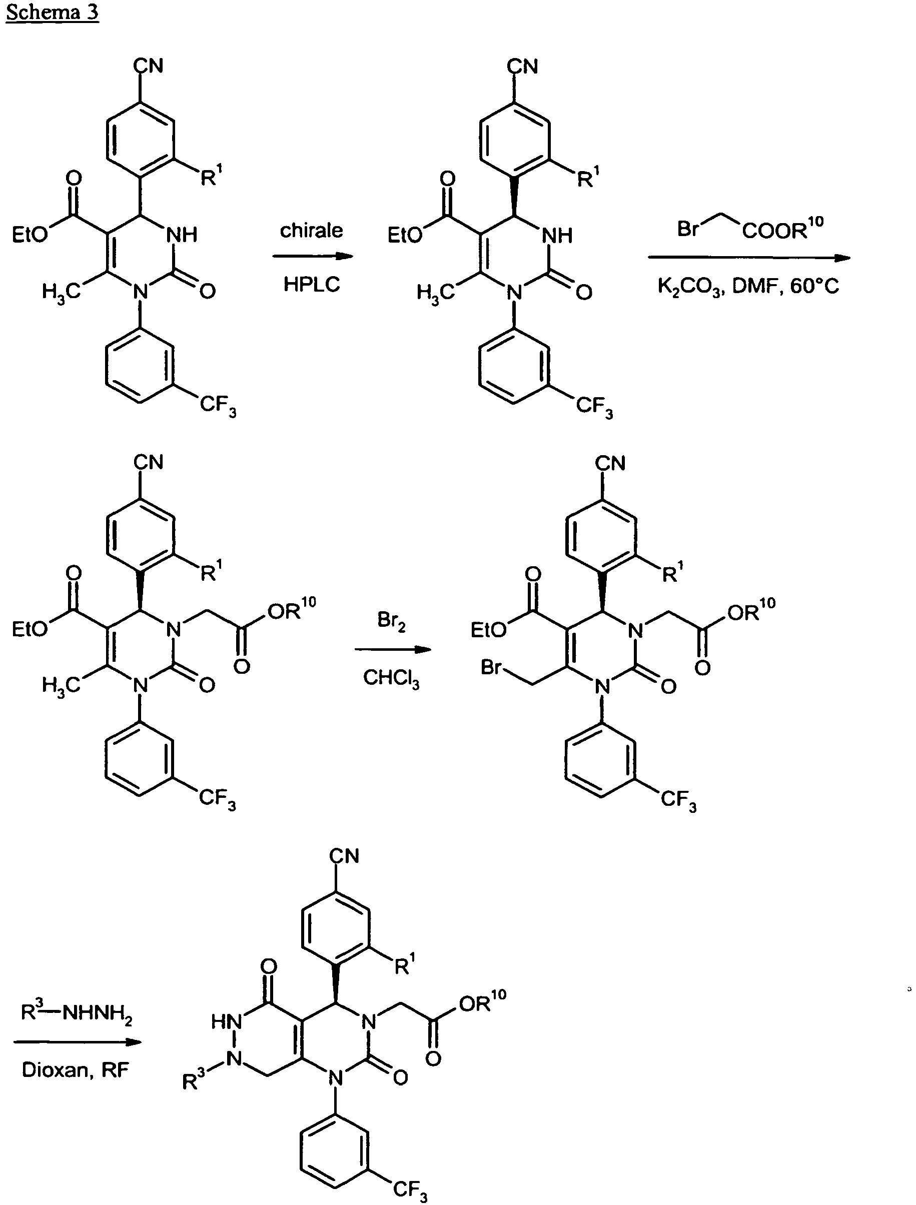 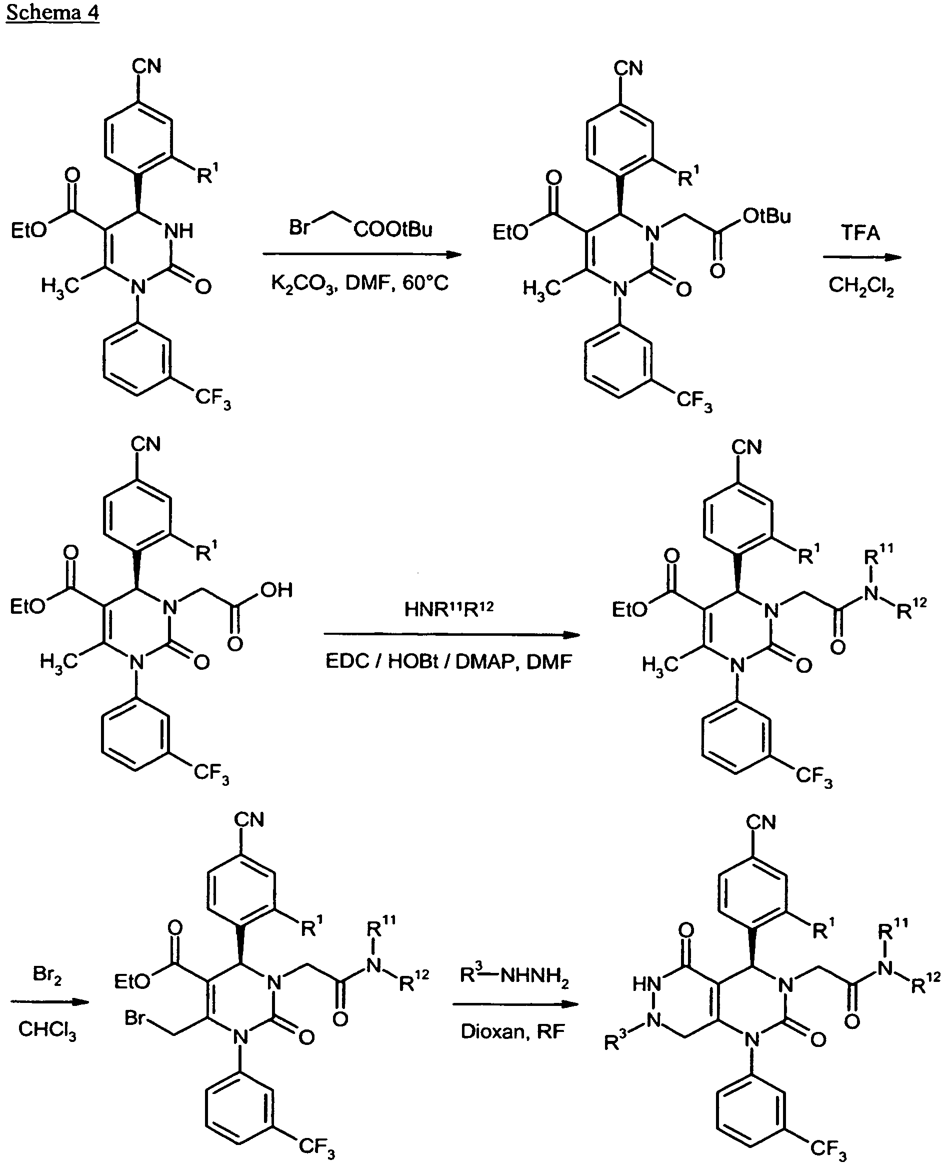 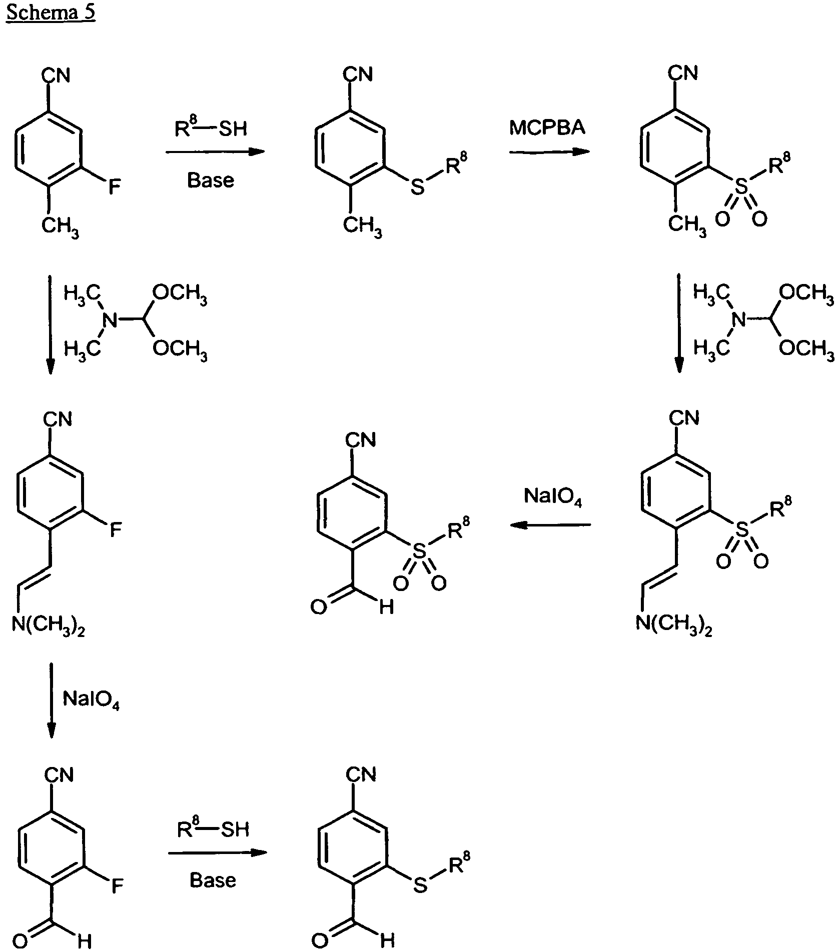 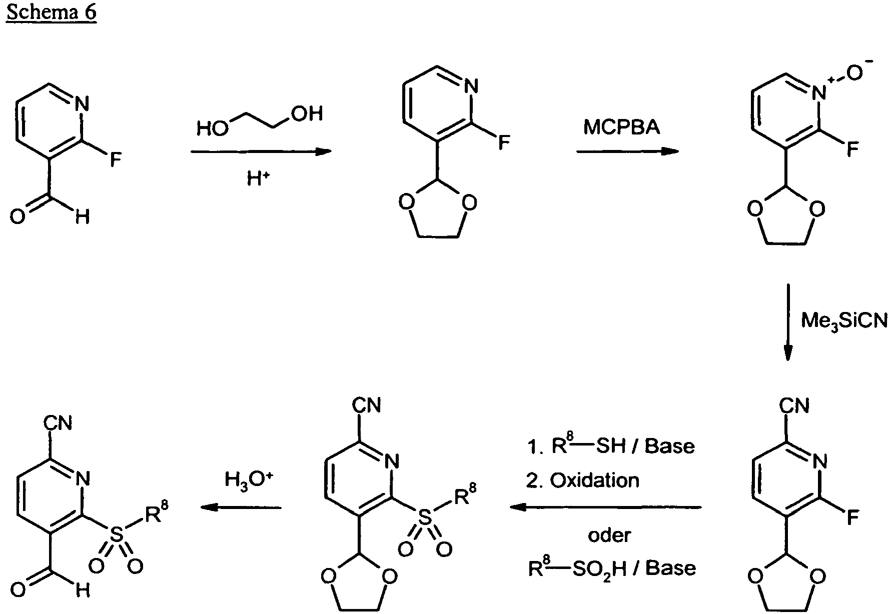 [vgl. z.B. W.K. Fife, J. Org. Chem. 48, 1375 (1983); H. Vorbrüggen und K. Krolikiewicz, Synthesis, 316 (1983); R.T. Shuman et al., J. Org. Chem. 55, 738 (1990); C.S. Burgey et al., J. Med. Chem. 46 (4), 461 (2003); J.J. Li et al., J. Med. Chem. 39, 1846 (1996); K.N. Dack et al., Bioorg. Med. Chem. Lett. 8 (16), 2061 (1998)].

Die erfindungsgemäßen Verbindungen besitzen wertvolle pharmakologische Eigenschaften und können zur Vorbeugung und Behandlung von Erkrankungen bei Menschen und Tieren verwendet werden.

Die erfindungsgemäßen Verbindungen sind potente niedermolekulare, nicht-reaktive und selektive Inhibitoren der humanen neutrophilen Elastase. Darüber hinaus weisen die erfindungsgemäßen Verbindungen vorteilhafte pharmakokinetische Eigenschaften auf, wie beispielsweise eine gute Bioverfügbarkeit und/oder Halbwertszeit nach oraler Applikation oder eine nur geringe Bindung an Plasmaproteine.

Die erfindungsgemäßen Verbindungen eignen sich daher in besonderem Maße zur Behandlung und/oder Prävention von Erkrankungen und pathologischen Prozessen, insbesondere solchen, bei denen im Zuge eines Entzündungsgeschehens und/oder eines Gewebe- oder Gefäßumbaus die neutrophile Elastase (HNE) involviert ist.

Dazu zählen im Sinne der vorliegenden Erfindung insbesondere Erkrankungen wie die pulmonale arterielle Hypertonie (PAH) und andere Formen der pulmonalen Hypertonie (PH), die chronisch-obstruktive Lungenerkrankung (COPD), das akute Atemwegssyndrom (ARDS), akute Lungenschädigung (ALI), alpha-1-Antitrypsin-Defizienz (AATD), Lungenfibrose, Lungenemphysem (z.B. durch Zigarettenrauch induziertes Lungenemphysem), zystische Fibrose (CF), akutes Koronarsyndrom (ACS), Herzmuskelentzündungen (Myokarditis) und andere autoimmune Herzerkrankungen (Perikarditis, Endokarditis, Valvolitis, Aortitis, Kardiomyopathien), Myokardinfarkt, kardiogener Schock, Herzinsuffizienz, Aneurysmen, Sepsis (SIRS), multiples Organversagen (MODS, MOF), Arteriosklerose, entzündliche Erkrankungen der Niere, chronische Darmentzündungen (IBD, CD, UC), Pankreatitis, Peritonitis, rheumatoide Erkrankungen, entzündliche Hauterkrankungen sowie entzündliche Augenerkrankungen.

Die erfindungsgemäßen Verbindungen können weiterhin verwendet werden zur Behandlung und/ oder Prävention von asthmatischen Erkrankungen unterschiedlicher Schweregrade mit intermittierendem oder persistierendem Verlauf (refraktives Asthma, bronchiales Asthma, allergisches Asthma, intrinsisches Asthma, extrinsisches Asthma, durch Medikamente oder durch Staub induziertes Asthma), von verschiedenen Formen der Bronchitis (chronische Bronchitis, infektiöse Bronchitis, eosinophile Bronchitis), von Bronchiolitis obliterans, Bronchiektasie, Pneumonie, Farmerlunge und verwandten Krankheiten, Husten- und Erkältungskrankheiten (chronischer entzündlicher Husten, iatrogener Husten), Nasenschleimhautentzündungen (einschließlich medikamentöse Rhinitis, vasomotorische Rhinitis und jahreszeitabhängige, allergische Rhinitis, z.B. Heuschnupfen) und von Polypen.

Darüber hinaus können die erfindungsgemäßen Verbindungen auch eingesetzt werden zur Behandlung und/oder Prävention von mikro- und makrovaskulären Schädigungen (Vasculitis), Reperfusionsschäden, arteriellen sowie venösen Thrombosen, diabetischer und nicht-diabetischer Nephropathie, der Glomerulonephritis, der Glomerulosklerose, des nephrotischen Syndroms, der hypertensiven Nephrosklerose, der Mikroalbuminurie, der akuten und chronischen Niereninsuffizienz, des akuten und chronischen Nierenversagens, Cystitis, Urethritis, Prostatitis, Epidymititis, Oophoritis, Salpingitis, Vulvovaginitis, erektiler Dysfunktion, Hunner-Geschwür, Peyronie-Krankheit, arterieller Hypertonie, Schock, atrialen und ventrikulären Arrhythmien, transitorischen und ischämischen Attacken, Herzmuskelschwäche, Hirnschlag, endothelialer Dysfunktion, peripheren und kardialen Gefäßerkrankungen, peripheren Durchblutungsstörungen, Ödembildung wie zum Beispiel pulmonales Ödem, Himödern, renales Ödem und Herzinsuffizienz-bedingtes Ödem, Restenosen wie nach Thrombolysetherapien, percutan-transluminalen Angioplastien (PTA), transluminalen Koronarangioplastien (PTCA), Herztransplantationen und Bypass-Operationen, bei erhöhtem Spiegel von Fibrinogen und von LDL geringer Dichte sowie bei erhöhten Konzentrationen von Plasminogenaktivator-Inhibitor 1 (PAI-1), von Dyslipidämien (Hypercholesterolämie, Hypertriglyceridämie, erhöhte Konzentrationen der postprandialen Plasma-Triglyceride, Hypoalphalipoproteinämie, kombinierte Hyperlipidämien) sowie metabolischen Erkrankungen (Metabolisches Syndrom, Hyperglykämie, Insulin-abhängiger Diabetes, Nicht-Insulin-abhängiger Diabetes, Gestationsdiabetes, Hyperinsulinämie, Insulinresistenz, Glukose-Intoleranz, Fettsucht (Adipositas) und diabetische Spätfolgen wie Retinopathie, Nephropathie und Neuropathie), Krebserkrankungen (Hautkrebs, Hirntumore, Brustkrebs, Knochenmarktumore, Leukämien, Liposarcome, Karzinome des Magen-Darm-Traktes, der Leber, Bauchspeicheldrüse, Lunge, Niere, Harnleiter, Prostata und des Genitaltraktes sowie bösartige Tumore des lymphoproliferativen Systems wie z.B. Hodgkin's und Non-Hodgkin's Lymphom), von Erkrankungen des Gastrointestinaltraktes und des Abdomen (Glossitis, Gingivitis, Periodontitis, Oesophagitis, eosinophile Gastroenteritis, Mastocytose, Morbus Crohn, Colitis, Proctitis, Pruritis ani, Diarrhöe, Zöliakie, Hepatitis, Leberfibrose, Leberzirrhose, Pankreatitis und Cholecystitis), von Erkrankungen des Zentralen Nervensystems und neurodegenerativen Störungen (Schlaganfall, Alzheimer'sche Krankheit, Parkinson'sche Krankheit, Demenz, Epilepsie, Depressionen, Multiple Sklerose), Immunerkrankungen, Schilddrüsenerkrankungen (Hyperthyreose), Hauterkrankungen (Psoriasis, Akne, Ekzeme, Neurodermitis, vielfältige Formen der Dermatitis wie z.B. Dermatitis abacribus, Dermatitis actinica, Dermatitis allergica, Dermatitis ammoniacalis, Dermatitis artefacta, Dermatitis autogenica, Dermatitis atrophicans, Dermatitis calorica, Dermatitis combustionis, Dermatitis congelationis, Dermatitis cosmetica, Dermatitis escharotica, Dermatitis exfoliativa, Dermatitis gangraenose, Dermatitis haemostatica, Dermatitis herpetiformis, Dermatitis lichenoides, Dermatitis linearis, Dermatitis maligna, Dermatitis medimencatosa, Dermatitis palmaris et plantaris, Dermatitis parasitaria, Dermatitis photoallergica, Dermatitis phototoxica, Dermatitis pustularis, Dermatitis seborrhoica, Dermatitis solaris, Dermatitis toxica, Dermatitis ulcerosa, Dermatitis veneata, infektiöse Dermatitis, pyogene Dermatitis und Rosazea-artige Dermatitis, sowie Keratitis, Bullosis, Vasculitis, Cellulitis, Panniculitis, Lupus erythematodes, Erythema, Lymphome, Hautkrebs, Sweet-Syndrom, Weber-Christian-Syndrom, Narbenbildung, Warzenbildung, Frostbeulen), von entzündlichen Augenerkrankungen (Saccoidosis, Blepharitis, Conjunctivitis, Iritis, Uveitis, Chorioiditis, Ophthalmitis), viralen Erkrankungen (durch Influenza-, Adeno- und Coronaviren, wie z.B. HPV, HCMV, HIV, SARS), von Erkrankungen des Skelettknochens und der Gelenke sowie der Skelettmuskel (vielfältige Formen der Arthritis wie z.B. Arthritis alcaptonurica, Arthritis ankylosans, Arthritis dysenterica, Arthritis exsudativa, Arthritis fungosa, Arthritis gonorrhoica, Arthritis mutilans, Arthritis psoriatica, Arthritis purulenta, Arthritis rheumatica, Arthritis serosa, Arthritis syphilitica, Arthritis tuberculosa, Arthritis urica, Arthritis villonodularis pigmentosa, atypische Arthritis, hämophile Arthritis, juvenile chronische Arthritis, rheumatoide Arthritis und metastatische Arthritis, des weiteren das Still-Syndrom, Felty-Syndrom, Sjörgen-Syndrom, Clutton-Syndrom, Poncet-Syndrom, Pott-Syndrom und Reiter-Syndrom, vielfältige Formen der Arthropathien wie z.B. Arthropathie deformans, Arthropathie neuropathica, Arthropathie ovaripriva, Arthropathie psoriatica und Arthropathie tabica, systemische Sklerosen, vielfältige Formen der entzündlichen Myopathien wie z.B. Myopathie epidemica, Myopathie fibrosa, Myopathie myoglobinurica, Myopathie ossificans, Myopathie ossificans neurotica, Myopathie ossificans progressiva multiplex, Myopathie purulenta, Myopathie rheumatica, Myopathie trichinosa, Myopathie tropica und Myopathie typhosa, sowie das Günther-Syndrom und das Münchmeyer-Syndrom), von entzündlichen Arterienveränderungen (vielfältige Formen der Arteritis wie z.B. Endarteritis, Mesarteritis, Periarteritis, Panarteritis, Arteritis rheumatica, Arteritis deformans, Arteritis temporalis, Arteritis cranialis, Arteritis gigantocellularis und Arteritis granulomatosa, sowie das Horton-Syndrom, Churg-Strauss-Syndrom und die Takayasu-Arteritis), des Muckle-Well-Syndroms, der Kikuchi-Krankheit, von Polychondritis, Sklerodermia sowie von weiteren Erkrankungen mit einer entzündlichen oder immunologischen Komponente, wie beispielsweise Katarakt, Kachexie, Osteoporose, Gicht, Inkontinenz, Lepra, Sezary-Syndrom und paraneoplastisches Syndrom, bei Abstossungsreaktionen nach Organtransplantationen und zur Wundheilung und Angiogenese insbesondere bei chronischen Wunden.

Aufgrund ihres Eigenschaftsprofils eignen sich die erfindungsgemäßen Verbindungen insbesondere zur Behandlung und/oder Prävention von pulmonaler arterieller Hypertonie (PAH) und anderen Formen der pulmonalen Hypertonie (PH), chronisch-obstruktiven Lungenerkrankungen (COPD), akuter Lungenschädigung (ALI), akutem Atemwegssyndrom (ARDS), Bronchiektasie, Bronchiolitis obliterans, Lungenemphysem, alpha-1-Antitrypsin-Defizienz (AATD), zystischer Fibrose (CF), Sepsis und systemisch-inflammatorischem Response-Syndrom (SIRS), multiplem Organversagen (MOF, MODS), entzündlichen Darmerkrankungen (IBD, Morbus Crohn, Colitis), chronischer Bronchitis, Asthma, Rhinitis, rheumatoider Arthritis, entzündlichen Haut- und Augenkrankheiten, Arteriosklerose und Krebserkrankungen.

Weiterer Gegenstand der vorliegenden Erfindung ist die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen in einem Verfahren zur Behandlung und/oder Prävention von Erkrankungen, insbesondere der zuvor genannten Erkrankungen.

Weiterer Gegenstand der vorliegenden Erfindung ist ein Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfindung sind daher Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Als geeignete Kombinationswirkstoffe seien beispielhaft und vorzugsweise genannt:
- die Signaltransduktionskaskade inhibierende Verbindungen, beispielhaft und vorzugsweise aus der Gruppe der Kinase-Inhibitoren, insbesondere aus der Gruppe der Tyrosinkinase- und/oder Serin/Threoninkinase-Inhibitoren;
- Verbindungen, die den Ab- und Umbau der Extrazellulärmatrix inhibieren, beispielhaft und vorzugsweise Inhibitoren der Matrix-Metalloproteasen (MMPs), insbesondere Inhibitoren von Stromelysin, Kollagenasen, Gelatinasen und Aggrecanasen (hierbei vor allem von MMP-1, MMP-3, MMP-8, MMP-9, MMP-10, MMP-11 und MMP-13) sowie der Metallo-Elastase (MMP-12);
- Verbindungen, die die Bindung von Serotonin an dessen Rezeptor blockieren, beispielhaft und vorzugsweise Antagonisten des 5-HT_{2b}-Rezeptors;
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- NO-unabhängige, jedoch Häm-abhängige Stimulatoren der löslichen Guanylatcyclase, wie insbesondere die in WO 00/06568, WO 00/06569, WO 02/42301 und WO 03/095451 beschriebenen Verbindungen;
- NO- und Häm-unabhängige Aktivatoren der löslichen Guanylatcyclase, wie insbesondere die in WO 01/19355, WO 01/19776, WO 01/19778, WO 01/19780, WO 02/070462 und WO 02/070510 beschriebenen Verbindungen;
- Prostacyclin-Analoga, wie beispielhaft und vorzugsweise Iloprost, Beraprost, Treprostinil oder Epoprostenol;
- Verbindungen, die die lösliche Epoxidhydrolase (sEH) inhibieren, wie beispielsweise N,N'-Dicyclohexylharnstoff, 12-(3-Adamantan-1-yl-ureido)-dodecansäure oder 1-Adamantan-1-yl-3-{5-[2-(2-ethoxyethoxy)ethoxy]pentyl}-harnstoff;
- den Energiestoffwechsel des Herzens beeinflussende Verbindungen, wie beispielhaft und vorzugsweise Etomoxir, Dichloracetat, Ranolazine oder Trimetazidine;
- Verbindungen, die den Abbau von cyclischem Guanosinmonophosphat (cGMP) und/oder cyclischem Adenosinmonophosphat (cAMP) inhibieren, wie beispielsweise Inhibitoren der Phosphodiesterasen (PDE) 1, 2, 3, 4 und/oder 5, insbesondere PDE 5-Inhibitoren wie Sildenafil, Vardenafil und Tadalafil;
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin All-Antagonisten, ACE-Inhibitoren, Vasopeptidase-Inhibitoren, Endothelin-Antagonisten, Renin-Inhibitoren, alpha-Rezeptoren-Blocker, beta-Rezeptoren-Blocker, Mineralocorticoid-Rezeptor-Antagonisten, Rho-Kinase-Inhibitoren sowie der Diuretika;
- bronchodilatorisch wirkende Mittel, beispielhaft und vorzugsweise aus der Gruppe der betaadrenergen Rezeptor-Agonisten, wie insbesondere Albuterol, Isoproterenol, Metaproterenol, Terbutalin, Formoterol oder Salmeterol, oder aus der Gruppe der Anticholinergika, wie insbesondere Ipratropiumbromid;
- anti-inflammatorisch wirkende Mittel, beispielhaft und vorzugsweise aus der Gruppe der Glucocorticoide, wie insbesondere Prednison, Prednisolon, Methylprednisolon, Triamcinolon, Dexamethason, Beclomethason, Betamethason, Flunisolid, Budesonid oder Fluticason; und/ oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Bei einer bevorzugten Ausführungsform der Erfindung werden die erfindungsgemäßen Verbindungen in Kombination mit einem Kinase-Inhibitor, wie beispielhaft und vorzugsweise Bortezomib, Canertinib, Erlotinib, Gefitinib, Imatinib, Lapatinib, Lestaurtinib, Lonafamib, Pegaptinib, Pelitinib, Semaxanib, Sorafenib, Sunitinib, Tandutinib, Tipifamib, Vatalanib, Fasudil, Lonidamin, Leflunomid, BMS-3354825 oder Y-27632, eingesetzt.

Bei einer bevorzugten Ausführungsform der Erfindung werden die erfindungsgemäßen Verbindungen in Kombination mit einem Serotonin-Rezeptor-Antagonisten, wie beispielhaft und vorzugsweise PRX-08066, eingesetzt.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban, DU-176b, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten, Rho-Kinase-Inhibitoren sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Inhibitor, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Rho-Kinase-Inhibitor, wie beispielhaft und vorzugsweise Fasudil, Y-27632, SLx-2119, BF-66851, BF-66852, BF-66853, KI-23095, SB-772077, GSK-269962A oder BA-1049, verabreicht.

Bei einer bevorzugten Ausführungsform der Erfindung werden die erfindungsgemäßen Verbindungen in Kombination mit einem Diuretikum, wie beispielhaft und vorzugsweise Furosemid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Torcetrapib (CP-529 414), JJT-705 oder CETP-vaccine (Avant), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin, Cerivastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nicht-toxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. inhalativ, intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer, Aerosole), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale, die intravenöse und die inhalative Applikation.

Die erfindungsgemäßen Verbindungen können in die angeführten Applikationsformen überführt werden. Dies kann in an sich bekannter Weise durch Mischen mit inerten, nicht-toxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich, sofern nicht anders angegeben, jeweils auf das Volumen.

### A. Beispiele

### Abkürzungen und Akronyme:

- aq.: wässrig, wässrige Lösung
- c: Konzentration
- cat.: katalytisch
- CDI: *N,N'*-Carbonyldiimidazol
- DC: Dünnschichtchromatographie
- DCI: direkte chemische Ionisation (bei MS)
- dest.: destilliert
- DIEA: *N,N*-Diisopropylethylamin
- DMAP: 4-*N,N*-Dimethylaminopyridin
- DMF: Dimethylformamid
- DMSO: Dimethylsulfoxid
- d. Th.: der Theorie (bei Ausbeute)
- EDC: *N'*-(3-Dimethylaminopropyl)-*N*-ethylcarbodiimid-Hydrochlorid
- ee: Enantiomerenüberschuss
- ent: enantiomerenrein, Enantiomer
- eq.: Äquivalent(e)
- ESI: Elektrospray-Ionisation (bei MS)
- Et: Ethyl
- GC-MS: Gaschromatographie-gekoppelte Massenspektrometrie
- h: Stunde(n)
- HATU: *O*-(7-Azabenzotriazol-1-yl)-*N,N,N,N'*-tetramethyluronium-Hexafluorophosphat
- HOBt: 1-Hydroxy-1*H*-benzotriazol-Hydrat
- HPLC: Hochdruck-, Hochleistungsflüssigchromatographie
- konz.: konzentriert
- LC-MS: Flüssigchromatographie-gekoppelte Massenspektrometrie
- MCPBA: *meta*-Chlorperbenzoesäure
- Me: Methyl
- min: Minute(n)
- MPLC: Mitteldruckflüssigchromatographie
- MS: Massenspektrometrie
- MTBE: Methyl-*tert*.-butylether
- NMR: Kemresonanzspektrometrie
- Ph: Phenyl
- PyBOP: Benzotriazol-1-yloxy-tris(pyrrolidino)phosphonium-hexafluorophosphat
- quant.: quantitativ (bei Ausbeute)
- rac: racemisch, Racemat
- RF: unter Rückfluss, Rückflusstemperatur
- RT: Raumtemperatur
- Rₜ: Retentionszeit (bei HPLC)
- Schmp.: Schmelzpunkt
- tBu: *tert*.-Butyl
- TFA: Trifluoressigsäure
- TFAA: Trifluoressigsäureanhydrid
- THF: Tetrahydrofuran
- UV: Ultraviolett-Spektrometrie
- v/v: Volumen zu Volumen-Verhältnis (einer Lösung)

### HPLC-, LC-MS- und GC-MS-Methoden:

### Methode 1 (LC-MS):

Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 2 (LC-MS):

Gerätetyp MS: Micromass ZQ; Gerätetyp HPLC: HP 1100 Series; UV DAD; Säule: Phenomenex Gemini 3µ 30 mm x 3.0 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 3 (LC-MS):

Instrument: Micromass Quattro LCZ mit HPLC Agilent Serie 1100; Säule: Phenomenex Onyx Monolithic C18, 100 mm x 3 mm; Eluent A: 1 1 Wasser + 0.5 ml 50%-ige Ameisensäure, Eluent B: 11 Acetonitril + 0.5 ml 50%-ige Ameisensäure; Gradient: 0.0 min 90% A → 2 min 65% A → 4.5 min 5% A → 6 min 5% A; Fluss: 2 ml/min; Ofen: 40°C; UV-Detektion: 208-400 nm.

### Methode 4 (LC-MS):

Gerätetyp MS: Waters ZQ; Gerätetyp HPLC: Waters Alliance 2795; Säule: Phenomenex Onyx Monolithic C18, 100 mm x 3 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2 min 65% A → 4.5 min 5% A → 6 min 5% A; Fluss: 2 ml/min; Ofen: 40°C; UV-Detektion: 210 nm.

### Methode 5 (LC-MS):

Instrument: Micromass QuattroPremier mit Waters UPLC Acquity; Säule: Thermo Hypersil GOLD 1.9µ 50 mm x 1 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 0.1 min 90% A → 1.5 min 10% A → 2.2 min 10% A; Fluss: 0.33 ml/min; Ofen: 50°C; UV-Detektion: 210 nm.

### Methode 6 (LC-MS):

Gerätetyp MS: Micromass ZQ; Gerätetyp HPLC: Waters Alliance 2795; Säule: Merck Chromolith SpeedROD RP-18e 100 mm x 4.6 mm; Eluent A: Wasser + 500 µl 50%-ige Ameisensäure / Liter; Eluent B: Acetonitril + 500 µl 50%-ige Ameisensäure / Liter; Gradient: 0.0 min 10% B → 7.0 min 95% B → 9.0 min 95% B; Fluss: 0.0 min 1.0 ml/min → 7.0 min 2.0 ml/min → 9.0 min 2.0 ml/min; Ofen: 35°C; UV-Detektion: 210 nm.

### Methode 7 (LC-MS):

Gerätetyp MS: Micromass ZQ; Gerätetyp HPLC: Waters Alliance 2795; Säule: Phenomenex Synergi 2.5µ MAX-RP 100A Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 0.1 min 90% A → 3.0 min 5% A → 4.0 min 5% A → 4.01 min 90% A; Fluss: 2 ml/min; Ofen: 50°C; UV-Detektion: 210 nm.

### Methode 8 (analytische HPLC):

Instrument: HP 1100 mit DAD-Detektion; Säule: Kromasil 100 RP-18, 60 mm x 2.1 mm, 3.5 µm; Eluent A: 5 ml HClO₄ (70%-ig) / L Wasser, Eluent B: Acetonitril; Gradient: 0 min 2% B → 0.5 min 2% B → 4.5 min 90% B → 9 min 90% B → 9.2 min 2% B → 10 min 2% B; Fluss: 0.75 ml/min; Säulentemperatur: 30°C; UV-Detektion: 210 nm.

### Methode 9 (analytische HPLC):

Instrument: HP 1100 mit DAD-Detektion; Säule: Kromasil 100 RP-18, 60 mm x 2.1 mm, 3.5 µm; Eluent A: 5 ml HClO₄ (70%-ig) / L Wasser, Eluent B: Acetonitril; Gradient: 0 min 2% B → 0.5 min 2% B → 4.5 min 90% B → 6.5 min 90% B → 6.7 min 2% B → 7.5 min 2% B; Fluss: 0.75 ml/min; Säulentemperatur: 30°C; UV-Detektion: 210 nm.

### Methode 10 (präparative HPLC):

Gerät: Abimed Gilson Pump 305/306, Manometric Module 806; Säule: GromSil C18, 250 mm x 30 mm, 10 µm; Eluent A: Wasser + 0.1% Trifluoressigsäure, Eluent B: Acetonitril; Gradient: 0-3 min 10% B, Rampe 3.01-34 min 95% B, 34.01-38 min 95% B, 38.01-40 min 10% B; Fluss: 50 ml/min; UV-Detektion: 210 nm.

### Methode 11 (präparative HPLC):

Gerät: Abimed Gilson Pump 305/306, Manometric Module 806; Säule: GromSil 120 ODS-4HE, 250 mm x 40 mm, 10 µm; Eluent A: Wasser + 0.1% Trifluoressigsäure, Eluent B: Acetonitril; Gradient: 0-3 min 30% B, Rampe 3.01-40 min 95% B, 40.01-50 min 95% B, 50.01-55 min 30% B; Fluss: 50 ml/min; UV-Detektion: 210 nm.

### Methode 12 (präparative HPLC):

Gerät: Abimed Gilson Syringe Pump 402, Gilson 231XL Autosampler, Gilson Fraction Collector; Software: Gilson UniPoint 2.10; Säule: Kromasil C18, 125 mm x 20 mm, 5 µm, 100 Å; Eluent A: Wasser + 0.0 1 % Ameisensäure, Eluent B: Acetonitril; Gradient: 0 min 10% B → 2 min 10% B → 9 min 90% B → 12 min 90% B → 12.1 min 10% B → 15 min 10% B; Fluss: 0.35 ml/min; UV-Detektion: 254 nm.

### Methode 13 (präparative HPLC):

Säule: Gemini C 18, 5 µm, 250 mm x 21.2 mm (Fa. Phenomenex); Eluent: Wasser/Acetonitril 2:3 (v/v); Fluss: 25 ml/min; Temperatur: 30°C; UV-Detektion: 210 nm.

### Methode 14 (präparative HPLC):

Säule: XBridge C 18, 5 µm OBD, 150 mm x 19 mm (Fa. Waters); Eluent: Wasser mit 0.1% Diethylamin/Acetonitril 3:2 (v/v); Fluss: 25 ml/min; Temperatur: 30°C; UV-Detektion: 235 nm.

### Methode 15 (präparative HPLC):

Säule: Sunfire C18 OBD, 5 µm, 250 mm x 20 mm; Eluent: Wasser mit 0.2% Trifluoressigsäure/ Acetonitril 6:4 (v/v); Fluss: 25 ml/min; Temperatur: 24°C; UV-Detektion: 210 nm.

### Methode 16 (GC-MS):

Instrument: Micromass GCT, GC 6890; Säule: Restek RTX-35, 15 m x 200 µm x 0.33 µm; konstanter Fluss mit Helium: 0.88 ml/min; Ofen: 70°C; Inlet: 250°C; Gradient: 70°C, 30°C/min → 310°C (3 min halten).

**Ausgangsverbindungen und Intermediate:**

### Beispiel 1A

### 4-Methyl-3-(methylsulfanyl)benzonitril

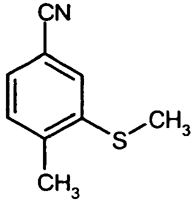

### Methode A:

Die Reaktion wurde unter Argon durchgeführt. 3-Fluor-4-methylbenzonitril (3000 mg, 22.2 mmol) und Natriummethanthiolat (1572 mg, 20.2 mmol) wurden in DMF (30 ml) vorgelegt, mit Kaliumcarbonat (6973 mg, 50.5 mmol) versetzt und über Nacht unter Rückfluss gerührt. Der Ansatz wurde danach eingeengt, der Rückstand mit Methylenchlorid/Methanol (10:1) aufgeschlämmt und das darin unlösliche Kaliumcarbonat abfiltriert. Das Filtrat wurde wieder eingeengt und der Rückstand an Kieselgel chromatographiert (Laufmittel: Cyclohexan/Essigsäureethylester 10:1). Man erhielt 2.51 g (64% d. Th.) der gewünschten Verbindung.

### Methode B:

Die Reaktion wurde unter Zuhilfenahme eines Chlorlaugenwäschers durchgeführt. 3-Fluor-4-methylbenzonitril (200 g, 1479.9 mmol) wurde in DMF (1.5 Liter) vorgelegt, auf 40°C erwärmt und portionsweise (je ca. 25 g) mit Natriummethanthiolat (insgesamt 126.8 g, 1627.9 mmol) versetzt. Während der Zugabe stieg die Temperatur auf 100°C an. Das Reaktionsgemisch wurde zunächst 1.5 h bei 175°C Badtemperatur und dann über Nacht bei Raumtemperatur gerührt. Der Ansatz wurde danach auf Wasser (7.5 Liter) gegossen und zweimal mit Essigsäureethylester (jeweils 1875 ml) extrahiert. Die vereinigten organischen Phasen wurden mit gesättigter Natriumchlorid-Lösung (1875 ml) gewaschen, am Rotationsverdampfer eingeengt und der Rückstand an Kieselgel chromatographiert (Laufmittel: Petrolether/Essigsäureethylester 95:5, ca. 30 Liter). Nach Abziehen des Lösungsmittels am Rotationsverdampfer und Trocknen im Hochvakuum erhielt man 172 g (71% d. Th.) der gewünschten Verbindung.
GC-MS (Methode 16): Rₜ = 5.25 min; MS (ESIpos): *m*/*z* (%) = 163.0 (100) [M]⁺
¹H-NMR (400 MHz, DMSO-d₆): δ = 2.30 (s, 3H), 2.54 (s, 3H), 7.38 (d, 1H), 7.52 (dd, 1H), 7.58 (br. s, 1H).

### Beispiel 2A

### 4-Methyl-3-(methylsulfonyl)benzonitril

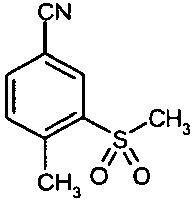

### Methode A:

4-Methyl-3-(methylsulfanyl)benzonitril (14050 mg, 80.1 mmol; Beispiel 1A) wurde in Dichlormethan (700 ml) gelöst, auf 0°C gekühlt und langsam mit 3-Chlorperbenzoesäure (50923 mg, 206.6 mmol) versetzt. Anschließend wurde zunächst 40 min bei 0°C und dann über Nacht bei Raumtemperatur gerührt. Die ausgefallene 3-Chlorbenzoesäure wurde abfiltriert, das Filtrat mit 1 N Natronlauge gewaschen, die organische Phase über Natriumsulfat getrocknet und eingeengt. Der Rückstand wurde mittels Kieselgel-Chromatographie gereinigt (Laufmittel: Cyclohexan/Essigsäureethylester 1:1, 1:2). Man erhielt 13.65 g (8 1 % d. Th.) der gewünschten Verbindung.

### Methode B:

3-Chlorperbenzoesäure (2501 g, 10144.4 mmol) wurde in 27.2 Liter Dichlormethan gelöst, auf 10°C gekühlt und portionsweise mit 4-Methyl-3-(methylsulfanyl)benzonitril (552 g, 3381.5 mmol; Beispiel 1A) versetzt. Nach vollständiger Zugabe wurde 5 h bei RT gerührt. Die ausgefallene 3-Chlorbenzoesäure wurde abgesaugt und der Feststoff mit Dichlormethan (3 Liter) nachgewaschen. Die vereinigten Filtrate wurden mit 1 N Natronlauge (15 Liter) gerührt, das Gemisch filtriert und die organische Phase abgetrennt. Diese wurde nochmals mit 1 N Natronlauge (15 Liter) gerührt, von der Natronlauge abgetrennt, getrocknet und am Rotationsverdampfer eingeengt. Der Rückstand wurde in Diethylether (4 Liter) aufgeschlämmt, 10 min gerührt und dann filtriert. Der Feststoff wurde mit etwas Diethylether nachgewaschen und im Hochvakuum getrocknet. Man erhielt 613 g (93% d. Th.) der gewünschten Verbindung.
GC-MS (Methode 16): Rₜ = 6.59 min; MS (ESIpos): *m*/*z* (%) = 195.0 (100) [M]⁺
¹H-NMR (400 MHz, DMSO-d₆): δ = 2.30 (s, 3H), 2.54 (s, 3H), 7.38 (d, 1H), 7.52 (dd, 1H), 7.58 (br. s, 1H).

### Beispiel 3A

### 4-[2-(Dimethylamino)ethenyl]-3-(methylsulfonyl)benzonitril

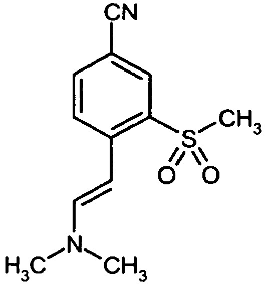

### Methode A:

Die Reaktion wurde unter Argon durchgeführt. 4-Methyl-3-(methylsulfonyl)benzonitril (13.0 g, 66.6 mmol; Beispiel 2A) und 1,1-Dimethoxy-*N,N*-dimethylmethanamin (10.315 g, 86.6 mmol) wurden 14 h bei 140°C in DMF (200 ml) gerührt. Zur Vervollständigung der Reaktion wurde danach erneut 1,1-Dimethoxy-*N,N*-dimethylmethanamin (3.967 g, 33.3 mmol) zugegeben und weitere 24 h bei 140°C gerührt. Das DMF wurde dann am Rotationsverdampfer abgezogen und der Rückstand ohne weitere Aufreinigung in der Folgestufe umgesetzt.

### Methode B:

Die Reaktion wurde unter Argon durchgeführt. 4-Methyl-3-(methylsulfonyl)benzonitril (612 g, 3134.6 mmol; Beispiel 2A) wurde in DMF (6.12 Liter) vorgelegt, mit 1,1-Dimethoxy-*N,N*-di-methylmethanamin (859 g, 7209.5 mmol) versetzt und 7 h bei 140°C gerührt. Die Reaktionsmischung wurde dann auf 35 Liter 10%-ige Natriumchlorid-Lösung gegossen und zweimal mit jeweils 10 Liter Essigsäureethylester extrahiert. Die vereinigten organischen Phasen wurden mit gesättigter Natriumchlorid-Lösung (5 Liter) gewaschen, getrocknet, am Rotationsverdampfer eingeengt und der Rückstand über Nacht im Hochvakuum getrocknet. Auf diese Weise erhielt man 1098 g (98% d. Th.) der gewünschten Verbindung.
GC-MS (Methode 16): Rₜ = 8.95 min; MS (ESIpos): *m*/*z* (%) = 250.0 (10) [M]⁺.

### Beispiel 4A

### 4-Formyl-3-(methylsulfonyl)benzonitril

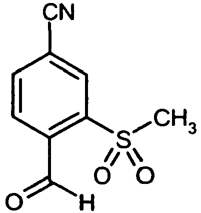

### Methode A:

4-[2-(Dimethylamino)ethenyl]-3-(methylsulfonyl)benzonitril (16666 mg, 66.6 mmol; Beispiel 3A) wurde in Wasser/THF (1:1, 500 ml) vorgelegt, mit Natriumperiodat (42722 mg, 199.7 mmol) versetzt und über Nacht bei Raumtemperatur gerührt. Der ausgefallene Feststoff wurde abfiltriert und mit Essigsäureethylester nachgewaschen. Die vereinigten organischen Phasen wurden mit gesättigter Natriumhydrogencarbonat- und gesättigter Natriumchlorid-Lösung gewaschen, über Natriumsulfat getrocknet, filtriert und eingeengt. Der Rückstand wurde per Kieselgel-Chromatographie gereinigt (Laufmittel: Cyclohexan/Essigsäureethylester 1:1). Man erhielt 4.6 g (33% d. Th.) der gewünschten Verbindung.

### Methode B:

4-[2-(Dimethylamino)ethenyl]-3-(methylsulfonyl)benzonitril (1098 g, 3070.5 mmol; Beispiel 3A) wurde in THF/Wasser (1:1, 13.8 Liter) vorgelegt, mit Natriumperiodat (1970 g, 9211.4 mmol) versetzt und 1 h bei Raumtemperatur gerührt. Der ausgefallene Feststoff wurde abgesaugt und mit Essigsäureethylester (17 Liter) nachgewaschen. Die vereinigten Filtrate wurden mit Wasser (17 Liter) versetzt, und nach erfolgter Extraktion wurde die wässrige Phase abgetrennt. Die organische Phase wurde mit gesättigter Natriumhydrogencarbonat-Lösung (8.5 Liter) und gesättigter Natriumchlorid-Lösung (8.5 Liter) gewaschen, dann getrocknet und am Rotationsverdampfer eingeengt. Die Reinigung des Rückstands erfolgte mittels Kieselgel-Chromatographie (Laufmittel: Dichlormethan/Essigsäureethylester 9:1, 60 Liter). Die Produktfraktionen wurden eingeengt, der Rückstand in Petrolether aufgeschlämmt, dann abgesaugt und der Feststoff im Hochvakuum über Nacht getrocknet. Auf diese Weise erhielt man 436 g (65% d. Th.) der gewünschten Verbindung.
GC-MS (Methode 16): Rₜ = 6.89 min; MS (ESIpos): *m*/*z* (%) = 191.1 (15) [M-18]⁺, 161.0 (100)
¹H-NMR (400 MHz, DMSO-d₆): δ = 3.57 (s, 3H), 8.10 (d, 1H), 8.39 (dd, 1H), 8.45 (d, 1H), 10.63 (s, 1H).

### Beispiel 5A

### Ethyl (4R)-4-(4-cyanophenyl)-6-methyl-2-oxo-1-[3-(trifluormethyl)phenyl]-1,2,3,4-tetrahydropyrimidin-5-carboxylat

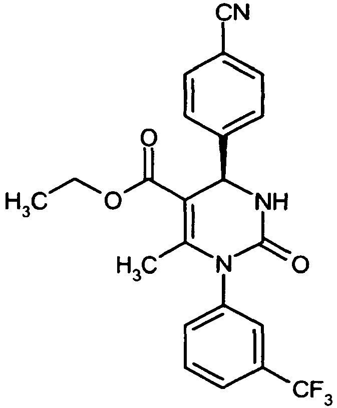

Die Titelverbindung wurde, wie in WO 2008/003412 (Beispiel 1) beschrieben, hergestellt.

### Beispiel 6A

### Ethyl (4R)-6-(brommethyl)-4-(4-cyanophenyl)-2-oxo-1-[3-(trifluormethyl)phenyl]-1,2,3,4-tetra-hydropyrimidin-5-carboxylat

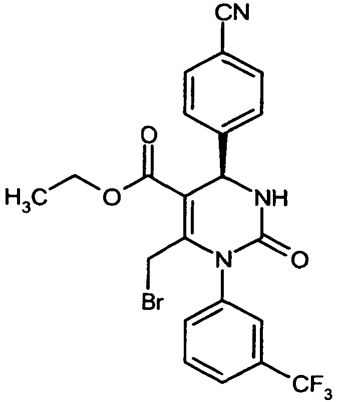

20 g (46.576 mmol) der unter Beispiel 5A beschriebenen Verbindung wurden in 350 ml Chloroform gelöst und bei 0°C mit 8.2 g (51.234 mmol) Brom versetzt. Das Eisbad wurde entfernt und der Ansatz eine Stunde gerührt. Das Gemisch wurde danach mit 10%-iger Natriumthiosulfat-Lösung gewaschen, die organische Phase abgetrennt und diese über Natriumsulfat getrocknet. Nach Filtration wurde die Lösung am Rotationsverdampfer eingeengt und der Rückstand mit Diethylether ausgerührt. Der Feststoff wurde abgesaugt und im Vakuum getrocknet. Man erhielt 21.1 g (87% d. Th.) der Zielverbindung.
LC-MS (Methode 1): Rₜ = 2.41 min; MS (ESIpos): *m*/*z* = 510 [M+H]⁺.
¹H-NMR-Daten der racemischen Verbindung siehe WO 2004/024700 (Beispiel 19).

### Beispiel 7A

### [(6R)-6-(4-Cyanophenyl)-5-(ethoxycarbonyl)-4-methyl-2-oxo-3-[3-(trifluormethyl)phenyl]-3,6-dihydropyrimidin-1(2H)-yl]essigsäure

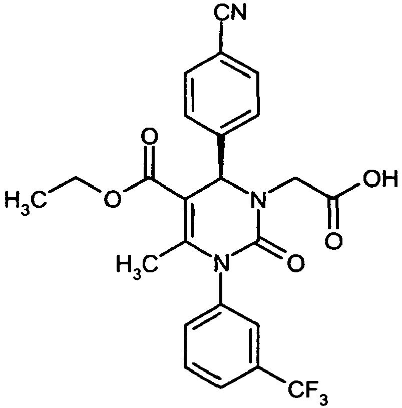

Die Titelverbindung wurde, wie in WO 2008/003412 (Beispiel 13) beschrieben, hergestellt.

### Beispiel 8A

### Ethyl (4R)-3-(2-amino-2-oxoethyl)-4-(4-cyanophenyl)-6-methyl-2-oxo-1-[3-(trifluormethyl)-phenyl]-1,2,3,4-tetrahydropyrimidin-5-carboxylat

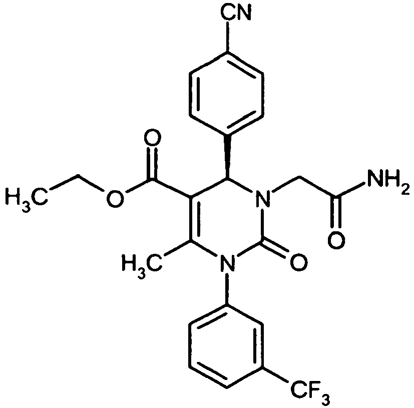

Die Titelverbindung wurde, wie in WO 2008/003412 (Beispiel 22) beschrieben, hergestellt.

### Beispiel 9A

### Ethyl (4R)-4-(4-cyanophenyl)-3-[2-(dimethylamino)-2-oxoethyl]-6-methyl-2-oxo-1-[3-(trifluormethyl)phenyl]-1,2,3,4-tetrahydropyrimidin-5-carboxylat

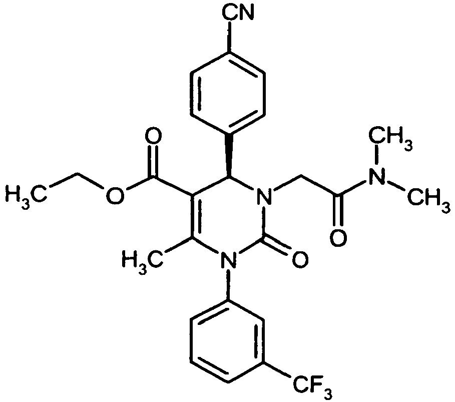

Die Titelverbindung wurde, wie in WO 2008/003412 (Beispiel 30) beschrieben, hergestellt.

### Beispiel 10A

### Ethyl (4R)-4-(4-cyanophenyl)-6-methyl-2-oxo-3-(2-oxo-2-pyrrolidin-1-ylethyl)-1-[3-(trifluormethyl)phenyl]-1,2,3,4-tetrahydropyrimidin-5-carboxylat

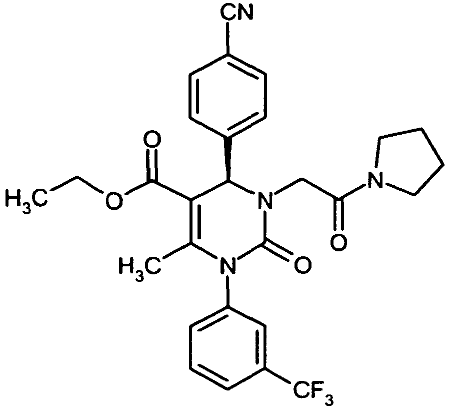

500 mg (1.026 mmol) der unter Beispiel 7A beschriebenen Verbindung wurden in 1.5 ml DMF gelöst und nacheinander mit 161 mg (2.257 mmol) Pyrrolidin, 251 mg (2.052 mmol) DMAP, 305 mg (2.257 mmol) HOBt sowie 393 mg (2.052 mmol) EDC versetzt. Das Gemisch wurde über Nacht bei Raumtemperatur gerührt und danach direkt, ohne weitere Aufarbeitung, mittels präparativer HPLC (Methode 10) gereinigt. Man erhielt 239 mg (41% d. Th.) der Zielverbindung.
LC-MS (Methode 2): Rₜ = 2.67 min; MS (ESIpos): *m*/*z* (%) = 541.2 (100) [M+H]⁺.
¹H-NMR (400 MHz, DMSO-d₆). δ = 1.12 (t, 3H), 1.74 (m, 2H), 1.84 (m, 2H), 2.04 (s, 3H), 3.29 (m, 4H), 3.58 (d, 1H), 4.05 (m, 2H), 4.35 (d, 1H), 5.53 (s, 1H), 7.61 (d, 1H), 7.67 (d, 2H), 7.72 (t, 2H), 7.81 (d, 1H), 7.88 (d, 2H).

Analog der Vorschrift für das Beispiel 10A wurde aus dem unter Beispiel 7A hergestellten Edukt und 2-(Methylamino)ethanol die Verbindung in der folgenden Tabelle hergestellt:

| **Beispiel** | **Struktur** | **Ausbeute** | **Analytische Daten** |
|---|---|---|---|
| **11A** | 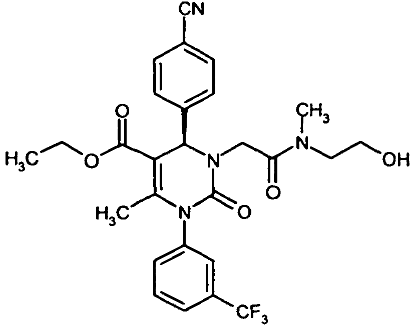 | 77% d. Th. | LC-MS (Methode 3): Rₜ = 3.46 min; MS (ESIpos): *m*/*z* = 545 [M+H]⁺. |

### Beispiel 12A

### Ethyl (4R)-3-(2-amino-2-oxoethyl)-6-(brommethyl)-4-(4-cyanophenyl)-2-oxo-1-[3-(trifluormethyl)phenyl]-1,2,3,4-tetrahydropyrimidin-5-carboxylat

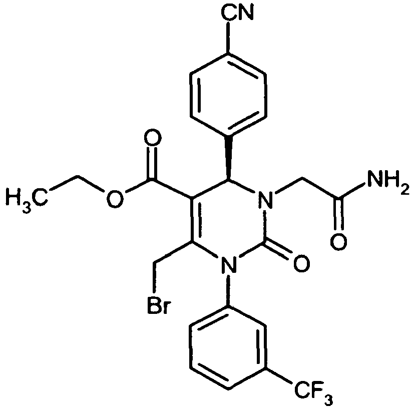

600 mg (1.233 mmol) der unter Beispiel 8A beschriebenen Verbindung wurden in 15 ml Chloroform gelöst und bei 0°C mit 197 mg (1.233 mmol) Brom versetzt. Das Eisbad wurde entfernt und der Ansatz eine Stunde gerührt. Das Gemisch wurde danach mit 10%-iger Natriumthiosulfat-Lösung gewaschen, die organische Phase abgetrennt und diese über Natriumsulfat getrocknet. Nach Filtration wurde die Lösung am Rotationsverdampfer eingeengt und der Rückstand mittels präparativer HPLC (Methode 11) gereinigt. Man erhielt 479 mg (69% d. Th.) der Zielverbindung.
LC-MS (Methode 4): Rₜ = 3.43 min; MS (ESIpos): *m*/*z* (%) = 567.1 (100) [M+H]⁺.
¹H-NMR (400 MHz, DMSO-d₆): δ = 1.16 (t, 3H), 3.41 (d, 1H), 4.11 (m, 3H), 4.27 (br. s, 1H), 4.46 (br. s, 1H), 5.58 (s, 1H), 7.13 (s, 1H), 7.43 (s, 1H), 7.66 (d, 2H), 7.75 (d, 2H), 7.81 (s, 1H), 7.87 (d, 1H), 7.92 (d, 2H).

Analog der Vorschrift für das Beispiel 12A wurden aus den angegebenen Edukten die Verbindungen in der folgenden Tabelle hergestellt:

| **Beispiel** | **Struktur** | **Edukt** | **Ausbeute** | **Analytische Daten** |
|---|---|---|---|---|
| **13A** | 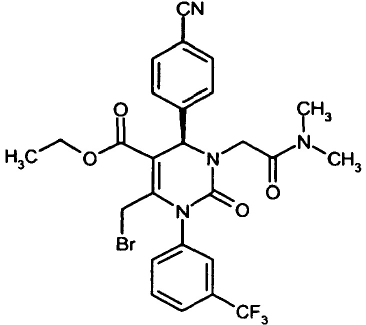 | **9A** | 75% d. Th. | LC-MS (Methode 2): Rₜ = 2.68 min; MS (ESIpos): *m*/*z* = 595 [M+H]⁺. |
| **14A** | 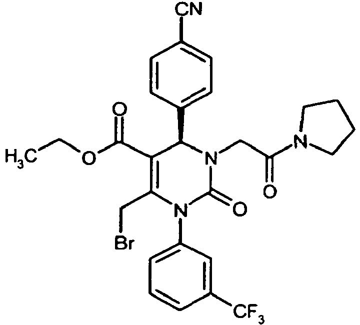 | **10A** | 39% d. Th. | LC-MS (Methode 3): Rₜ = 3.88 min; MS (ESIpos): *m*/*z* = 621 [M+H]⁺. |
| **15A** | 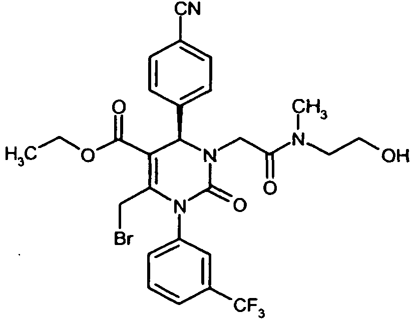 | **11A** | 65% d. Th. | LC-MS (Methode 4): Rₜ = 3.45 min; MS (ESIpos): *m*/*z* = 625 [M+H]⁺. |

### Beispiel 16A

### Ethyl (4R)-4-(4-cyanophenyl)-3-(2-methoxy-2-oxoethyl)-6-methyl-2-oxo-1-[3-(trifluormethyl)-phenyl]-1,2,3,4-tetrahydropyrimidin-5-carboxylat

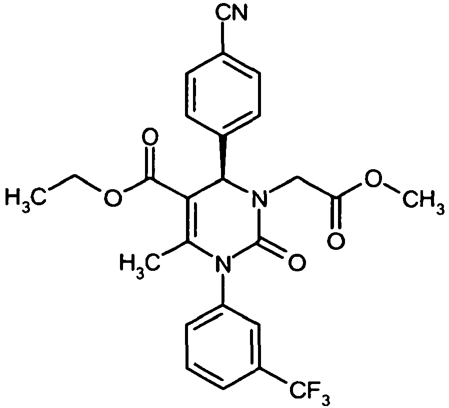

500 mg (1.164 mmol) der Verbindung aus Beispiel 5A wurden zusammen mit 220 mg (1.397 mmol) Bromessigsäuremethylester und 322 mg (2.329 mmol) Kaliumcarbonat in 20 ml DMF über Nacht bei 60°C gerührt. Der Feststoff wurde danach abfiltriert, das Filtrat am Rotationsverdampfer eingeengt, der Rückstand in Dichlormethan gelöst und diese Lösung dreimal mit Wasser gewaschen. Die organische Phase wurde abgetrennt, über Natriumsulfat getrocknet, filtriert und eingeengt. Das Rohprodukt wurde durch Chromatographie an Kieselgel gereinigt (Eluent Toluol/Essigsäureethylester 3:1). Man erhielt 419 mg (72% d. Th.) des gewünschten Produkts. LC-MS (Methode 4): Rₜ = 3.76 min; MS (ESIpos): *m*/*z* (%) = 502.1 (100) [M+H]⁺; MS (ESIneg): *m*/*z* (%) = 500.2 (100) [M-H]⁻.
¹H-NMR (400 MHz, DMSO-d₆): δ = 7.89 (d, 2H), 7.83 (d, 1H), 7.79 (s, 1H), 7.74 (t, 2H), 7.70 (s, 1H), 7.67 (d, 1H), 5.64 (s, 1H), 4.19 (d, 1H), 4.05 (m, 2H), 3.93 (d, 1H), 3.56 (s, 3H), 2.07 (s, 3H), 1.12 (t, 3H).

### Beispiel 17A

### Ethyl (4R)-6-(brommethyl)-4-(4-cyanophenyl)-3-(2-methoxy-2-oxoethyl)-2-oxo-1-[3-(trifluormethyl)phenyl]-1,2,3,4-tetrahydropyrimidin-5-carboxylat

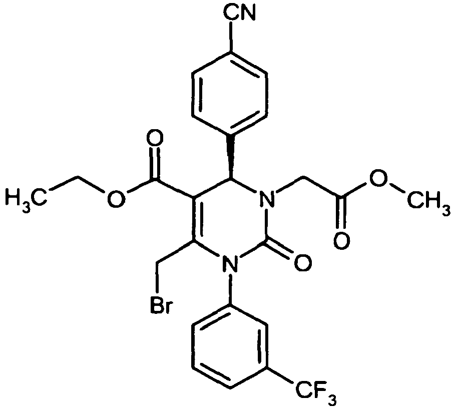

100 mg (0.199 mmol) der unter Beispiel 16A beschriebenen Verbindung wurden in 2 ml Chloroform gelöst und bei 0°C mit 35 mg (0.219 mmol) Brom versetzt. Nach 30 Minuten wurde das Eisbad entfernt und der Ansatz über Nacht weitergerührt. Das Gemisch wurde danach dreimal mit 10%-iger Natriumthiosulfat-Lösung gewaschen. Die vereinten wässrigen Phasen wurden mit Dichlormethan rückextrahiert. Die vereinigten organischen Phasen wurden über Natriumsulfat getrocknet, filtriert und im Vakuum vom Lösungsmittel befreit. Nach Reinigung des Rohprodukts mittels präparativer HPLC (Methode 10) erhielt man 65 mg (54% d. Th.) der Zielverbindung.
LC-MS (Methode 2): Rₜ = 2.83 min; MS (ESIpos): *m*/*z* (%) = 582.0 (100) [M+H]⁺.
¹H-NMR (400 MHz, DMSO-d₆): δ = 7.91 (d, 2H), 7.88 (d, 1H), 7.79 (br. s, 1H), 7.78-7.71 (m, 2H), 7.69 (d, 2H), 5.69 (s, 1H), 4.60 (br. d, 1H), 4.19 (d, 1H), 4.17-4.09 (m, 3H), 4.02 (d, 1H), 3.54 (s, 3H), 1.15 (t, 3H).

### Beispiel 18A

### (rac)-Ethyl 4-(4-cyano-2-(methylsulfonyl)phenyl)-6-methyl-2-oxo-1-[3-(trifluormethyl)phenyl]-1,2,3,4-tetrahydropyrimidin-5-carboxylat

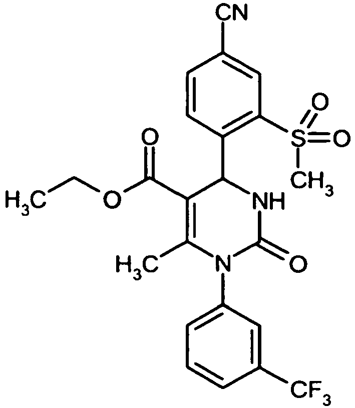

Die Reaktion wurde unter Argon durchgeführt. Phosphorsäuretriethylester (4.18 g, 22.9 mmol) und Diphosphorpentoxid (2.17 g, 15.3 mmol) wurden über Nacht bei 50°C gerührt. Dann wurde mit Methyl-*tert*.-butylether (60 ml) verdünnt, und 4-Formyl-3-(methylsulfonyl)benzonitril (4.00 g, 19.1 mmol; Beispiel 4A), 1-[3-(Trifluormethyl)phenyl]harnstoff (3.90 g, 19.1 mmol) sowie Acetessigsäureethylester (3.73 g, 28.7 mmol) wurden hinzugefügt. Das Gemisch wurde über Nacht unter Rückfluss gerührt. Der entstandene Niederschlag wurde abgesaugt und mit Diethylether (300 ml) gewaschen. Da die Umsetzung nicht vollständig verlaufen war, wurden nochmals Phosphorsäuretriethylester (5.36 g, 29.4 mmol) und Diphosphorpentoxid (2.71 g, 19.1 mmol) über Nacht bei 50°C gerührt und dann zusammen mit dem zuvor isolierten Feststoff sowie Methyl-*tert*.-butylether (25 ml) eine weitere Nacht unter Rückfluss gerührt. Der entstandene Niederschlag wurde abermals abgesaugt und mit Diethylether gewaschen. Man erhielt 5.93 g (61% d. Th.) der Zielverbindung.
HPLC (Methode 8): Rₜ = 4.56 min; MS (DCI/NH₃): *m*/*z* = 508.1 [M+H]⁺, 525 [M+NH₄]⁺
¹H-NMR (400 MHz, DMSO-d₆): δ = 0.94 (t, 3H), 2.13 (s, 3H), 3.50 (s, 3H), 3.89-4.02 (q, 2H), 6.41 (s, 1H), 7.25 (s, 1H), 7.68-7.90 (m, 4H), 8.09 (d, 1H), 8.26 (d, 1H), 8.39 (s, 1H).

### Beispiel 19A

### (rac)-Ethyl 6-(brommethyl)-4-[4-cyano-2-(methylsulfonyl)phenyl]-2-oxo-1-[3-(trifluormethyl)-phenyl]-1,2,3,4-tetrahydropyrimidin-5-carboxylat

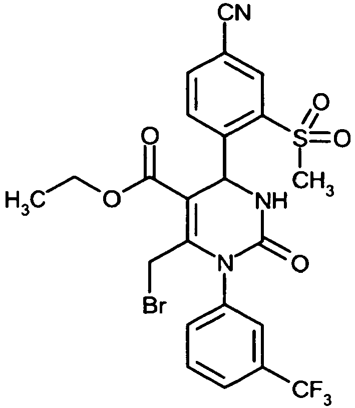

Die unter Beispiel 18A beschriebene Verbindung (3.00 g, 5.62 mmol) wurde in Chloroform (50 ml) gelöst und bei 0°C mit Brom (987 mg, 6.18 mmol) versetzt. Das Eisbad wurde entfernt und der Ansatz eine Stunde gerührt. Das Gemisch wurde danach mit 10%-iger Natriumthiosulfat-Lösung gewaschen, die organische Phase abgetrennt und diese über Natriumsulfat getrocknet. Nach Filtration wurde die Lösung am Rotationsverdampfer eingeengt und der Rückstand mit Diethylether ausgerührt. Der Feststoff wurde abgesaugt und im Vakuum getrocknet. Man erhielt 2.97 g (90% d. Th.) der Zielverbindung.
HPLC (Methode 8): Rₜ = 4.73 min; MS (DCI/NH₃): m/z = 586, 588 [M+H]⁺, 603, 605 [M+NH₄]⁺
¹H-NMR (400 MHz, DMSO-d₆): δ = 0.97 (t, 3H), 3.50 (s, 3H), 3.96-4.07 (q, 2H), 4.13-4.24 (d, 1H), 4.65-4.75 (d, 1H), 6.48 (d, 1H), 7.46 (d, 1H), 7.72-8.12 (m, 5H), 8.31 (d, 1H), 8.42 (s, 1H).

### Beispiel 20A

### (rac)-Ethyl 6-methyl-4-[4-cyano-2-(trifluormethyl)phenyl]-2-oxo-1-[3-(trifluormethyl)phenyl]-1,2,3,4-tetrahydropyrimidin-5-carboxylat

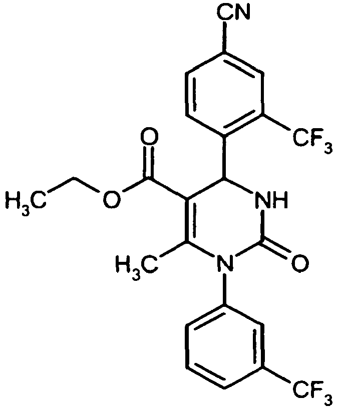

Die Reaktion wurde unter Argon durchgeführt. Zu einer Lösung von Polyphosphorsäureethylester (2.0 g; hergestellt aus Phosphorsäuretriethylester und Diphosphorpentoxid analog zum Verfahren in Beispiel 18A) in THF (25 ml) wurden nacheinander 4-Formyl-3-(trifluormethyl)benzonitril (996 mg, 5.0 mmol; zur Herstellung vgl. WO 98/37058), 1-[3-(Trifluormethyl)phenyl]harnstoff (1.02 g, 5.0 mmol) sowie Acetessigsäureethylester (651 mg, 5.0 mmol) hinzugegeben. Das Gemisch wurde 19 h unter Rückfluss gerührt und danach eingeengt. Der Rückstand wurde mit Ethylacetat (150 ml) versetzt und nacheinander mit Wasser (50 ml), gesättigter Natriumhydrogencarbonat-Lösung (50 ml) sowie gesättigter Natriumchlorid-Lösung (50 ml) gewaschen. Die organische Phase wurde über Natriumsulfat getrocknet und eingeengt. Der Rückstand wurde mittels Kieselgel-Chromatographie gereinigt (Laufmittel: Cyclohexan → Cyclohexan/Essigsäureethylester 2:1 ). Man erhielt 1.55 g (62% d. Th.) des Zielprodukts.
LC-MS (Methode 2): Rₜ = 2.81 min; MS (ESIpos): *m*/*z* (%) = 498 (100) [M+H]⁺; MS (ESIneg): *m*/*z* (%) = 453.0 (100), 469.2 (80) [M-H]⁻.
¹H-NMR (400 MHz, DMSO-d₆): δ = 0.85 (t, 3H), 2.10 (s, 3H), 3.90 (q, 2H), 5.75 (s, 1H), 7.70-7.90 (m, 4H), 8.00-8.15 (m, 2H), 8.20 (d, 1H), 8.30 (s, 1H).

### Beispiel 21A

### (rac)-Ethyl 6-(brommethyl)-4-[4-cyano-2-(trifluormethyl)phenyl]-2-oxo-1-[3-(trifluormethyl)-phenyl]-1,2,3,4-tetrahydropyrimidin-5-carboxylat

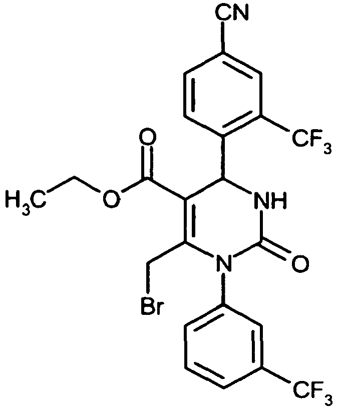

Die unter Beispiel 20A beschriebene Verbindung (497 mg, 1.0 mmol) wurde in Chloroform (10 ml) gelöst und bei 0°C mit Brom (176 mg, 1.10 mmol) versetzt. Das Eisbad wurde entfernt und der Ansatz für 1.5 h bei RT gerührt. Das Reaktionsgemisch wurde dann mit Dichlormethan (100 ml) versetzt und nacheinander mit 10%-iger wässriger Natriumthiosulfat-Lösung (70 ml) und konzentrierter wässriger Kochsalz-Lösung (50 ml) gewaschen. Die organische Phase wurde abgetrennt, über Natriumsulfat getrocknet, filtriert und am Rotationsverdampfer eingeengt. Man erhielt 735 mg (quant.) der Zielverbindung, welche ohne weitere Aufreinigung weiter umgesetzt wurde.
LC-MS (Methode 3): Rₜ = 4.08 min; MS (ESIpos): *m*/*z* (%) = 576 (40) [M+H]⁺.

### Beispiel 22A

### N-[4-Fluor-3-(trifluormethyl)phenyl]harnstoff

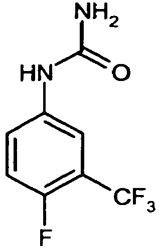

2500 mg (13.957 mmol) 4-Fluor-3-(trifluormethyl)anilin wurden in 15 ml 1N Salzsäure gelöst und mit 1132 mg (13.957 mmol) Kaliumcyanat versetzt. Die Suspension wurde über Nacht bei Raumtemperatur gerührt und dann mit Essigsäureethylester verdünnt, so dass eine klare zweiphasige Lösung entstand. Die organische Phase wurde abgetrennt und die wässrige mit Essigsäureethylester extrahiert. Nach Trocknen der vereinigten organischen Phasen und Abziehen des Lösungsmittels am Rotationsverdampfer wurde das Rohprodukt an Kieselgel chromatographiert (Eluent: Dichlormethan/Methanol 80:1, dann 10:1). Es wurden 2180 mg (70% d. Th.) der Zielverbindung erhalten.
LC-MS (Methode 2): Rₜ = 1.82 min; MS (ESIpos): *m*/*z* (%) = 223.0 (100) [M+H]⁺.

### Beispiel 23A

### 4-(4-Cyanophenyl)-1-[4-fluor-3-(trifluormethyl)phenyl]-6-methyl-2-oxo-1,2,3,4-tetrahydropyrimidin-5-carbonsäureethylester

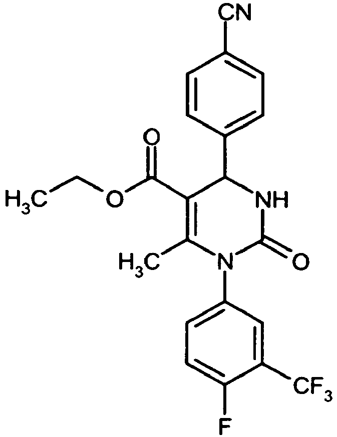

Die Reaktion wurde unter Argon durchgeführt. Zu einer Lösung von Polyphosphorsäureethylester (2.0 g; hergestellt aus Phosphorsäuretriethylester und Diphosphorpentoxid analog zum Verfahren in Beispiel 18A) in THF (25 ml) wurden nacheinander 4-Formylbenzonitril (656 mg, 5.0 mmol), 1-[4-Fluor-3-(trifluormethyl)phenyl]harnstoff (1.11 g, 5.0 mmol) sowie Acetessigsäureethylester (651 mg, 5.0 mmol) hinzugegeben. Das Gemisch wurde 19 h unter Rückfluss gerührt und danach eingeengt. Der Rückstand wurde mit Ethylacetat (150 ml) versetzt und nacheinander mit Wasser (50 ml), gesättigter Natriumhydrogencarbonat-Lösung (50 ml) sowie gesättigter Natriumchlorid-Lösung (50 ml) gewaschen. Die organische Phase wurde über Natriumsulfat getrocknet und eingeengt. Der Rückstand wurde mittels Kieselgel-Chromatographie gereinigt (Laufmittel: Cyclohexan → Cyclohexan/Essigsäureethylester 2:3). Man erhielt 1.40 g (63% d. Th.) des Zielprodukts.
LC-MS (Methode 1): Rₜ = 2.42 min; MS (ESIpos): *m*/*z* (%) = 448.2 (100) [M+H]⁺; MS (ESIneg): *m*/*z* (%) = 446.2 (100) [M-H]⁻.

### Beispiel 24A

### 4-(4-Cyanophenyl)-1-[4-fluor-3-(trifluormethyl)phenyl]-6-(brommethyl)-2-oxo-1,2,3,4-tetrahydropyrimidin-5-carbonsäureethylester

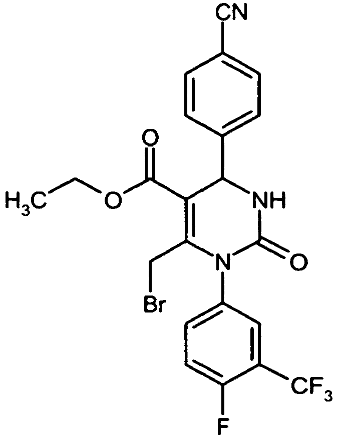

4-(4-Cyanophenyl)-1-[4-fluor-3-(trifluormethyl)phenyl]-6-methyl-2-oxo-1,2,3,4-tetrahydropyrimidin-5-carbonsäureethylester (447 mg, 1.0 mmol) wurde in Chloroform (10 ml) gelöst und bei 0°C mit Brom (176 mg, 1.10 mmol) versetzt. Das Eisbad wurde entfernt und der Ansatz für 0.5 h bei Raumtemperatur gerührt. Das Reaktionsgemisch wurde dann mit Chloroform (20 ml) verdünnt und nacheinander mit 10%-iger wässriger Natriumthiosulfat-Lösung (10 ml) und konzentrierter wässriger Kochsalz-Lösung (10 ml) gewaschen. Die organische Phase wurde abgetrennt, über Natriumsulfat getrocknet, filtriert und am Rotationsverdampfer eingeengt. Man erhielt 570 mg (quant.) der Zielverbindung, welche ohne weitere Aufreinigung weiter umgesetzt wurde.
LC-MS (Methode 2): Rₜ = 2.79 min; MS (ESIpos): *m*/*z* (%) = 526.0 (100) [M+H]⁺.

### Ausführungsbeispiele:

### Beispiel 1

### 4-{(4R)-7-Methyl-2,5-dioxo-1-[3-(trifluormethyl)phenyl]-1,2,3,4,5,6,7,8-octahydropyrimido-[4,5-d]pyridazin-4-yl}benzonitril

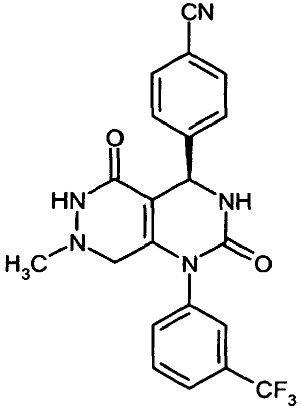

101 g (198.7 mmol) Ethyl (4*R*)-6-(brommethyl)-4-(4-cyanophenyl)-2-oxo-1-[3-(trifluormethyl)-phenyl]-1,2,3,4-tetrahydropyrimidin-5-carboxylat (Beispiel 6A) wurden in 2400 ml Dioxan vorgelegt. Zu der Lösung wurden 27.46 g (596.1 mmol) Methylhydrazin getropft und das Reaktionsgemisch anschließend 3 h bei Siedehitze gerührt. Der Ansatz wurde danach eingeengt und der Rückstand in Dichlormethan gelöst und mit Wasser gewaschen. Die organische Phase wurde über Natriumsulfat getrocknet und eingeengt. Der verbleibende Rückstand wurde über Kieselgel chromatographiert (Eluent: Dichlormethan/Methanol 95:5). Der nach dem Einengen der Produktfraktionen erhaltene Feststoff wurde mit Diethylether ausgerührt, abgesaugt und anschließend 4 Tage bei 50°C im Vakuum getrocknet. Man erhielt 56.8 g (66% d. Th.) der Titelverbindung.
LC-MS (Methode 2): Rₜ = 1.99 min; MS (ESIpos): *m*/*z* (%) = 428.1 (100) [M+H]⁺; MS (ESIneg): *m*/*z* (%) = 426.2 (100) [M-H]⁻.
¹H-NMR (400 MHz, DMSO-d₆): δ = 2.36 (s, 3H), 3.06-3.14 (d, 1H), 3.60-3.69 (d, 1H), 5.42 (d, 1H), 7.64-7.74 (m, 4H), 7.78-7.86 (d, 2H), 7.88-7.90 (d, 2H), 8.30 (d, 1H), 8.86 (s, 1H).

### Beispiel 2

### (rac)-4-{7-Methyl-2,5-dioxo-1-[3-(trifluormethyl)phenyl]-1,2,3,4,5,6,7,8-octahydropyrimido-[4,5-d]pyridazin-4-yl}-3-(methylsulfonyl)benzonitril

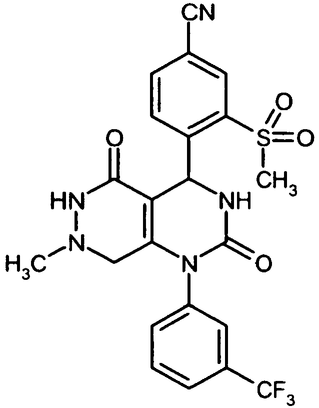

Ethyl 6-(brommethyl)-4-[4-cyano-2-(methylsulfonyl)phenyl]-2-oxo-1-[3-(trifluormethyl)phenyl]-1,2,3,4-tetrahydropyrimidin-5-carboxylat (150 mg, 0.256 mmol; Beispiel 19A) wurde in Dioxan (3.5 ml) vorgelegt. Zu dem Reaktionsgemisch wurde Methylhydrazin (35.4 mg, 0.767 mmol) getropft und der Ansatz anschließend 3 h bei Siedehitze gerührt. Die Reaktionsmischung wurde danach direkt mittels präparativer HPLC (Methode 12) aufgetrennt. Man erhielt 55 mg (39% d. Th.) der Zielverbindung.
HPLC (Methode 8): Rₜ = 3.95 min; MS (ESIpos): *m*/*z* (%) = 505.9 (100) [M+H]⁺
¹H-NMR (400 MHz, DMSO-d₆): δ = 2.52 (s, 3H), 3.23-3.29 (d, 1H), 3.55 (s, 3H), 3.55-3.62 (d, 1H), 6.53 (s, 1H), 7.70-7.95 (m, 5H), 8.19 (d, 1H), 8.26 (d, 1H), 8.31 (s, 1H), 8.93 (s, 1H).

### Beispiel 3

### 4-{(4S)-7-Methyl-2,5-dioxo-1-[3-(trifluormethyl)phenyl]-1,2,3,4,5,6,7,8-octahydropyrimido-[4,5-d]pyridazin-4-yl}-3-(methylsulfonyl)benzonitril

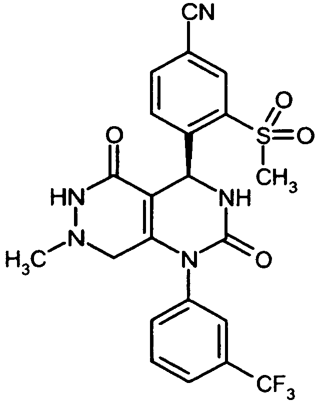

(*rac*)-4-{7-Methyl-2,5-dioxo-1-[3-(trifluormethyl)phenyl]-1,2,3,4,5,6,7,8-octahydropyrimido[4,5-d]pyridazin-4-yl}-3-(methylsulfonyl)benzonitril (Beispiel 2; 55 mg) wurde durch HPLC-Chromatographie an chiraler Phase in die Enantiomere aufgetrennt [Probenvorbereitung: Probe gelöst in THF/Ethylacetat 1:10 (22 ml); Injektionsvolumen: 11 ml; Säule: chirale Kieselgel-Phase basierend auf dem Selektor Poly(N-methacryloyl-L-leucin-d-menthylamid), 450 mm x 30 mm; Eluent: Ethylacetat; Fluss: 50 ml/min; Temperatur: 25°C; UV-Detektion: 260 nm]. Es wurden 16 mg des 4S Enantiomeren in Form eines farblosen, amorphen Feststoffs erhalten.

Rₜ = 7.84 min; ee = 99.0% [Säule: chirale Kieselgel-Phase basierend auf dem Selektor Poly(*N-*methacryloyl-L-leucin-D-menthylamid), 250 mm x 4.6 mm; Eluent: Ethylacetat; Fluss: 2 ml/min; Temperatur: 25°C; UV-Detektion: 265 nm]
HPLC (Methode 9): Rₜ = 3.86 min; MS (DCI/NH₃): *m*/*z* = 506.1 [M+H]⁺
¹H-NMR (400 MHz, DMSO-d₆): δ = 2.52 (s, 3H), 3.22-3.29 (d, 1H), 3.55 (s, 3H), 3.56-3.62 (d, 1H), 6.53 (s, 1H), 7.68-7.97 (m, 5H), 8.19 (d, 1H), 8.26 (d, 1H), 8.31 (s, 1H), 8.93 (s, 1H).

### Beispiel 4

### (rac)-4-{7-Methyl-2,5-dioxo-1-[3-(trifluormethyl)phenyl]-1,2,3,4,5,6,7,8-octahydropyrimido-[4,5-d]pyridazin-4-yl}-3-(trifluormethyl)benzonitril

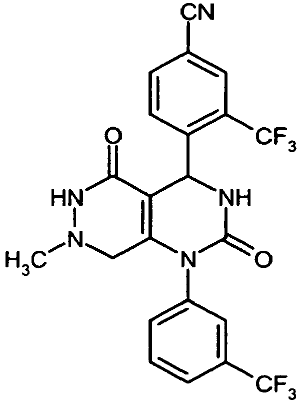

Ethyl 6-(brommethyl)-4-[4-cyano-2-(trifluormethyl)phenyl]-2-oxo-1-[3-(trifluormethyl)phenyl]-1,2,3,4-tetrahydropyrimidin-5-carboxylat (576 mg, 1 mmol; Beispiel 21A) wurde unter Argonschutzgasatmosphäre in Dioxan (20 ml) vorgelegt. Zu dem Reaktionsgemisch wurde Methylhydrazin (128 mg, 3 mmol) getropft und der Ansatz anschließend 8 h bei Siedehitze gerührt (Badtemperatur 120°C). Die Reaktionsmischung wurde danach eingeengt und der Rückstand mittels präparativer HPLC gereinigt (Säule: Gromsil C18 10 µm; Eluent: Acetonitril/Wasser + 0.1% TFA 10:90 → 90:10). Man erhielt die Zielverbindung als einen Feststoff, der noch einmal aus Acetonitril/Wasser umgefällt wurde (Ausbeute: 205 mg, 41% d. Th.).
LC-MS (Methode 4): Rₜ = 2.87 min; MS (ESIpos): m/z (%) = 496.2 (100) [M+H]⁺; MS (ESIneg): m/z (%) = 451.2 (100), 494 (20) [M-H]⁻.
¹H-NMR (400 MHz, DMSO-d₆): δ = 2.55 (s, 3H), 3.25 (d, 1H), 3.55 (d, 1H), 5.70 (s, 1H), 7.70-7.80 (m, 3H), 7.90 (s, 1H), 8.10-8.30 (m, 4H), 8.75 (s, 1H).

### Beispiel- 5

### 4-{(4R)-7-Methyl-2,5-dioxo-1-[3-(trifluormethyl)phenyl]-1,2,3,4,5,6,7,8-octahydropyrimido-[4,5-d]pyridazin-4-yl}-3-(trifluormethyl)benzonitril

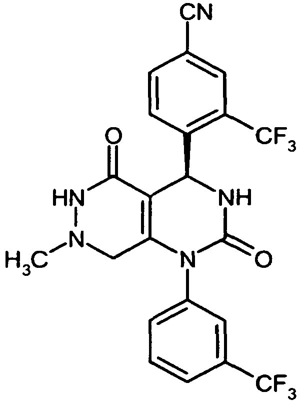

4-{(*rac*)-7-Methyl-2,5-dioxo-1-[3-(trifluormethyl)phenyl]-1,2,3,4,5,6,7,8-octahydropyrimido[4,5-d]pyridazin-4-yl}-3-(trifluormethyl)benzonitril (Beispiel 4; 180 mg) wurde durch HPLC-Chromatographie an chiraler Phase in die Enantiomere aufgetrennt [Säule: Daicel Chiralpak IA, 5 µm, 250 mm x 20 mm; Probenvorbereitung: Probe gelöst in Methanol/MTBE 1:1 (20 ml); Injektionsvolumen: 1 ml; Eluent: MTBE/Methanol 95:5; Fluss: 15 ml/min; Temperatur: 30°C; UV-Detektion: 220 nm]. Es wurden 75 mg (83% d. Th.) des 4R-Enantiomeren in Form eines Feststoffs erhalten. Der Enantiomerenüberschuss (ee-Wert) wurde chromatographisch bestimmt [Säule: Daicel Chiralpak IA, 5 µm, 250 mm x 4.6 mm; Eluent: Methanol/MTBE 1:9; Fluss: 1 ml/min; Temperatur: 25°C; UV-Detektion: 220 nm; Rₜ = 6.17 min; ee = 99.5%].
LC-MS (Methode 3): Rₜ = 3.06 min; MS (ESIpos): *m*/*z* (%) = 496.2 (100) [M+H]⁺; MS (ESIneg): *m*/*z* (%) = 451.1 (100), 494 (20) [M-H]⁻.

### Beispiel 6

### 4-{(4R)-2,5-Dioxo-7-phenyl-1-[3-(trifluormethyl)phenyl]-1,2,3,4,5,6,7,8-octahydropyrimido-[4,5-d]pyridazin-4-yl}benzonitril

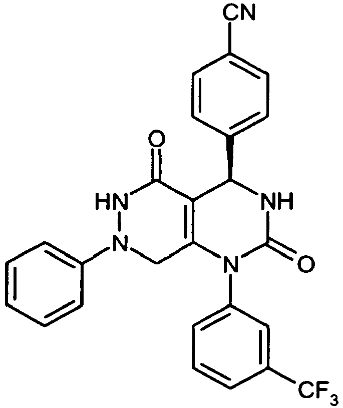

80 mg (0.16 mmol) Ethyl (4*R*)-6-(brommethyl)-4-(4-cyanophenyl)-2-oxo-1-[3-(trifluormethyl)-phenyl]-1,2,3,4-tetrahydropyrimidin-5-carboxylat (Beispiel 6A) wurden in 2 ml Dioxan gelöst, mit 51 mg (0.47 mmol) Phenylhydrazin versetzt und 3 Stunden bei 120°C gerührt. Das Reaktionsgemisch wurde danach eingeengt und der verbleibende Rückstand mittels präparativer HPLC (Methode 10) gereinigt. Die Produktfraktionen wurden vereinigt, eingeengt und der verbleibende Feststoff im Vakuum getrocknet (Ausbeute: 24.0 mg, 31% d. Th.).
HPLC (Methode 8): Rₜ = 4.31 min; MS (ESIpos): *m*/*z* (%) = 490 (100) [M+H]⁺
¹H-NMR (400 MHz, DMSO-d₄): δ = 3.92-4.02 (d, 1H), 4.22-4.33 (d, 1H), 5.40-5.45 (d, 1H), 6.61-6.70 (d, 2H), 6.70-6.91 (t, 1H), 7.12-7.19 (t, 2H), 7.21-7.27 (d, 2H), 7.43-7.57 (m, 1H), 7.57-7.64 (br. s, 1H), 7.64-7.70 (d, 2H), 7.72-7.80 (t, 1H), 7.85-7.90 (d, 1H), 8.27-8.34 (d, 1H), 9.79 (s, 1H).

### Beispiel 7

### 4-{2,5-Dioxo-7-phenyl-1-[3-(trifluormethyl)phenyl]-1,2,3,4,5,6,7,8-octahydropyrimido[4,5-d]-pyridazin-4-yl} -3-(methylsulfonyl)benzonitril

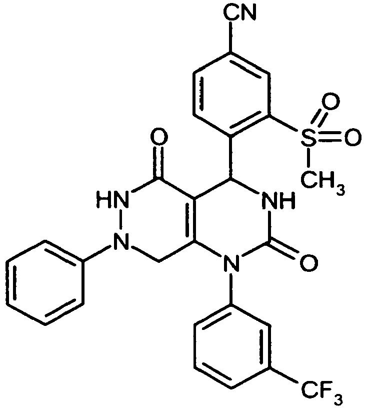

Ethyl 6-(brommethyl)-4-[4-cyano-2-(methylsulfonyl)phenyl]-2-oxo-1-[3-(trifluormethyl)phenyl]-1,2,3,4-tetrahydropyrimidin-5-carboxylat (150 mg, 0.256 mmol; Beispiel 19A) wurde in Dioxan (3.5 ml) vorgelegt. Zu dem Reaktionsgemisch wurde Phenylhydrazin (83.0 mg, 0.767 mmol) getropft und der Ansatz anschließend 3 h bei Siedehitze gerührt. Die Reaktionsmischung wurde danach direkt mittels präparativer HPLC (Methode 12) aufgetrennt. Man erhielt 9 mg (6% d. Th.) der Zielverbindung.
HPLC (Methode 8): Rₜ = 4.38 min; MS (ESIpos): *m*/*z* (%) = 568.0 (100) [M+H]⁺; MS (ESIneg): *m*/*z* (%) = 566.0 (100) [M-H]⁻
¹H-NMR (400 MHz, DMSO-d₆): δ = 3.54 (s, 3H), 4.04-4.11 (d, 1H), 4.23-4.30 (d, 1H), 6.45 (s, 1H), 6.75 (d, 2H), 7.05 (t, 1H), 7.21-7.32 (m, 3H), 7.64-7.91 (m, 6H), 8.27 (d, 1H), 9.88 (s, 1H).

### Beispiel 8

### 4-{(4R)-7-(2-Fluorbenzyl)-2,5-dioxo-1-[3-(trifluormethyl)phenyl]-1,2,3,4,5,6,7,8-octahydropyrimido[4,5-d]pyridazin-4-yl}benzonitril

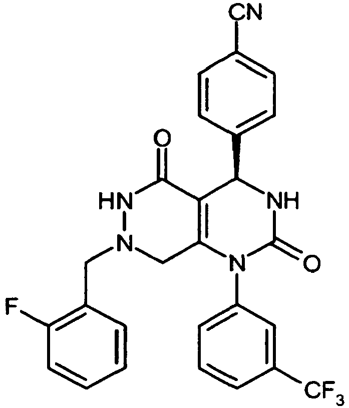

150 mg (0.30 mmol) Ethyl(4*R*)-6-(brommethyl)-4-(4-cyanophenyl)-2-oxo-1-[3-(trifluormethyl)-phenyl]-1,2,3,4-tetrahydropyrimidin-5-carboxylat (Beispiel 6A) wurden in 3.5 ml Dioxan gelöst. Es wurden 124 mg (0.89 mmol) *o*-Fluorbenzylhydrazin zugegeben und der Ansatz über Nacht bei 120°C gerührt. Das Reaktionsgemisch wurde danach eingeengt und der verbleibende Rückstand mittels präparativer HPLC (Methode 10) gereinigt. Die Produktfraktionen wurden vereinigt, eingeengt und der verbleibende Feststoff im Vakuum getrocknet (Ausbeute: 35.0 mg, 22.7% d. Th.).
LC-MS (Methode 4): Rₜ = 3.23 min; MS (ESIpos): *mlz* (%) = 522.2 (100) [M+H]⁺; MS (ESIneg): *m*/*z* (%) = 520.2 (100) [M-H]⁻.
¹H-NMR (400 MHz, DMSO-d₆): δ = 2.93-3.07 (d, 1H), 3.49-3.62 (d, 1H), 3.71-3.85 (m, 2H), 5.44 (s, 1H), 7.00-7.10 (t, 2H), 7.13-7.22 (m, 1H), 7.23-7.33 (m, 1H), 7.59-7.67 (m, 2H), 7.68-7.76 (m, 3H), 7.80 (br. s, 1H), 7.86-7.95 (d, 2H), 8.28-8.35 (d, 1H), 9.09 (s, 1H).

Analog zur Vorschrift für das Beispiel 8 wurden aus dem unter Beispiel 6A hergestellten Edukt und dem entsprechenden, kommerziell erhältlichen Hydrazin-Derivat die Verbindungen in der folgenden Tabelle hergestellt:

| **Beispiel** | **Struktur** | **Ausbeute** | **Analytische Daten** |
|---|---|---|---|
| **9** | 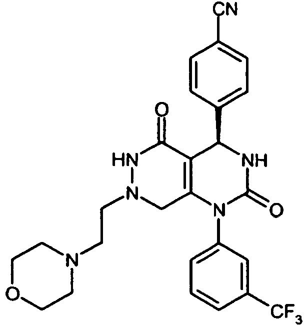 | 51% d. Th. | HPLC (Methode 8): R, = 3.66 min; MS (ESIpos): *m*/*z* = 527 [M+H]⁺. |
| **10** | 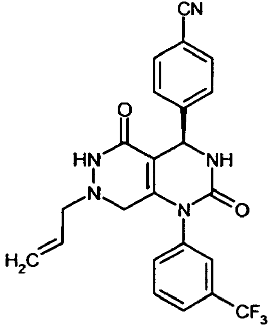 | 8% d. Th. | LC-MS (Methode 4): Rₜ = 2.94 min; MS (ESIpos): *m*/*z* = 454 [M+H]⁺. |
| **11** | 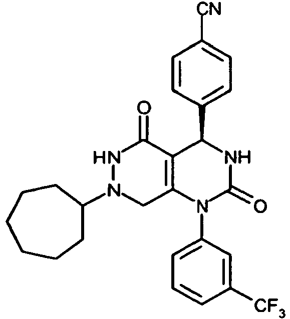 | 11% d. Th. | LC-MS (Methode 4): Rₜ = 3.39 min; MS (ESIpos): *m*/*z* = 510 [M+H]⁺. |
| **12** | 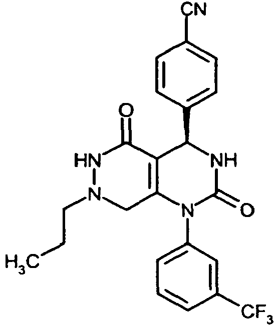 | 22% d. Th. | LC-MS (Methode 2): Rₜ = 2.18 min; MS (ESIpos): *m*/*z* = 456 [M+H]⁺. |
| **13** | 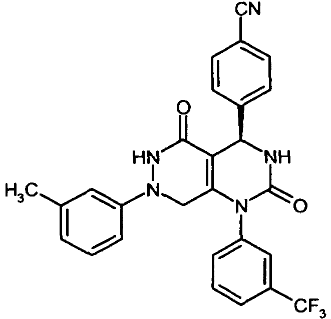 | 5% d. Th. | LC-MS (Methode 3): Rₜ =3.45 min; MS (ESIpos): *mlz* = 504 [M+H]⁺. |
| **14** | 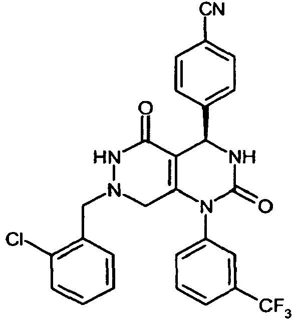 | 28% d. Th. | LC-MS (Methode 3): Rₜ = 3.50 min; MS (ESIpos): *m*/*z* = 538 [M+H]⁺. |
| **15** | 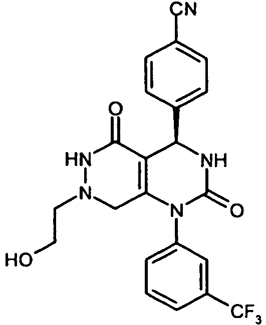 | 32% d. Th. | HPLC (Methode 9): Rₜ =3.63 min; MS (ESIpos): *m*/*z* = 458 [M+H]⁺. |
| **16** | 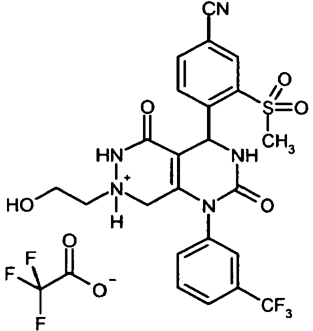 | 14% d. Th. | HPLC (Methode 9): Rₜ = 3.72 min; MS (DCI/NH₃): *m*/*z* = 536.1 [M+H]⁺. ¹H-NMR (400 MHz, DMSO-d₆): δ = 2.75-2.92 (m, 2H), 3.67 (t, 2H), 3.41-3.49 (d, 1H), 3.55 (s, 3H), 3.63-3.70 (d, 1H), 3.97 (t, 1H), 6.52 (s, 1H), 7.67-7.93 (m, 5H), 8.14-8.33 (m, 3H), 8.99 (s, 1H). |

### Beispiel 17

### 4-{(4R)-7-Ethyl-2,5-dioxo-1-[3-(trifluormethyl)phenyl]-1,2,3,4,5,6,7,8-octahydropyrimido[4,5-d]-pyridazin-4-yl}benzonitril

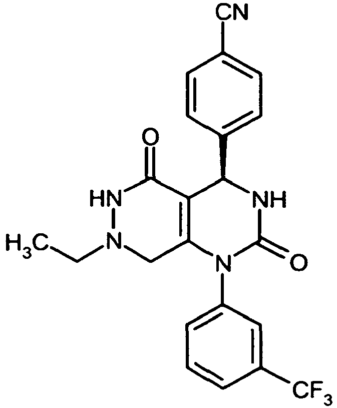

200 mg (0.393 mmol) Ethyl (4*R*)-6-(brommethyl)-4-(4-cyanophenyl)-2-oxo-1-[3-(trifluormethyl)-phenyl]-1,2,3,4-tetrahydropyrimidin-5-carboxylat (Beispiel 6A) wurden in 1 ml Dioxan gelöst, mit 71 mg (0.472 mmol) Ethylhydrazin-Oxalat versetzt und 3 Stunden bei 120°C gerührt. Das Reaktionsgemisch wurde danach eingeengt und der verbleibende Rückstand mittels präparativer HPLC (Methode 10) gereinigt. Die Produktfraktionen wurden vereinigt, eingeengt und der verbleibende Feststoff im Vakuum getrocknet (Ausbeute: 26.0 mg, 15% d. Th.).
LC-MS (Methode 3): Rₜ = 2.97 min; MS (ESIpos): *m*/*z* (%) = 442.3 (100) [M+H]⁺.
¹H-NMR (400 MHz, DMSO-d₆): δ = 9.03 (s, 1H), 8.28 (d, 1H), 7.87 (d, 2H), 7.82 (d, 2H), 7.72 (t, 1H), 7.69 (s, 1H), 7.65 (d, 2H), 5.42 (br. s, 1H), 3.67 (d, 1H), 3.14 (d, 1H), 2.61 (q, 2H), 0.77 (t, 3H).

### Beispiel 18

### 4-[4-Cyano-2-(methylsulfonyl)phenyl]-7-ethyl-2,5-dioxo-1-[3-(trifluormethyl)phenyl]-1,2,3,4,5,6,7,8-octahydropyrimido[4,5-d]pyridazin-7-ium-Trifluoracetat

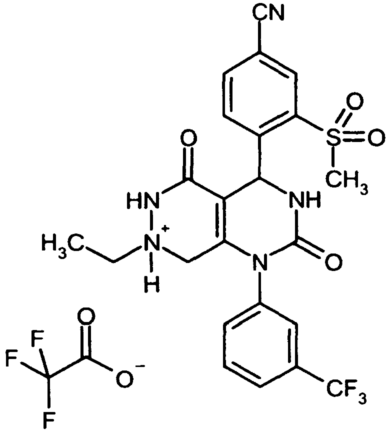

Ethyl 6-(brommethyl)-4-[4-cyano-2-(methylsulfonyl)phenyl]-2-oxo-1-[3-(trifluormethyl)phenyl]-1,2,3,4-tetrahydropyrimidin-5-carboxylat (150 mg, 0.256 mmol; Beispiel 19A) wurde in Dioxan (3.5 ml) vorgelegt. Zu dem Reaktionsgemisch wurde Ethylhydrazin-Oxalat (115 mg, 0.767 mmol) gegeben und der Ansatz anschließend 3 h bei Siedehitze gerührt. Die Reaktionsmischung wurde danach direkt mittels zweimaliger präparativer HPLC aufgetrennt (zunächst nach Methode 12, dann nach Methode 15). Man erhielt 18 mg (14% d. Th.) der Zielverbindung.
HPLC (Methode 9): Rₜ = 4.04 min; MS (DCI/NH₃): *m*/*z* (%) = 520.1 (100) [M+H]⁺.
¹H-NMR (400 MHz, DMSO-d₆): δ = 0.85 (t, 3H), 2.77 (q, 2H), 3.26-3.34 (d, 1H), 3.55 (s, 3H), 3.57-3.64 (d, 1H), 6.52 (s, 1H), 6.70-7.99 (m, 5H), 8.17 (d, 1H), 8.26 (d, 1H), 8.31 (s, 1H), 9.08 (s, 1H).

### Beispiel 19

### 4-(4R)-2,5-Dioxo-7-[4-(trifluormethyl)benzyl]-1-[3-(trifluormethyl)phenyl]-1,2,3,4,5,6,7,8-octa-hydropyrimido[4,5-d]pyridazin-4-ylbenzonitril

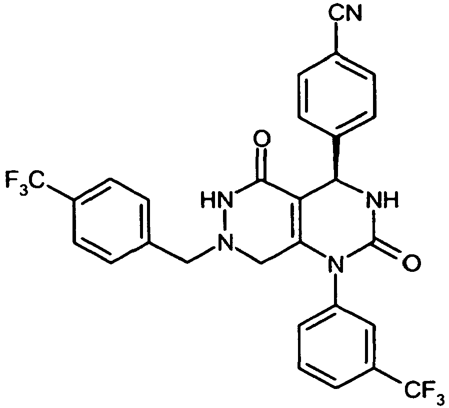

150 mg (0.295 mmol) Ethyl (4*R*)-6-(brommethyl)-4-(4-cyanophenyl)-2-oxo-1-[3-(trifluormethyl)-phenyl]-1,2,3,4-tetrahydropyrimidin-5-carboxylat (Beispiel 6A) wurden in 3 ml Dioxan gelöst, mit 168 mg (0.885 mmol) [4-(Trifluormethyl)benzyl]hydrazin versetzt und über Nacht bei 120°C gerührt. Das Reaktionsgemisch wurde danach eingeengt und der verbleibende Rückstand mittels präparativer HPLC (Methode 10) aufgereinigt. Die produkthaltigen Fraktionen wurden vereinigt und eingeengt. Der Rückstand wurde durch erneute präparative HPLC (Methode 13) nachgereinigt. Nach dem Einengen der Produktfraktionen erhielt man 38 mg (23% d. Th.) der Zielverbindung.
LC-MS (Methode 2): Rₜ = 2.59 min; MS (ESIpos): *m*/*z* (%) = 572.1 (100) [M+H]⁺.
¹H-NMR (400 MHz, DMSO-d₆): δ = 9.08 (s, 1H), 8.32 (d, 1H), 7.92 (d, 2H), 7.84 (s, 1H), 7.75 (d, 2H), 7.71 (d, 1H), 7.63-7.57 (m, 4H), 7.37 (d, 2H), 5.45 (d, 1H), 3.83 (dd, 2H), 3.53 (d, 1H), 2.97 (d, 1H).

### Beispiel 20

### 4-(4R)-7-(Cyclopropylmethyl)-2,5-dioxo-1-[3-(trifluormethyl)phenyl]-1,2,3,4,5,6,7,8-octahydropyrimido[4,5-d]pyridazin-4-ylbenzonitril

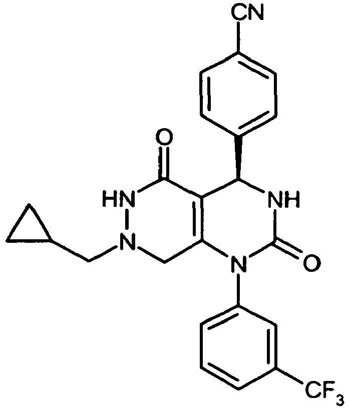

150 mg (0.295 mmol) Ethyl (4*R*)-6-(brommethyl)-4-(4-cyanophenyl)-2-oxo-1-[3-(trifluormethyl)-phenyl]-1,2,3,4-tetrahydropyrimidin-5-carboxylat (Beispiel 6A) wurden in 2 ml Dioxan gelöst, mit 254 mg (2.951 mmol) (Cyclopropylmethyl)hydrazin versetzt und über Nacht bei 120°C gerührt. Das Reaktionsgemisch wurde danach eingeengt und der verbleibende Rückstand mittels präparativer HPLC (Methode 10) aufgereinigt. Die produkthaltigen Fraktionen wurden vereinigt und eingeengt. Der Rückstand wurde durch erneute präparative HPLC (Methode 14) nachgereinigt. Nach dem Einengen der Produktfraktionen erhielt man 5 mg (4% d. Th.) der Zielverbindung.
¹H-NMR (400 MHz, DMSO-d₆): δ = 8.94 (s, 1H), 8.27 (d, 1H), 7.86 (br. d, 3H), 7.81-7.80 (m, 1H), 7.73-7.70 (m, 2H), 7.64 (d, 2H), 5.42 (s, 1H), 3.74 (d, 1H), 3.29 (d, 1H), 2.46 (dd, 1H), 2.34 (dd, 1H), 0.51 (m, 1H), 0.25 (m, 1H), 0.16 (m, 1H), -0.12 (m, 1H), -0.19 (m, 1H).

### Beispiel 21

### Ethyl [(4R)-4-(4-cyanophenyl)-2,5-dioxo-1-[3-(trifluormethyl)phenyl]-2,3,4,5,6,8-hexahydropyrimido[4,5-d]pyridazin-7(1H)-yl]acetat

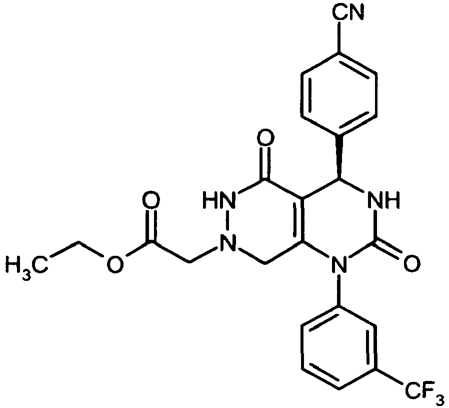

300 mg (0.59 mmol) Ethyl (4*R*)-6-(brommethyl)-4-(4-cyanophenyl)-2-oxo-1-[3-(trifluormethyl)-phenyl]-1,2,3,4-tetrahydropyrimidin-5-carboxylat (Beispiel 6A) wurden in 5 ml Dioxan gelöst. Es wurden 456 mg (2.95 mmol) Hydrazinoessigsäureethylester-Hydrochlorid, welches zuvor in Methanol gelöst und über eine StratoSphere-Kartusche (PL-HCO₃ MP SPE, Fa. Polymere Laboratories) gegeben worden war, hinzugefügt. Der Ansatz wurde anschließend über Nacht unter Rückfluss gerührt. Das Reaktionsgemisch wurde danach eingeengt und der verbleibende Rückstand mittels präparativer HPLC (Methode 10) gereinigt. Die Produktfraktionen wurden vereinigt, eingeengt und der verbleibende Feststoff im Vakuum getrocknet (Ausbeute: 45.0 mg, 15% d. Th.).
LC-MS (Methode 5): Rₜ = 1.05 min; MS (ESIpos): *m*/*z* (%) = 500.2 (100) [M+H]⁺; MS (ESIneg): *m*/*z* (%) = 498.3 (100) [M-H]⁻.
¹H-NMR (400 MHz, DMSO-d₆): δ = 1.05-1.13 (t, 3H), 3.36-3.41 (d, 1H), 3.42-3.47 (s, 1H), 3.55-3.63 (d, 1H), 3.67-3.77 (d, 1H), 3.91-4.05 (m, 2H), 5.42 (s, 1H), 7.62-7.74 (m, 4H), 7.78-7.85 (m, 2H), 7.86-7.92 (d, 2H), 8.27-8.33 (d, 1H), 8.97 (s, 1H).

Analog der Vorschrift für das Beispiel 21 wurde aus dem unter Beispiel 6A hergestellten Edukt und dem entsprechenden Hydrazin-Hydrochlorid die Verbindung in der folgenden Tabelle hergestellt:

| **Beispiel** | **Struktur** | **Ausbeute** | **Analytische Daten** |
|---|---|---|---|
| 22 | 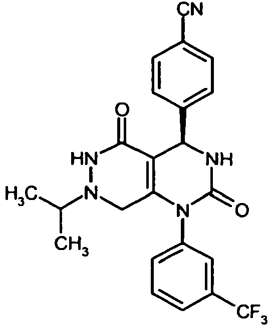 | 15% d. Th. | LC-MS (Methode 7): R, = 1.67 min; MS (ESIpos): *m*/*z* = 456 [M+H]⁺. |

### Beispiel 23

### 2-[(4R)-4-(4-Cyanophenyl)-7-methyl-2,5-dioxo-1-[3-(trifluormethyl)phenyl]-1,4,5,6,7,8-hexahydropyrimido[4,5-d]pyridazin-3(2H)-yl]-N,N-dimethylacetamid

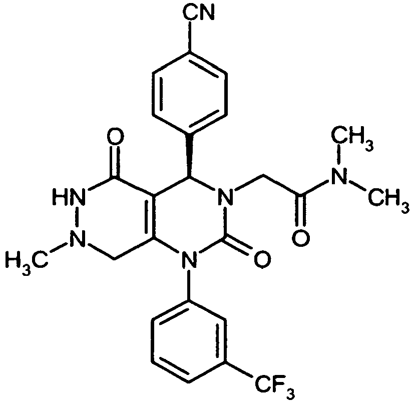

100 mg (0.169 mmol) der Verbindung aus Beispiel 13A wurden in 2 ml Dioxan gelöst und mit 23 mg (0.506 mmol) Methylhydrazin versetzt. Das Gemisch wurde zwei Stunden bei 120°C gerührt und dann eingeengt. Der Rückstand wurde in DMSO aufgenommen und mittels präparativer HPLC (Methode 10) gereinigt. Man erhielt 53 mg (61 % d. Th.) der Zielverbindung.
HPLC (Methode 8): Rₜ = 3.98 min; MS (ESIpos): *m*/*z* (%) = 513.2 (100) [M+H]⁺
¹H-NMR (400 MHz, DMSO-d₆): δ = 2.34 (s, 3H), 2.81 (s, 3H), 2.85 (s, 3H), 3.06 (d, 1H), 3.52 (d, 1H), 3.77 (d, 1H), 4.50 (d, 1H), 5.53 (s, 1H), 7.87 (d, 1H), 7.71 (d, 1H), 7.73 (d, 2H), 7.82 (d, 2H), 7.88 (d, 2H), 8.87 (s, 1H).

Analog der Vorschrift für das Beispiel 23 wurden aus den angegebenen Edukten und dem entsprechenden Hydrazin-Derivat die Verbindungen in der folgenden Tabelle hergestellt:

| **Beispiel** | **Struktur** | **Edukt** | **Ausbeute** | **Analytische Daten** |
|---|---|---|---|---|
| **24** | 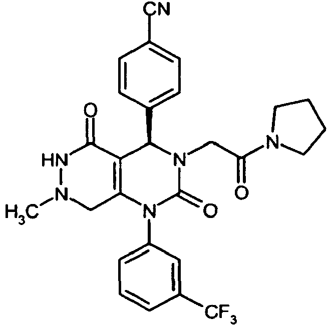 | 14A | 53% d. Th. | LC-MS (Methode 3): 3.10 min; MS (ESIpos): *m*/*z* = 539 [M+H]⁺. |
| **25** | 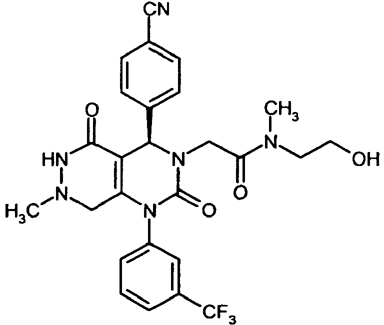 | 15A | 53% d. Th. | LC-MS (Methode 4): Rₜ = 2.50 min; MS (ESIpos): *m*/*z* = 543 [M+H]⁺. |
| **26** | 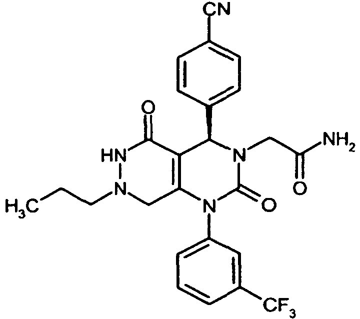 | 12A | 11% d. Th. | LC-MS (Methode 2): Rₜ=2.11 min; MS (ESIpos): *m*/*z* = 513 [M+H]⁺. |

### Beispiel 27

### 2-[(4R)-4-(4-Cyanophenyl)-7-(2-hydroxyethyl)-2,5-dioxo-1-[3-(trifluormethyl)phenyl]-1,4,5,6,7,8-hexahydropyrimido[4,5-d]pyridazin-3(2H)-yl]acetamid

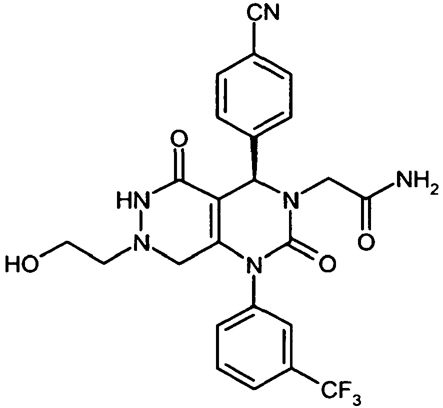

200 mg (0.393 mmol) der Verbindung aus Beispiel 12A wurden in 2 ml Dioxan gelöst, mit 32 mg (0.425 mmol) 2-Hydrazinoethanol versetzt und 4 Stunden bei 120°C gerührt. Das Reaktionsgemisch wurde danach eingeengt und der Rückstand in einem Gemisch aus Wasser und Methylenchlorid aufgenommen. Die organische Phase wurde abgetrennt, über Natriumsulfat getrocknet, filtriert und bis fast zur Trockene eingeengt. Zu diesem Rückstand wurde eine kleine Menge Kieselgel gegeben und der Rest des Lösungsmittels dann im Vakuum abgezogen. Die Reinigung dieses Rohprodukts erfolgte anschließend durch zweimalige Chromatographie an Kieselgel (Laufmittel jeweils Dichlormethan/Methanol 10:1). Es wurden so 13 mg (7% d. Th.) der Zielverbindung erhalten.
LC-MS (Methode 2): Rₜ = 1.71 min; MS (ESIpos): *m*/*z* (%) =515.0 (100) [M+H]⁺; MS (ESIneg): *m*/*z* (%) =513.2 (100) [M-H]⁻.
¹H-NMR (400 MHz, DMSO-d₄): δ = 8.93 (s, 1H), 7.89 (d, 2H), 7.82 (d, 2H), 7.74-7.69 (m, 4H), 7.40 (s, 1H), 7.15 (s, 1H), 5.54 (s, 1H), 4.43 (t, 1H), 4.17 (d, 1H), 3.76 (d, 1H), 3.30-3.21 (m, 4H), 2.71-2.57 (m, 2H).

### Beispiel 28

### Methyl [(4R)-4-(4-cyanophenyl)-7-methyl-2,5-dioxo-1-[3-(trifluormethyl)phenyl]-1,4,5,6,7,8-hexa-hydropyrimido[4,5-d]pyridazin-3(2H)-yl]acetat

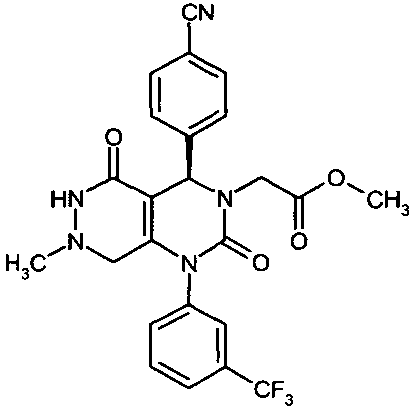

65 mg (0.112 mmol) der Verbindung aus Beispiel 17A wurden in 2 ml Dioxan gelöst und mit 16 mg (0.336 mmol) Methylhydrazin versetzt. Das Gemisch wurde über Nacht bei 120°C gerührt und dann eingeengt. Der Rückstand wurde in DMSO aufgenommen und mittels präparativer HPLC (Methode 10) gereinigt. Man erhielt 40 mg (72% d. Th.) der Zielverbindung.
LC-MS (Methode 4): Rₜ = 2.96 min; MS (ESIpos): *m*/*z* (%) = 500.3 (100) [M+H]⁺; MS (ESIneg): *m*/*z* (%) = 498.3 (100) [M-H]⁻.
¹H-NMR (400 MHz, DMSO-d₆): δ = 8.90 (s, 1H), 7.88 (br. d, 3H), 7.85-7.82 (m, 1H), 7.76-7.72 (m, 4H), 5.66 (s, 1H), 4.19 (d, 1H), 3.82 (d, 1H), 3.78 (d, 1H), 3.55 (s, 3H), 3.08 (d, 1H), 2.36 (s, 3H).

### Beispiel 29

### 4-{(4R)-7-(3-Methylbutyl)-2,5-dioxo-1-[3-(trifluormethyl)phenyl]-1,2,3,4,5,6,7,8-octahydropyrimido[4,5-d]pyridazin-4-yl}benzonitril

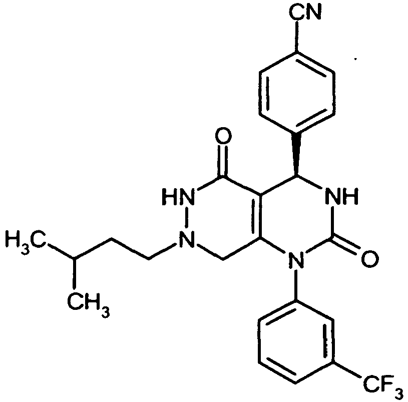

150 mg (0.295 mmol) der Verbindung aus Beispiel 6A wurden in 3 ml Dioxan gelöst und mit 91 mg (0.885 mmol) (3-Methylbutyl)hydrazin versetzt. Das Gemisch wurde 3 h bei 120°C gerührt und dann eingeengt. Der Rückstand wurde in DMSO aufgenommen und mittels präparativer HPLC (Methode 10) gereinigt. Man erhielt 43 mg (29% d. Th.) der Zielverbindung.
LC-MS (Methode 4): Rₜ = 3.31 min; MS (ESIpos): *m*/*z* (%) = 484 (100) [M+H]⁺.
¹H-NMR (400 MHz, DMSO-d₆): δ = 9.00 (s, 1H), 8.29 (d, 1H), 7.88-7.64 (m, 8H), 5.45 (d, 1H), 3.70 (d, 1H), 3.07 (d, 1H), 2.57-2.45 (m, 2H), 1.38 (m, 1H), 1.03 (m, 2H), 0.71 (d, 3H), 0.66 (d, 3H).

### Beispiel 30

### (rac)-4- {1-[4-Fluor-3-(trifluormethyl)phenyl]-7-methyl-2,5-dioxo-1,2,3,4,5,6,7,8-octahydropyrimido[4,5-d]pyridazin-4-yl}benzonitril

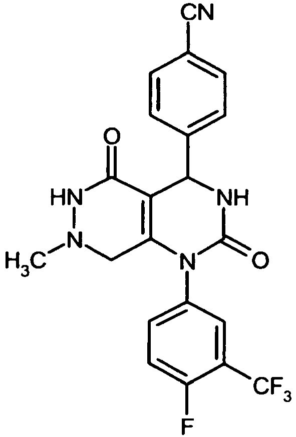

527 mg (1.0 mmol) 4-(4-Cyanophenyl)-1-[4-fluor-3-(trifluormethyl)phenyl]-6-(brommethyl)-2-oxo-1,2,3,4-tetrahydropyrimidin-5-carbonsäureethylester wurden in 25 ml Dioxan gelöst und mit 138 mg (3.0 mmol; 3 eq.) Methylhydrazin versetzt. Das Gemisch wurde 1 h bei 120°C gerührt und dann eingeengt. Der Rückstand wurde direkt mittels präparativer HPLC gereinigt (Säule: Gromsil C-18 10 µm; Eluent: Acetonitril/Wasser + 0.1% TFA 10:90 → 90:10). Man erhielt 270 mg (55% d. Th.) der Zielverbindung.
LC-MS (Methode 3): Rₜ = 2.91 min; MS (ESIpos): *m*/*z* (%) = 446.1 (100) [M+H]⁺; MS (ESIneg): m/z (%) = 401.0 (100), 444.2 (50) [M-H]⁻.
¹H-NMR (400 MHz, DMSO-d₆): δ = 2.37 (s, 3H), 3.14-3.18 (d, 1H), 3.65-3.70 (d, 1H), 5.43 (s, 1H), 7.60-7.90 (m, 7H), 8.30 (d, 1H), 8.84 (s, 1H).

### Beispiel 31

### (4R)-4-{1-[4-Fluor-3-(trifluormethyl)phenyl]-7-methyl-2,5-dioxo-1,2,3,4,5,6,7,8-octahydropyrimido[4,5-d]pyridazin-4-yl}benzonitril

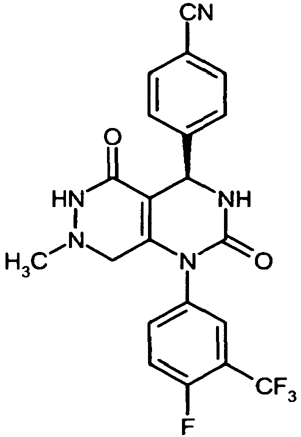

*(rac)-4-* {1-[4-Fluor-3-(trifluormethyl)phenyl]-7-methyl-2,5-dioxo-1,2,3,4,5,6,7,8-oetahydropyrimido[4,5-d]pyridazin-4-yl}benzonitril (250 mg) wurde durch HPLC-Chromatographie an chiraler Phase in die Enantiomere aufgetrennt [Probenvorbereitung: Probe gelöst in THF/Ethylacetat 2:5 (14 ml); Injektionsvolumen: 14 ml; Säule: chirale Kieselgel-Phase basierend auf dem Selektor Poly(*N*-methacryloyl-L-leucin-dicyclopropylmethylamid), 250 mm x 20 mm; Eluent: Ethylacetat; Fluss: 50 ml/min; Temperatur: 24°C; UV-Detektion: 260 nm]. Als Fraktion 2 wurden 118 mg des 4*R*-Enantiomeren in Form eines farblosen, amorphen Feststoffs erhalten (das 4*S*-Enantiomer wurde als früher eluierende Fraktion 1 erhalten). Das 4*R*-Enantiomer wurde dann mittels präparativer HPLC an achiraler Phase nochmals nachgereinigt (Säule: Gromsil C-18 10 µm; Eluent: Acetonitril/Wasser + 0.1% TFA 10:90 → 90:10). Man erhielt so 117 mg der Titelverbindung.

Rₜ = 4.91 min; ee >99.0% [Säule: chirale Kieselgel-Phase basierend auf dem Selektor Poly-(*N*-methacryloyl-L-leucin-dicyclopropylmethylamid), 250 mm x 4.6 mm; Eluent: Ethylacetat/Methanol 10:1; Fluss: 2 ml/min; Temperatur: 25°C; UV-Detektion: 260 nm].
LC-MS (Methode 1): R₁ = 1.78 min; MS (ESIpos): *m*/*z* (%) = 446.2 (100) [M+H]⁺; MS (ESIneg): *m*/*z* (%) = 401.2 (100), 444.2 (50) [M-H]⁻.
¹H-NMR (400 MHz, DMSO-d₆): δ = 2.37 (s, 3H), 3.14-3.18 (d, 1H), 3.65-3.70 (d, 1H), 5.43 (s, 1H), 7.59-7.89 (m, 7H), 8.29 (d, 1H), 8.83 (s, 1H).

### B. Bewertung der pharmakoloeischen Wirksamkeit

Die pharmakologische Wirkung der erfindungsgemäßen Verbindungen kann in den nachstehend beschriebenen Assays gezeigt werden:

### Abkürzungen:

- AMC: 7-Amido-4-methylcumarin
- BNP: brain natriuretic peptide
- BSA: bovine serum albumin
- HEPES: *N*-(2-Hydroxyethyl)piperazin-*N*'-2-ethansulfonsäure
- HNE: humane neutrophile Elastase
- IC: Inhibitionskonzentration
- MeOSuc: Methoxysuccinyl
- NADP: Nikotinamid-Adenin-Dinukleotid-Phosphat
- v/v: Volumen zu Volumen-Verhältnis (einer Lösung)
- w/v: Gewicht zu Volumen-Verhältnis (einer Lösung)

### B-1. In vitro HNE-Inhibitionstest

Die Wirkstärke der erfindungsgemäßen Verbindungen wird in einem *in vitro*-Hemmtest ermittelt. Die HNE-vermittelte amidolytische Spaltung eines geeigneten Peptidsubstrates führt hierbei zu einer Fluoreszenzlichtzunahme. Die Signalintensität des Fluoreszenzlichtes ist direkt proportional zur Enzymaktivität. Die Wirkkonzentration einer Testverbindung, bei der die Hälfte des Enzyms inhibiert ist (50% Signalintensität des Fluoreszenzlichtes), wird als IC₅₀-Wert angegeben.

### Ausführung:

In einer 384 Loch-Mikrotiterplatte werden in einem Testvolumen von insgesamt 50 µl Testpuffer (0.1 M HEPES pH 7.4, 0.5 M NaCl, 0.1% w/v BSA, 1% v/v DMSO), Enzym (80 pM HNE; Fa. Serva, Heidelberg) und Substrat (20 µM MeOSuc-Ala-Ala-Pro-Val-AMC; Fa. Bechern, Weil am Rhein) bei An- und Abwesenheit der Testsubstanz zwei Stunden bei 37°C inkubiert. Die Fluoreszenzlichtintensität der Testansätze wird gemessen (Ex. 380 nm, Em. 460 nm). Die IC₅₀-Werte werden durch eine Auftragung der Fluoreszenzlichtintensität gegenüber der Wirkstoffkonzentration ermittelt.

Für die erfindungsgemäßen Verbindungen repräsentative IC₅₀-Werte sind in der folgenden Tabelle A wiedergegeben:

**Tabelle A: Hemmung der humanen neutrophilen Elastase (HNE)**

| **Ausführungsbeispiel Nr.** | **IC₅₀ [nM]** |
|---|---|
| 1 | 28 |
| 2 | 3 |
| 4 | 10 |
| 8 | 32 |
| 9 | 23 |
| 17 | 16 |
| 23 | 4 |
| 27 | 11 |
| 31 | 28 |

### B-2. Tiermodell der pulmonalen arteriellen Hypertonie

Die Monocrotalin-induzierte pulmonale Hypertonie der Ratte ist ein weit verbreitetes Tiermodell für die pulmonale arterielle Hypertonie. Das Pyrrolizidin-Alkaloid Monocrotalin wird nach subkutaner Injektion in der Leber zum toxischen Monocrotalinpyrrol metabolisiert und führt innerhalb weniger Tage zu einer Endothelschädigung im Lungenkreislauf, gefolgt von einem Remodeling der kleinen pulmonalen Arterien (Mediahypertrophie, *de novo*-Muskularisierung). Eine einmalige subkutane Injektion ist ausreichend, um bei Ratten innerhalb von 4 Wochen eine ausgeprägte pulmonale Hypertonie zu induzieren [Cowan et al., Nature Med. 6, 698-702 (2000)].

Für das Modell werden männliche Sprague-Dawley-Ratten verwendet. An Tag 0 erhalten die Tiere eine subkutane Injektion von 60 mg/kg Monocrotalin. Die Behandlung der Tiere beginnt erst frühestens 14 Tage nach der Monocrotalin-Injektion und erstreckt sich über einen Zeitraum von mindestens 14 Tagen. Am Studienende erfolgen hämodynamische Untersuchungen der Tiere sowie eine Ermittlung der arteriellen und zentralvenösen Sauerstoffsättigung. Für die hämodynamische Messung werden die Ratten initial mit Pentobarbital (60 mg/kg) anästhesiert. Anschließend werden die Tiere tracheotomiert und künstlich beatmet (Frequenz: 60 Atemzüge/min; Verhältnis Inspiration zu Exspiration: 50:50; positiver endexspiratorischer Druck: 1 cm H₂O; Atemzugvolumen: 10 ml/kg Körpergewicht; FIO₂: 0.5). Die Narkose wird durch Isofluran-Inhalationsnarkose aufrechterhalten. Der systemische Blutdruck wird in der linken *A. carotis* mittels eines Millar-Microtip-Katheters ermittelt. Ein Polyethylenkatheter wird über die rechte *V. jugularis* in den rechten Ventrikel vorgeschoben zur Bestimmung des rechten Ventrikeldruckes. Das Herzminutenvolumen wird mittels Thermodilution ermittelt. Im Anschluß an die Hämodynamik wird das Herz entnommen und das Verhältnis rechter zu linker Ventrikel inklusive Septum bestimmt. Weiterhin werden Plasmaproben zur Bestimmung von Biomarkern (zum Beispiel proBNP) und Plasma-Substanzspiegeln gewonnen.

### B-3. Tiermodell des akuten Lungenversagens

Elastase-induziertes Lungenversagen an Maus, Ratte oder Hamster ist ein weit verbreitetes Tiermodell für akutes Lungenversagen (auch: "acute lung injury", "acute respiratory distress syndrome") [Tremblay et al., Chest 121, 582-588 (2002); Kuraki et al., Am. J. Resp. Crit. Care Med. 166, 596-500 (2002)]. Die Behandlung der Tiere erfolgt 1 Stunde vor orotrachealer Instillation der humanen neutrophilen Elastase (HNE). 2 Stunden nach der orotrachealen HNE-Instillation wird eine bronchoalveolare Lavage durchgeführt und der Hämoglobingehalt sowie das Differentialzellbild in der Lavage bestimmt.

### B-4. Tiermodell des Lungenemphysems

Elastase-induziertes Lungenemphysem an Maus, Ratte oder Hamster ist ein weit verbreitetes Tiermodell für Lungenemphysem [Sawada et al., Exp. Lung Res. 33, 277-288 (2007)]. Die Tiere erhalten eine orotracheale Instillation porciner Pankreas-Elastase. Die Behandlung der Tiere beginnt am Tag der Instillation der porcinen Pankreas-Elastase und erstreckt sich über einen Zeitraum von 3 Wochen. Am Studienende wird die Lungen-Compliance bestimmt und eine Alveolarmorphometrie durchgeführt.

### B-5. Bestimmung pharmakokinetischer Kenngrößen nach intravenöser und oraler Gabe

Die zu untersuchende Substanz wird Tieren (z.B. Maus, Ratte, Hund) intravenös als Lösung appliziert, die orale Applikation erfolgt als Lösung oder Suspension über eine Schlundsonde. Nach Substanzgabe wird den Tieren zu festgelegten Zeitpunkten Blut entnommen. Dieses wird heparinisiert, anschließend wird daraus durch Zentrifugation Plasma gewonnen. Die Substanz wird im Plasma über LC/MS-MS analytisch quantifiziert. Aus den so ermittelten Plasmakonzentration-Zeit-Verläufen werden die pharmakokinetischen Kenngrößen wie AUC (Fläche unter der Konzentration-Zeit-Kurve), Cₘₐₓ (maximale Plasmakonzentration), T_{1/2} (Halbwertszeit) und CL (Clearance) mittels eines validierten pharmakokinetischen Rechenprogramms berechnet.

### B-6. Bestimmung der Plasmaprotein-Bindung

Die Proteinbindung von Testsubstanzen in Plasma verschiedener Spezies wird durch die Ultrafiltrationsmethode bestimmt. Dabei wird die Substanz aus einer Acetonitril-Stammlösung dem Plasma üblicherweise in einer Endkonzentration von 1000 ng/ml zupipettiert, wobei die Endkonzentration an Acetonitril 1 % nicht übersteigt. Das Plasma wird durch eine Cellulose-Membran filtriert (z.B. Centrifree Micropartition Device, Fa. Amicon-Millipore, Witten), um das Protein und die an das Protein gebundene Substanz abzutrennen. Die Konzentration der ungebundenen Substanz im Filtrat wird bestimmt. Zusätzlich wird auf analoge Weise die unspezifische Bindung der Substanz (ohne Plasma) an die Filtrationseinheit bestimmt. Diese unspezifische Bindung an die Filtereinheit, die 20% nicht übersteigen sollte, wird bei der Berechnung der Proteinbindung der Substanz berücksichtigt.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 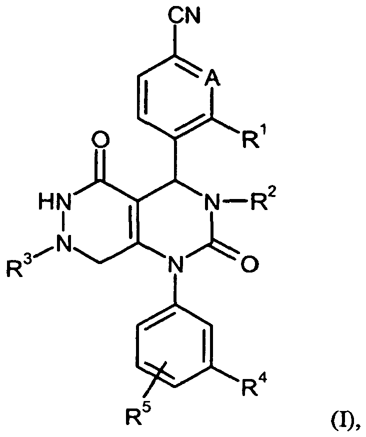 in welcher
A für CH oder N steht,
R¹ für Wasserstoff, Halogen, Cyano, Nitro, (C₁-C₆)-Alkyl, Difluormethyl, Trifluormethyl, (C₁-C₆)-Alkoxy, Difluormethoxy, Trifluormethoxy, Amino, Mono- oder Di-(C₁-C₆)-alkylammo
oder
für eine Gruppe der Formel -NH-C(=O)-R⁶, -NH-C(=O)-NHR⁶, -NH-SO₂-R⁷ oder -S(O)ₙ-R⁸ steht, worin
R⁶ Wasserstoff oder (C₁-C₆)-Alkyl bedeutet,
R⁷ (C₁-C₆)-Alkyl bedeutet,
R⁸ (C₁-C₆)-Alkyl, das mit Hydroxy, (C₁-C₄)-Alkoxy, Amino, Mono- oder Di-(C₁-C₄)-alkylamino, Hydroxycarbonyl, Aminocarbonyl, (C₃-C₆)-Cycloalkyl oder Phenyl substituiert sein kann, oder (C₂-C₆)-Alkenyl, (C₃-C₆)-Cycloalkyl oder Phenyl bedeutet,
wobei die genannten (C₃-C₆)-Cycloalkyl-Gruppen bis zu zweifach, gleich oder verschieden, mit (C₁-C₄)-Alkyl, Hydroxy und (C₁-C₄)-Alkoxy substituiert sein können
und
die genannten Phenyl-Gruppen bis zu zweifach, gleich oder verschieden, mit Fluor, Chlor, Cyano, (C₁-C₄)-Alkyl, Difluormethyl, Trifluormethyl, (C₁-C₄)-Alkoxy, Difluormethoxy und Trifluormethoxy substituiert sein können,
und
n die Zahl 0, 1 oder 2 bedeutet,
R² für Wasserstoff, für (C₁-C₆)-Alkyl oder (C₂-C₆)-Alkenyl, welche jeweils bis zu dreifach mit Fluor substituiert sein können, oder für Phenyl, Pyridyl oder Pyrimidinyl steht,
wobei Phenyl, Pyridyl und Pyrimidinyl ihrerseits bis zu zweifach, gleich oder verschieden, mit Fluor, Chlor, Cyano, (C₁-C₄)-Alkyl, Trifluormethyl, (C₁-C₄)-Alkoxy und Trifluormethoxy substituiert sein können,
oder
R² für eine Gruppe der Formel -C(=O)-O-R⁹, -L¹-C(=O)-O-R¹⁰, -L²-C(=O)-NR¹¹R¹², -L²-SO₂-NR¹¹R¹², -L²-C(=O)-NR¹³-NR¹¹R¹² oder -L²-SO₂-R¹⁴ steht, worin
L¹ (C₁-C₆)-Alkandiyl bedeutet,
L² eine Bindung oder (C₁-C₆)-Alkandiyl bedeutet,
R⁹ (C₁-C₆)-Alkyl bedeutet,
R¹⁰ Wasserstoff oder (C₁-C₆)-Alkyl bedeutet,
R¹¹ und R¹² gleich oder verschieden sind und unabhängig voneinander Wasserstoff, (C₁-C₆)-Alkyl, (C₃-C₆)-Cycloalkyl oder 4- bis 6-gliedriges Heterocyclyl bedeuten,
wobei (C₁-C₆)-Alkyl, (C₃-C₆)-Cycloalkyl und 4- bis 6-gliedriges Heterocyclyl ihrerseits bis zu zweifach, gleich oder verschieden, mit Fluor, Hydroxy, (C₁-C₄)-Alkoxy, Oxo, Amino, Mono- oder Di-(C₁-C₄)-alkylamino, Hydroxycarbonyl, (C₁-C₄)-Alkoxycarbonyl und Aminocarbonyl substituiert sein können und wobei in (C₁-C₆)-Alkyl eine CH₂-Gruppe, soweit in einer chemisch stabilen Verbindung resultierend, gegen ein O-Atom ausgetauscht sein kann,
oder
R¹¹ und R¹² zusammen mit dem Stickstoffatom, an das sie gebunden sind, einen 4-bis 6-gliedrigen Heterocyclus bilden, der ein weiteres Ring-Heteroatom aus der Reihe N, O, S, SO oder SO₂ enthalten und bis zu zweifach, gleich oder verschieden, mit (C₁-C₄)-Alkyl, Hydroxy, (C₁-C₄)-Alkoxy, Oxo, Amino, Mono- und Di-(C₁-C₄)-alkylamino substituiert sein kann, wobei (C₁-C₄)-Alkyl seinerseits mit Hydroxy oder (C₁-C₄)-Alkoxy substituiert sein kann,
R¹³ Wasserstoff oder (C₁-C₄)-Alkyl bedeutet
und
R¹⁴ (C₁-C₆)-Alkyl, (C₃-C₆)-Cycloalkyl, Phenyl oder 5- oder 6-gliedriges Heteroaryl bedeutet,
wobei (C₁-C₆)-Alkyl mit Fluor, Chlor, Hydroxy, (C₁-C₄)-Alkoxy, Mono- oder Di-(C₁-C₄)-alkylamino substituiert sein kann
und
Phenyl und 5- oder 6-gliedriges Heteroaryl ihrerseits bis zu zweifach, gleich oder verschieden, mit Fluor, Chlor, Cyano, (C₁-C₄)-Alkyl, Trifluormethyl, (C₁-C₄)-Alkoxy und Trifluormethoxy substituiert sein können,
R³ für (C₁-C₆)-Alkyl oder (C₂-C₆)-Alkenyl, welche jeweils mit Hydroxy, (C₁-C₄)-Alkoxy, Amino, Mono- oder Di-(C₁-C₄)-alkylamino, Hydroxycarbonyl, (C₁-C₄)-Alkoxycarbonyl, Aminocarbonyl oder Mono- oder Di-(C₁-C₄)-alkylaminocarbonyl substituiert sein können,
oder
für eine Gruppe der Formel -L³-R¹⁵ steht, worin
L³ eine Bindung oder (C₁-C₄)-Alkandiyl bedeutet
und
R¹⁵ (C₃-C-₇)-Cycloalkyl, 4- bis 7-gliedriges Heterocyclyl, Phenyl oder 5- oder 6-gliedriges Heteroaryl bedeutet,
wobei (C₃-C₇)-Cycloalkyl und 4- bis 7-gliedriges Heterocyclyl ihrerseits bis zu zweifach, gleich oder verschieden, mit (C₁-C₄)-Alkyl, Oxo, Hydroxy und (C₁-C₄)-Alkoxy substituiert sein können
und
Phenyl und 5- oder 6-gliedriges Heteroaryl ihrerseits bis zu zweifach, gleich oder verschieden, mit Fluor, Chlor, Cyano, (C₁-C₄)-Alkyl, Trifluormethyl, (C₁-C₄)-Alkoxy, Trifluormethoxy und Amino substituiert sein können,
R⁴ für Nitro oder Trifluormethyl steht
und
R⁵ für Wasserstoff, Fluor oder Chlor steht,
sowie ihre Salze, Solvate und Solvate der Salze.

2. Verbindung der Formel (I) nach Anspruch 1, in welcher
A für CH steht,
R¹ für Wasserstoff, Fluor, Chlor, Cyano, Nitro, (C₁-C₄)-Alkyl, Difluormethyl, Trifluormethyl, (C₁-C₄)-Alkoxy, Difluormethoxy, Trifluormethoxy, Amino, Mono- oder Di-(C₁-C₄)-alkylamino
oder
für eine Gruppe der Formel -NH-C(=O)-R⁶, -NH-SO₂-R⁷ oder -SO₂-R⁸ steht, worin
R⁶ und R⁷ jeweils (C₁-C₄)-Alkyl bedeuten
und
R⁸ (C₁-C₄)-Alkyl, das mit Hydroxy, (C₁-C₄)-Alkoxy, Hydroxycarbonyl, Aminocarbonyl, (C₃-C₆)-Cycloalkyl oder Phenyl substituiert sein kann, oder (C₃-C₆)-Cycloalkyl oder Phenyl bedeutet,
wobei die genannten Phenyl-Gruppen bis zu zweifach, gleich oder verschieden, mit Fluor, Chlor, Cyano, Methyl, Trifluormethyl, Methoxy und Trifluormethoxy substituiert sein können,
R² für Wasserstoff, (C₁-C₄)-Alkyl oder (C₂-C₄)-Alkenyl
oder
für eine Gruppe der Formel -L¹-C(=O)-O-R¹⁰, -L²-C(=O)-NR¹¹R¹² oder -L²-SO₂-R¹⁴ steht, worin
L¹ Methylen oder Ethan-1,2-diyl bedeutet,
L² eine Bindung, Methylen, Ethan-1,1-diyl oder Ethan-1,2-diyl bedeutet,
R¹⁰ Wasserstoff oder (C₁-C₄)-Alkyl bedeutet,
R¹¹ Wasserstoff oder (C₁-C₄)-Alkyl, das mit Hydroxy oder (C₁-C₄)-Alkoxy substituiert sein kann, bedeutet,
R¹² Wasserstoff, (C₁-C₆)-Alkyl oder (C₃-C₆)-Cycloalkyl bedeutet,
wobei (C₁-C₆)-Alkyl bis zu zweifach, gleich oder verschieden, mit Hydroxy, (C₁-C₄)-Alkoxy, Hydroxycarbonyl, (C₁-C₄)-Alkoxycarbonyl und Aminocarbonyl substituiert sein kann und in (C₁-C₆)-Alkyl eine CH₂-Gruppe, soweit in einer chemisch stabilen Verbindung resultierend, gegen ein O-Atom ausgetauscht sein kann,
oder
R¹¹ und R¹² zusammen mit dem Stickstoffatom, an das sie gebunden sind, einen 5- oder 6-gliedrigen Heterocyclus bilden, der ein weiteres Ring-Heteroatom aus der Reihe N, O oder S enthalten und mit (C₁-C₄)-Alkyl, Hydroxy, (C₁-C₄)-Alkoxy oder Oxo substituiert sein kann,
wobei (C₁-C₄)-Alkyl seinerseits mit Hydroxy oder (C₁-C₄)-Alkoxy substituiert sein kann,
und
R¹⁴ (C₁-C₄)-Alkyl, (C₃-C₆)-Cycloalkyl oder Phenyl bedeutet,
wobei Phenyl bis zu zweifach, gleich oder verschieden, mit Fluor, Chlor, Cyano, Methyl, Trifluormethyl, Methoxy und Trifluormethoxy substituiert sein kann,
R³ für (C₁-C₄)-Alkyl, das mit Hydroxy, (C₁-C₄)-Alkoxy, Hydroxycarbonyl, (C₁-C₄)-Alkoxycarbonyl, Aminocarbonyl, Mono- oder Di-(C₁-C₄)-alkylaminocarbonyl oder 5- oder 6-gliedrigem Heteroaryl substituiert sein kann, für (C₂-C₄)-Alkenyl
oder
für eine Gruppe der Formel -L³-R¹⁵ steht, worin
L³ eine Bindung oder (C₁-C₄)-Alkandiyl bedeutet
und
R¹⁵ (C₃-C₇)-Cycloalkyl, 4- bis 6-gliedriges Heterocyclyl oder Phenyl bedeutet,
wobei 4- bis 6-gliedriges Heterocyclyl seinerseits mit Oxo substituiert sein kann
und
Phenyl seinerseits bis zu zweifach, gleich oder verschieden, mit Fluor, Chlor, Cyano, Methyl, Trifluormethyl, Methoxy und Trifluormethoxy substituiert sein kann,
R⁴ für Trifluormethyl steht
und
R⁵ für Wasserstoff oder Fluor steht,
sowie ihre Salze, Solvate und Solvate der Salze.

3. Verbindung der Formel (I) nach Anspruch 1 oder 2, in welcher
A für CH steht,
R¹ für Wasserstoff, Fluor, Chlor, Nitro, Methyl, Difluormethyl, Trifluormethyl, Methoxy, Trifluormethoxy oder eine Gruppe der Formel -SO₂-R⁸ steht, worin
R⁸ (C₁-C₄)-Alkyl, das mit Hydroxy, Methoxy oder Ethoxy substituiert sein kann, bedeutet,
R² für Wasserstoff, (C₁-C₄)-Alkyl oder eine Gruppe der Formel -CH₂-C(=O)-O-R10 oder -CH₂-C(=O)-NR¹¹R¹² steht, worin
R¹⁰ (C₁-C₄)-Alkyl bedeutet,
R¹¹ Wasserstoff oder Methyl bedeutet,
R¹² Wasserstoff oder (C₁-C₄)-Alkyl, das mit Hydroxy, Methoxy oder Ethoxy substituiert sein kann, bedeutet
oder
R¹¹ und R¹² zusammen mit dem Stickstoffatom, an das sie gebunden sind, einen Pyrrolidino-, Piperidino- oder Morpholino-Ring bilden,
R³ für (C₁-C₄)-Alkyl, das mit Hydroxy, Pyrrolidino, Piperidino, Morpholino oder Pyridyl substituiert sein kann, für Allyl oder für eine Gruppe der Formel -L³-R¹⁵ steht, worin
L³ eine Bindung, Methylen oder Ethan-1,2-diyl bedeutet
und
R¹⁵ (C₃-C₇)-Cycloalkyl oder Phenyl bedeutet,
wobei Phenyl seinerseits bis zu zweifach, gleich oder verschieden, mit Fluor, Chlor, Cyano, Methyl oder Trifluormethyl substituiert sein kann,
R⁴ für Trifluormethyl steht
und
R⁵ für Wasserstoff steht,
sowie ihre Salze, Solvate und Solvate der Salze.

4. Verbindung der Formel (I) nach Anspruch 1, 2 oder 3, in welcher
A für CH steht,
R¹ für Wasserstoff, Trifluormethyl oder Methylsulfonyl steht,
R² für Wasserstoff oder eine Gruppe der Formel -CH₂-C(=O)-NR¹¹R¹² steht, worin
R¹¹ und R¹² unabhängig voneinander Wasserstoff oder Methyl bedeuten
oder
R¹¹ und R¹² zusammen mit dem Stickstoffatom, an das sie gebunden sind, einen Pyrrolidino-Ring bilden,
R³ für Methyl, Ethyl, 2-Hydroxyethyl oder 2-(Morpholin-4-yl)ethyl steht,
R⁴ für Trifluormethyl steht
und
R⁵ für Wasserstoff steht,
sowie ihre Salze, Solvate und Solvate der Salze.

5. Verfahren zur Herstellung von Verbindungen der Formel (I), wie in den Ansprüchen 1 bis 4 definiert, **dadurch gekennzeichnet, dass** man zunächst eine Verbindung der Formel (II) 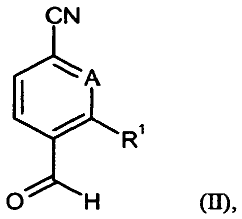 in welcher A und R¹ die in den Ansprüchen 1 bis 4 angegebenen Bedeutungen haben,
in Gegenwart einer Säure oder eines Säureanhydrids in einer 3-Komponenten-EintopfReaktion oder sequentiell mit einem Acetessigsäureester der Formel (III) 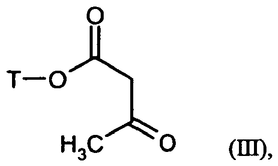 in welcher
T für Methyl oder Ethyl steht,
und einem Phenylhamstoff-Derivat der Formel (IV) 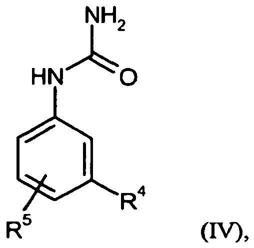 in welcher R⁴ und R⁵ die in den Ansprüchen 1 bis 4 angegebenen Bedeutungen haben,
zu einer Verbindung der Formel (V-A) 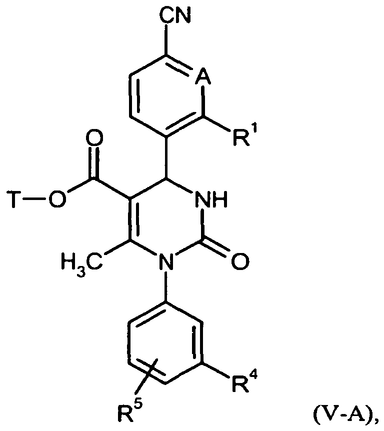 in welcher A, T, R¹, R⁴ und R⁵ jeweils die oben angegebenen Bedeutungen haben,
kondensiert und diese dann
[A] im Falle, dass R² in Formel (I) für Wasserstoff steht, in einem inerten Lösungsmittel zu einer Verbindung der Formel (VI-A) 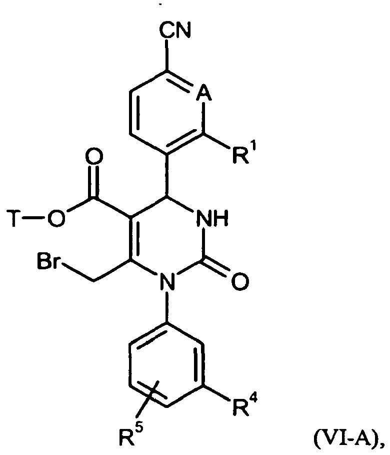 in welcher A, T, R¹, R⁴ und R⁵ jeweils die oben angegebenen Bedeutungen haben, bromiert und nachfolgend mit einem Hydrazin-Derivat der Formel (VII)
R³-NH-NH₂ (VII).
in welcher R³ die in den Ansprüchen 1 bis 4 angegebene Bedeutung hat,
unter Ausbildung eines Sechsrings zu einer Verbindung der Formel (I-A) 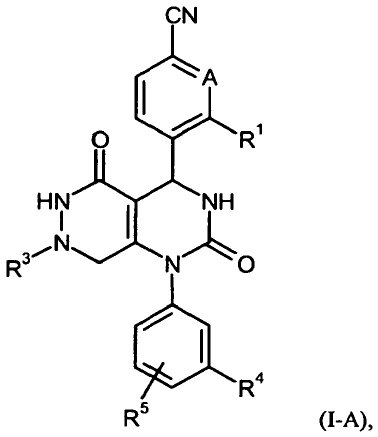 in welcher A, R¹, R³, R⁴ und R⁵ jeweils die oben angegebenen Bedeutungen haben, umsetzt
oder
[B] im Falle, dass R² in Formel (I) von Wasserstoff verschieden ist, zunächst mit einer Verbindung der Formel (VIII)
R^{2A}-X (VIII),
in welcher
R^{2A} die in den Ansprüchen 1 bis 4 angegebene Bedeutung von R² hat, jedoch nicht für Wasserstoff steht,
und
X für eine Abgangsgruppe wie beispielsweise Halogen, Mesylat, Tosylat oder Triflat steht,
in Gegenwart einer Base zu einer Verbindung der Formel (V-B) 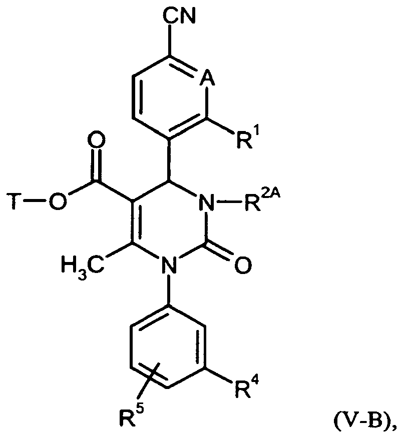 in welcher A, T, R¹, R^{2A}, R⁴ und R⁵ jeweils die oben angegebenen Bedeutungen haben,
reagiert, danach in einem inerten Lösungsmittel zu einer Verbindung der Formel (VI-B) 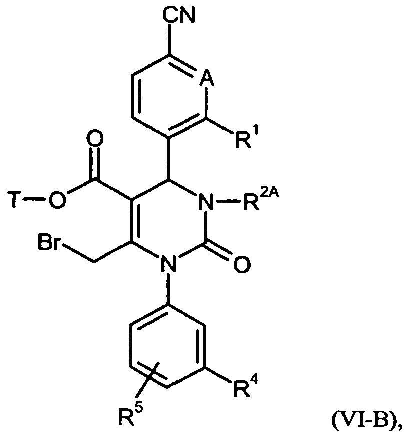 in welcher A, T, R¹, R^{2A}, R⁴ und R⁵ jeweils die oben angegebenen Bedeutungen haben,
bromiert und nachfolgend mit einem Hydrazin-Derivat der Formel (VII)
R³-NH-NH₂ (VII),
in welcher R³ die in den Ansprüchen 1 bis 4 angegebene Bedeutung hat,
unter Cyclisierung zu einer Verbindung der Formel (I-B) 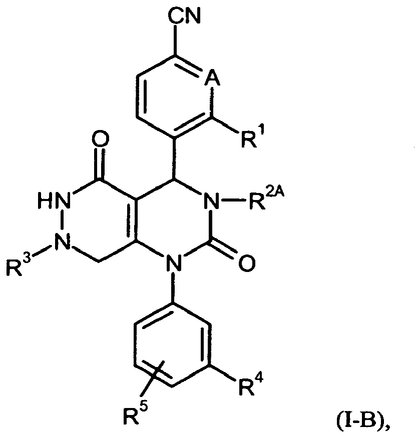 in welcher A, R¹, R^{2A}, R³, R⁴ und R⁵ jeweils die oben angegebenen Bedeutungen haben,
umsetzt
und gegebenenfalls die so erhaltenen Verbindungen der Formel (I-A) bzw. (I-B) nach dem Fachmann bekannten Methoden in ihre Enantiomere und/oder Diastereomere trennt und/oder mit den entsprechenden (i) Lösungsmitteln und/oder (ii) Basen oder Säuren in ihre Solvate, Salze und/oder Solvate der Salze überführt.

6. Verbindung, wie in einem der Ansprüche 1 bis 4 definiert, zur Behandlung und/oder Prävention von Krankheiten.

7. Verbindung, wie in einem der Ansprüche 1 bis 4 definiert, zur Verwendung in einem Verfahren zur Behandlung und/oder Prävention von pulmonaler arterieller Hypertonie (PAH) und anderen Formen der pulmonalen Hypertonie (PH), von chronisch-obstruktiven Lungenerkrankungen (COPD), der akuten Lungenschädigung (ALI), des akuten Atemwegssyndroms (ARDS), des Lungenemphysems, der alpha-1-Antitrypsin-Defizienz (AATD) und der zystischen Fibrose (CF).

8. Verwendung einer Verbindung, wie in einem der Ansprüche 1 bis 4 definiert, zur Herstellung eines Arzneimittels zur Behandlung und/oder Prävention von pulmonaler arterieller Hypertonie (PAH) und anderen Formen der pulmonalen Hypertonie (PH), von chronisch-obstruktiven Lungenerkrankungen (COPD), der akuten Lungenschädigung (ALI), des akuten Atemwegssyndroms (ARDS), des Lungenemphysems, der alpha-1-Antitrypsin-Defizienz (AATD) und der zystischen Fibrose (CF).

9. Arzneimittel enthaltend eine Verbindung, wie in einem der Ansprüche 1 bis 4 definiert, in Kombination mit einem oder mehreren inerten, nicht-toxischen, pharmazeutisch geeigneten Hilfsstoffen.

10. Arzneimittel enthaltend eine Verbindung, wie in einem der Ansprüche 1 bis 4 definiert, in Kombination mit einem oder mehreren weiteren Wirkstoffen ausgewählt aus der Gruppe bestehend aus Kinase-Inhibitoren, Stimulatoren und Aktivatoren der löslichen Guanylatcyclase, Prostacyclin-Analoga, Endothelinrezeptor-Antagonisten, Phosphodiesterase-Inhibitoren, beta-adrenerge Rezeptor-Agonisten, Inhibitoren der Matrix-Metalloproteasen, Serotoninrezeptor-Antagonisten, Anticholinergika und Glucocorticoide.

11. Arzneimittel nach Anspruch 9 oder 10 zur Behandlung und/oder Prävention von pulmonaler arterieller Hypertonie (PAH) und anderen Formen der pulmonalen Hypertonie (PH), von chronisch-obstruktiven Lungenerkrankungen (COPD), der akuten Lungenschädigung (ALI), des akuten Atemwegssyndroms (ARDS), des Lungenemphysems, der alpha-1-Antitrypsin-Defizienz (AATD) und der zystischen Fibrose (CF).

12. Verbindung oder Arzneimittel zum Verwendung in einem Verfahren zur Behandlung und/oder Prävention von pulmonaler arterieller Hypertonie (PAH) und anderen Formen der pulmonalen Hypertonie (PH), von chronisch-obstruktiven Lungenerkrankungen (COPD), der akuten Lungenschädigung (ALI), des akuten Atemwegssyndroms (ARDS), des Lungenemphysems, der alpha-1-Antitrypsin-Defizienz (AATD) und der zystischen Fibrose (CF) bei Menschen und Tieren unter Verwendung einer wirksamen Menge mindestens einer Verbindung, wie in einem der Ansprüche 1 bis 4 definiert, oder eines Arzneimittels, wie in einem der Ansprüche 9 bis 11 definiert.

## Claims

1. Compound of the formula (I) 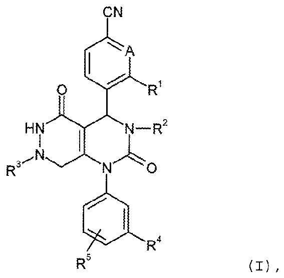 in which
A represents CH or N,
R¹ represents hydrogen, halogen, cyano, nitro, (C₁- C₆)-alkyl, difluoromethyl, trifluoromethyl, (C₁-C₅)-alkoxy, difluoromethoxy, trifluoromethoxy, amino, mono- or di-(C₁-C₆)-alkylamino
or
represents a group of the formula -NH-C(=O)-R⁶, -NH-C(=O)-NHR⁶, -NH-SO₂-R⁷ or -S(O)ₙ-R⁸ in which
R⁶ represents hydrogen or (C₁-C₆) -alkyl,
R⁷ represents (C₁-C₆)-alkyl,
R⁸ represents (C₁-C₆)-alkyl which may be substituted by hydroxyl, (C₁-C₄)-alkoxy, amino, mono- or di-(C₁-C₄) -alkylamino, hydroxycarbonyl, aminocarbonyl, (C₃-C₆)-cycloalkyl or phenyl, or represents (C₂-C₆) -alkenyl, (C₃-C₆)-cycloalkyl or phenyl,
where the (C₃-C₆)-cycloalkyl groups mentioned may be substituted up to two times by identical or different substituents from the group consisting of (C₁-C₄) -alkyl, hydroxyl and (C₁-C₄)-alkoxy
and
the phenyl groups mentioned may be substituted up to two times by identical or different substituents from the group consisting of fluorine, chlorine, cyano, (C₁-C₄)-alkyl, difluoromethyl, trifluoromethyl, (C₁-C₄)-alkoxy, difluoromethoxy and trifluoromethoxy,
and
n represents the number 0, 1 or 2,
R² represents hydrogen, represents (C₁-C₆)-alkyl or (C₂-C₆)-alkenyl-, each of which may be substituted up to three times by fluorine, or represents phenyl, pyridyl or pyrimidinyl,
where phenyl, pyridyl and pyrimidinyl for their part may be substituted up to two times by identical or different substituents from the group consisting of fluorine, chlorine, cyano, (C₁-C₄)-alkyl, trifluoromethyl, (C₁-C₄)-alkoxy and trifluoromethoxy,
or
R² represents a group of the formula -C(=C)-O-R⁹, -L¹-(=O)-O-R¹⁰, -L²-C(=O)-NR¹¹R¹², -L²-SO₂-NR¹¹R¹², -L²-C(=O)-NR¹³-NR¹¹R¹² or -L²-SO₂-R¹⁴ in which
L¹ represents (C₁-C₆)-alkanediyl,
L² represents a bond or (C₁-C₆)-alkanediyl,
R⁹ represents (C₁-C₆)-alkyl,
R¹⁰ represents hydrogen or (C₁-C₆)-alkyl,
R¹¹ and R¹² are identical or different and independently of one another represent hydrogen, (C₁-C₆)-alkyl, (C₃-C₆)-cycloalkyl or 4- to 6-membered heterocyclyl,
where (C₁-C₆)-alkyl, (C₃-C₆) -cycloalkyl and 4- to 6-membered heterocyclyl for their part may be substituted up to two times by identical or different substituents from the group consisting of fluorine, hydroxyl, (C₁-C₄)-alkoxy, oxo, amino, mono- or di-(C₁-C4)-alkylamino, hydroxycarbonyl, (C₁-C₄)-alkox-ycarbonyl and aminocarbonyl and where in (C₁-C₆)-alkyl a CH₂ group may be exchanged for an oxygen atom if this results in a chemically stable compound,
or
R¹¹ and R¹² together with the nitrogen atom to which they are attached form a 4- to 6-membered heterocycle which may contain a further ring heteroatom from the group consisting of N, O, S, SO and SO₂ and which may be substituted up to two times by identical or different substituents from the group consisting of (C₁-C₄)-alkyl, hydroxyl, (C₁-C₄)-alkoxy, oxo, amino, mono- and di-(C₁-C₄) -alkylamino,
where (C₁-C₄)-alkyl for its part may be substituted by hydroxyl or (C₁-C₄)-alkoxy,
R¹³ represents hydrogen or (C₁-C₄)-alkyl
and
R¹⁴ represents (C₁-C₆) -alkyl, (C₃-C₆)-cycloalkyl, phenyl or 5- or 6-membered heteroaryl,
where (C₁-C₆)-alkyl may be substituted by fluorine, chlorine, hydroxyl, (C₁-C₄) alkoxy, mono- or di-(C₁-C₄)-alkylamino
and
phenyl and 5- or 6-membered heteroaryl for their part may be substituted up to two times by identical or different substituents from the group consisting of fluorine, chlorine, cyano, (C₁-C₄)-alkyl, trifluoromethyl, (C₁-C₄)-alkoxy and trifluoromethoxy,
R³ represents (C₁-C₆)-alkyl or (C₂-C₆)-alkenyl, each of which may be substituted by hydroxyl, (C₁-C₄)-alkoxy, amino, mono- or di-(C₁-C₄)-alkylamino, hydroxycarbonyl, (C₁-C₄)-alkoxycarbonyl, aminocarbonyl or mono- or di-(C₁-C₄)-alkylaminocarbonyl, or represents a group of the formula -L³-R¹⁵ in which
L³ represents a bond or (C₁-C₄)-alkanediyl
and
R¹⁵ represents (C₃-C₄)-cycloalkyl, 4- to 7-membered heterocyclyl, phenyl or 5- or 6-membered heteroaryl,
where (C₃-C₇)-cycloalkyl- and 4- to 7-membered heterocyclyl for their part may be substituted up to two times by identical or different substituents from the group consisting of (C₁-C₄)-alkyl, oxo, hydroxyl and (C₁-C₄)-alkoxy
and
phenyl and 5- or 6-membered heteroaryl for their part may be substituted up to two times by identical or different substituents from the group consisting of fluorine, chlorine, cyano, (C₁-C₄)-alkyl, trifluoromethyl, (C₁-C₄)-alkoxy, trifluoromethoxy and amino,
R⁴ represents nitro or trifluoromethyl
and
R⁵ represents hydrogen, fluorine or chlorine,
and its salts, solvates and solvates of the salts.

2. Compound of the formula (I) according to Claim 1 in which
A represents CH,
R¹ represents hydrogen, fluorine, chlorine, cyano, nitro, (C₁-C₄)-alkyl, difluoromethyl, trifluoromethyl, (C₁-C₄)-alkoxy, difluoromethoxy, trifluoromethoxy, amino, mono- or di-(C₁-C₄)-alkylamino
or
represents a group of the formula -NH-C(=O)-R⁶, -NH-SO₂-R⁷ or -SO₂-R⁸ in which
R⁶ and R⁷ each represents (C₁-C₄)-alkyl
and
R⁸ represents (C₁-C₄)-alkyl which may be substituted by hydroxyl, (C₁-C₄)-alkoxy, hydroxycarbonyl, aminocarbonyl, (C₃-C₆)-cycloalkyl or phenyl, or represents (C₃-C₆)-cycloalkyl or phenyl,
where the mentioned phenyl groups may be substituted up to two times by identical or different substituents from the group consisting of fluorine, chlorine, cyano, methyl, trifluoromethyl, methoxy and trifluoromethoxy,
R² represents hydrogen, (C₁-C₄)-alkyl or (C₂-C₄)-alkenyl
or
represents a group of the formula -L⁴-C(=O)-OR¹⁰ -L²-C(=C)-NR¹¹R¹² or -L²SO₂-R¹⁴ in which
L¹ represents methylene or ethane-1,2-diyl,
L² represents a bond, methylene, ethane-1,1-diyl or ethane-1,2-diyl,
R¹⁰ represents hydrogen or (C₁-C₄)-alkyl,
R¹¹ represents hydrogen or (C₁-C₄) -alkyl which may be substituted by hydroxyl or (C₁-C₄)-Alkoxy,
R¹² represents hydrogen, (C₁-C₆)-alkyl or (C₃-C₆)-cycloalkyl,
where (C₁-C₆)-alkyl may be substituted up to two times by identical or different substituents from the group consisting of hydroxyl, (C₁-C₄)-alkoxy, hydroxycarbonyl, (C₁-C₄)-alkoxycarbonyl and aminocarbonyl and where in (C₁-C₅)-alkyl a CH₂ group may be exchanged for an oxygen atom if this results in a chemically stable compound,
or
R¹¹ and R¹² together with the nitrogen atom to which they are attached form a 5- or 6-membered heterocycle which may contain a further ring heteroatom from the group consisting of N, O and and which may be substituted by (C₁-C₄)-alkyl, hydroxyl, (C₁-C₄) -alkoxy or oxo,
where (C₁-C₄)-alkyl for its part may be substituted by hydroxy or (C₁-C₄)-alkoxy,
and
R¹⁴ represents (C₁-C₄)-alkyl, (C₃-C₆)-cycloalkyl or phenyl,
where phenyl may be substituted up to two times by identical or different substituents from the group consisting of fluorine, chlorine, cyano, methyl, trifluoromethyl, methoxy and trifluoromethoxy,
R³ represents (C₁-C₄)-alkyl which may be substituted by hydroxyl, (C₁-C4)-alkoxy, hydroxycarbonyl, (C₁-C₄)-alkoxycarbonyl, aminocarbonyl, mono- or di-(C₁-C₄)-alkylaminocarbonyl or 5- or 6-membered heteroaryl, represents (C₂-C₄) -alkenyl
or
represents a group of the formula -L³-R¹⁵ in which
L³ represents a bond or (C₁-C₄)-alkanediyl
and
R¹⁵ represents (C₃-C₇)-cycloalkyl, 4- to 6-membered heterocyclyl or phenyl,
where 4- to 6-membered heterocyclyl for its part may be substituted by oxo
and
phenyl for its part may be substituted up to two times by identical or different substituents from the group consisting of fluorine, chlorine, cyano, methyl, trifluoromethyl, methoxy and trifluoromethoxy,
R⁴ represents trifluoromethyl
and
R⁶ represents hydrogen or fluorine,
and its salts, solvates and solvates of the salts.

3. Compound of the formula (I) according to Claim 1 or 2 in which
A represents CH,
R¹ represents hydrogen, fluorine, chlorine, Nitro, methyl, difluoromethyl, trifluoromethyl, methoxy, trifluoromethoxy or a group of the formula -SO₂-R⁸ in which
R⁸ represents (C₁-C₄)-alkyl which may be substituted by hydroxyl, methoxy or ethoxy,
R² represents hydrogen, (C₁-C₄)-alkyl or a group of the formula -CH₂-C(=O)-O-R¹⁰ or -CH₂-C(=O)-NR¹¹R¹² in which
R¹⁰ represents (C₁-C₄)-alkyl,
R¹¹ represents hydrogen or methyl,
R¹² represents hydrogen or (C₁-C₄) -alkyl which may be substituted by hydroxyl, methoxy or ethoxy
or
R¹¹ and R¹² together with the nitrogen atom to which they are attached form a pyrrolidino, piperidino or morpholino ring,
R³ represents (C₁-C₄)-alkyl which may be substituted by hydroxyl, pyrrolidino, piperidino, morpholino or pyridyl, represents allyl or represents a group of the formula - L³-R¹⁵ in which
L³ represents a bond, methylene or ethane-1,2-diyl
and R¹⁵ represents (C₃-C₇)-cycloalkyl or phenyl,
where phenyl for its part may be substituted up to two times by identical or different substituents from the group consisting of fluorine, chlorine, cyano, methyl and trifluoromethyl,
R⁴ represents trifluoromethyl
and
R⁵ represents hydrogen,
and its salts, solvates and solvates of the salts.

4. Compound of the formula (I) according to any of Claims 1 to 3 in which
A represents CH,
R¹ represents hydrogen, trifluoromethyl or methylsulphonyl,
R² represents hydrogen or a group of the formula -CH₂-C(=O)-NR¹¹R¹² in which
R¹¹ and R¹² independently of one another represent hydrogen or methyl
or
R¹¹ and R¹² together with the nitrogen atom to which they are attached form a pyrrolidino ring,
R³ represents methyl, ethyl, 2-hydroxyethyl or 2-(morpholin-4-yl)ethyl,
R⁴ represents trifluoromethyl,
and
R⁵ represents hydrogen,
and its salts, solvates and solvates of the salts.

5. Process for preparing compounds of the formula (I) as defined in Claims 1 to 4, **characterized in that** initially a compound of the formula (II) 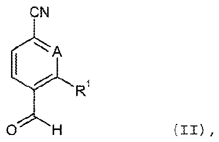 in which A and R¹ have the meanings given in Claims 1 to 4
is condensed in the presence of an acid or an acid anhydride in a 3-component one-pot reaction or sequentially with an acetoacetic ester of the formula (III) 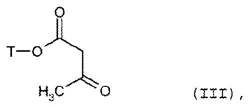 in which
T represents methyl or ethyl,
and a phenylurea derivative of the formula (IV) 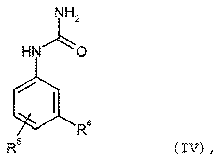 in which R⁴ and R⁵ have the meanings given in Claims 1 to 4,
to give a compound of the formula (V-A) 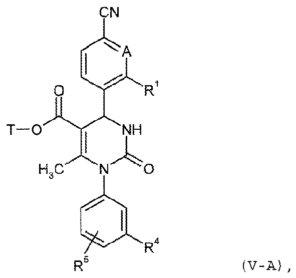 in which A, T, R¹, R⁴ and R⁵ each have the meanings given above,
and this compound is then
[A] in the case that R² in formula (I) represents hydrogen, brominated in an inert solvent to give a compound of the formula (VI-A) 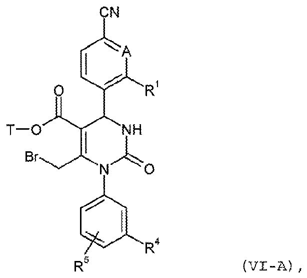 in which A, T, R¹, R⁴ and R⁵ each have the meanings given above,
and subsequently reacted with a hydrazine derivative of the formula (VII)
R³-NH-NH₂ (VII),
in which R³ has the meaning given in Claims 1 to 4,
with formation of a six-membered ring to give a compound of the formula (I-A) 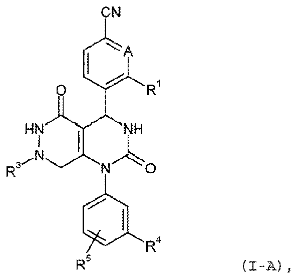 in which A, R¹, R³, R⁴ and R⁵ each have the meanings given above,
or
[B] in the case that R² in formula (I) is different from hydrogen, initially reacted with a compound of the formula (VIII)
R^{2A}-X (VIII),
in which
R^{2A} has the meaning of R² given in Claims 1 to 4, but does not represent hydrogen,
and
X represents a leaving group such as, for example, halogen, mesylate, tosylate or triflate,
in the presence of a base to give a compound of the formula (V-B) 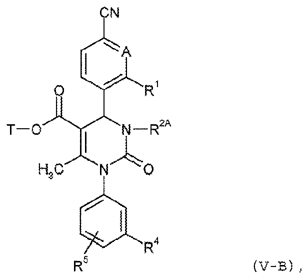 in which A, T, R¹, R^{2A}, R⁴ and R⁵ each have the meanings given above,
then brominated in an inert solvent to give a compound of the formula (VI-B) 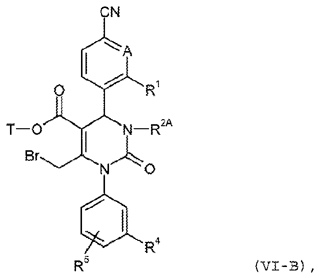 in which A, T, R¹, R^{2A}, R⁴ and R⁵ each have the meanings given above,
and subsequently reacted with a hydrazine derivative of the formula (VII)
R³-NH-NHz (VII),
in which R³ has the meaning given in Claims 1 to 4,
with cyclization to give a compound of the formula (I-B) 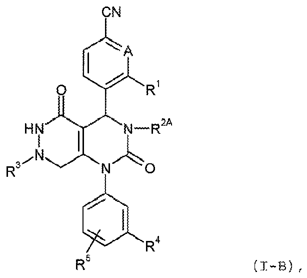 in which A, R¹, R^{2A} R³, R⁴ and R⁵ each have the meanings given above,
and the compound of the formula (I-A) or (I-B) obtained in this manner is, if appropriate, separated by methods known to the person skilled in the art into its enantiomers and/or diastereomers and/or converted with the appropriate (i) solvents and/or (ii) bases or acids into its solvates, salts and/or solvates of the salts.

6. Compound as defined in any of Claims 1 to 4 for the treatment and/or prevention of diseases.

7. Compound as defined in any of Claims 1 to 4 for use in a method for the treatment and/or prevention of pulmonary arterial hypertension (PAH) and other forms of pulmonary hypertension (PH), of chronic-obstructive pulmonary diseases (COPD), of acute lung injury (ALI), of acute respiratory distress syndrome (ARDS), of pulmonary emphysema, of alpha-1 antitrypsin deficiency (AATD) and of cystic fibrosis (CF).

8. Use of a compound as defined in any of Claims 1 to 4 for preparing a medicament for the treatment and/or prevention of pulmonary arterial hypertension (PAH) and other forms of pulmonary hypertension (PH), of chronic-obstructive pulmonary diseases (COPD), of acute lung injury (ALI), of acute respiratory distress syndrome (ARDS), of pulmonary emphysema, of alpha-1 antitrypsin deficiency (AATD) and of cystic fibrosis (CF).

9. Medicament comprising a compound as defined in any of Claims 1 to 4 in combination with one or more inert non-toxic pharmaceutically acceptable auxiliaries.

10. Medicament comprising a compound as defined in any of Claims 1 to 4 in combination with one or more further active compounds selected from the group of the kinase inhibitors, stimulators and activators of soluble guanylate cyclase, prostacyclin analogs, endothelin receptor antagonists, phosphodiesterase inhibitors, beta-adrenergic receptor agonists, matrix metalloprotease inhibitors, serotonin antagonists, anticholinergics and glucocorticoids.

11. Medicament according to Claim 9 or 10 for the treatment and/or prevention of pulmonary arterial hypertension (PAH) and other forms of pulmonary hypertension (PH), or chronic-obstructive pulmonary diseases (COPD), of acute lung injury (ALI), of acute respiratory distress syndrome (ARDS), of pulmonary emphysema, of alpha-1 antitrypsin deficiency (AATD) and of cystic fibrosis (CF).

12. Compound or medicament for use in a method for the treatment and/or prevention of pulmonary arterial hypertension (PAH) and other forms of pulmonary hypertension (PH), of chronic-obstructive pulmonary diseases (COPD), of acute lung injury (ALI), of acute respiratory distress syndrome (ARDS), of pulmonary emphysema, of alpha-1 antitrypsin deficiency (AATD) and of cystic fibrosis (CF) in humans and animals using an effective amount of at least one compound as defined in any of Claims 1 to 4 or a medicament as defined in any of Claims 9 to 11.

## Revendications

1. Composé de formule (I) 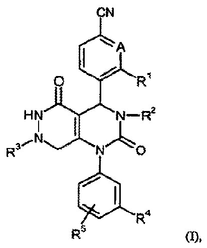 dans laquelle
A représente CH ou N,
R¹ représente hydrogène, halogène, cyano, nitro, (C₁-C₆)-alkyle, difluorométhyle, trifluorométhyle, (C₁-C₆) -alcoxy, difluorométhoxy, trifluorométhoxy, amino, mono-(C₁-C₆)-alkylamino ou di- (C₁-C₆)-alkylamino ou représente un groupe de formule -NH-C(=O)-R⁶, -NH-C(=O)-NHR⁶, -NH-SO₂-R⁷ ou -S(O)ₙ-R⁸, dans laquelle
R⁶ signifie hydrogène ou (C₁-C₆)-alkyle,
R⁷ signifie (C₁-C₆)-alkyle,
R⁸ signifie (C₁-C₆)-alkyle, qui peut être substitué par hydroxy, (C₁-C₄)-alcoxy, amino, mono-(C₁-C₄)-alkylamino ou di-(C₁-C₄)-alkylamino, hydroxycarbonyle, aminocarbonyle, (C₃-C₆)-cycloalkyle ou phényle ; ou signifie (C₂-C₆) -alcényle, (C₃-C₆)-cycloalkyle ou phényle,
les groupes (C₃-C₆)-cycloalkyle mentionnés pouvant être jusqu'à disubstitués, de manière identique ou différente, par (C₁-C₄)-alkyle, hydroxy et (C₁-C₄)-alcoxy et
les groupes phényle mentionnés pouvant être jusqu'à disubstitués, de manière identique ou différente, par fluor, chlore, cyano, (C₁-C₄)-alkyle, difluorométhyle, trifluorométhyle, (C₁-C₄)-alcoxy, difluorométhoxy et trifluorométhoxy, et
n vaut le nombre 0, 1 ou 2,
R² représente hydrogène, (C₁-C₆)-alkyle ou (C₂-C₆)-alcényle, qui peuvent être jusqu'à trisubstitués par fluor ; ou représente phényle, pyridyle ou pyrimidinyle,
phényle, pyridyle et pyrimidinyle pouvant à leur tour être jusqu'à disubstitués, de manière identique ou différente, par fluor, chlore, cyano, (C₁-C₄)-alkyle, trifluorométhyle, (C₁-C₄)-alcoxy et trifluorométhoxy, ou
R² représente un groupe de formule -C(=O)-O-R⁹, -L¹-C(=O)-O-R¹⁰, -L²-C(=O)-NR¹¹R¹², -L²-SO₂-NR¹¹-R¹², -L²-C(=O)-NR¹³-NR¹¹R¹² ou -L²-SO₂-R¹⁴, où
L¹ signifie (C₁-C₆)-alcanediyle,
L² signifie une liaison ou (C₁-C₆)-alcanediyle,
R⁹ signifie (C₁-C₆) -alkyle,
R¹⁰ signifie hydrogène ou (C₁-C₆) -alkyle,
R¹¹ et R¹² sont identiques ou différents et représentent, indépendamment l'un de l'autre, hydrogène, (C₁-C₆)-alkyle, (C₃-C₆)-cycloalkyle ou hétérocyclyle de 4 ou 6 chaînons, (C₁-C₆)-alkyle, (C₃-C₆)-cycloalkyle et hétérocyclyle de 4 à 6 chaînons pouvant à leur tour être jusqu'à disubstitués, de manière identique ou différente, par fluor, Hydroxy, (C₁-C₄)-alcoxy, oxo, amino, mono-(C₁-C₄)-alkylamino ou di-(C₁-C₄)-alkylamino, hydroxycarbonyle, (C₁-C₄)-alcoxycarbonyle et aminocarbonyle et, dans (C₁-C₆)-alkyle, un groupe CH₂ pouvant être remplacé par un atome O pour autant qu'on obtienne un composé chimiquement stable, ou
R¹¹ et R¹² forment ensemble avec l'atome d'azote auquel ils sont liés, un hétérocycle de 4 à 6 chaînons, qui peut contenir un autre hétéroatome de cycle de la série N, O, S, SO ou SO₂ et qui peut être jusqu'à disubstitué, de manière identique ou différente, par (C₁-C₄)-alkyle, hydroxy, (C₁-C₄)-alcoxy, oxo, amino, mono-(C₁-C₄)-alkylamino et di-(C₁-C₄)-alkylamino,
(C₁-C₄)-alkyle pouvant à son tour être substitué par hydroxy ou (C₂-C₄)-alcoxy,
R¹³ signifie hydrogène ou (C₁-C₄)-alkyle, et
R¹⁴ signifie (C₁-C₆) -alkyle, (C₃-C₆) -cycloalkyle, phényle ou hétéroaryle de 5 ou 6 chaînons, (C₁-C₆) -alkyle pouvant être substitué par fluor, chlore, hydroxy, (C₁-C₄)-alcoxy, mono-(C₁-C₄) -alkylamino ou di- (C₁-C₄) -alkylamino et phényle et hétéroaryle de 5 ou 6 chaînons pouvant à leur tour être jusqu'à disubstitués, de manière identique ou différente, par fluor, chlore, cyano, (C₁-C₄)-alkyle, trifluorométhyle, (C₁-C₄)-alcoxy et trifluorométhoxy,
R³ représente (C₁-C₆)-alkyle ou (C₂-C₆)-alcényle, qui peuvent à chaque fois être substitués par hydroxy, (C₁-C₄)-alcoxy, amino, mono-(C₁-C₄)-alkylamino ou di-(C₁-C₄)-alkylamino, hydroxycarbonyle, (C₁-C₄)-alcoxycarbonyle, aminocarbonyle ou mono-(C₁-C₄)-alkylaminocarbonyle ou di-(C₁-C₄)-alkylaminocarbonyle, ou
représente un groupe de formule -L³-R¹⁵, où
L³ signifie une liaison ou (C₁-C₄)-alcanediyle et
R¹⁵ signifie (C₃-C₇)-cycloalkyle, hétérocyclyle de 4 à 7 chaînons, phényle ou hétéroaryle de 5 ou 6 chaînons,
(C₃-C₇) -cycloalkyle et hétérocyclyle de 4 à 7 chaînons pouvant à leur tour être jusqu'à disubstitués, de manière identique ou différente, par (C₁-C₄) -alkyle, oxo, hydroxy et (C₁-C₄) -alcoxy et
phényle et hétéroaryle de 5 ou 6 chaînons pouvant à leur tour être jusqu'à disubstitués, de manière identique ou différente, par fluor, chlore, cyano, (C₁-C₄)-alkyle, trifluorométhyle, (C₁-C₄)-alcoxy, trifluorométhoxy et amino,
R⁴ représente nitro ou trifluorométhyle et
R⁵ représente hydrogène, fluor ou chlore,
ainsi que ses sels, solvates et les solvates des sels.

2. Composé de formule (I) selon la revendication 1, dans laquelle
A représente CH,
R¹ représente hydrogène, fluor, chlore, cyano, nitro, (C₁-C₄)-alkyle, difluorométhyle, trifluorométhyle, (C₁-C₄)-alcoxy, difluorométhoxy, trifluorométhoxy, amino, mono- (C₁-C₄) -alkylamino ou di-(C₁-C₄)-alkylamino ou
représente un groupe de formule -NH-C(=O)-R⁶, -NH-SO₂-R⁷ ou -SO₂-R⁸, dans laquelle R⁶ et R⁷ signifient à chaque fois (C₁-C₄)-alkyle et
R⁸ signifie (C₁-C₄)-alkyle, qui peut être substitué par hydroxy, (C₁-C₄)-alcoxy, hydroxycarbonyle, aminocarbonyle, (C₃-C₆)-cycloalkyle ou phényle ; ou signifie (C₃-C₆)-cycloalkyle ou phényle,
les groupes phényle mentionnés pouvant être jusqu'à disubstitués, de manière identique ou différente, par fluor, chlore, cyano, méthyle, trifluorométhyle, méthoxy et trifluorométhoxy,
R² signifie hydrogène, (C₁-C₄)-alkyle ou (C₂-C₄)-alcényle ou
représente un groupe de formule -L¹-C (=O) -O-R¹⁰, -L²-C(=O)-NR¹¹R¹² et -L²-SO₂-R¹⁴, où
L¹ signifie méthylène ou éthane-1,2-diyle,
L² signifie une liaison, méthylène, éthane-1,1-diyle ou éthane-1,2-diyle,
R¹⁰ signifie hydrogène ou (C₁-C₄) -alkyle,
R¹¹ signifie hydrogène ou (C₁-C₄)-alkyle, qui peut être substitué par hydroxy ou (C₁-C₄)-alcoxy,
R¹² signifie hydrogène, (C₁-C₆)-alkyle ou (C₃-C₆)-cycloalkyle,
(C₁-C₆)-alkyle pouvant à son tour être jusqu'à disubstitué, de manière identique ou différente, par hydroxy, (C₁-C₄)-alcoxy, hydroxycaxbonyle, (C₁-C₄)-alcoxycarbonyle et aminocarbonyle et, dans (C₁-C₆)-alkyle, un groupe CH₂ pouvant être remplacé par un atome O pour autant qu'on obtienne un composé chimiquement stable, ou
R¹¹ R¹² et ensemble avec l'atome d'azote auquel ils sont liés, forment un hétérocycle de 5 ou 6 chaînons, qui peut contenir un autre hétéroatome de cycle de la série N, 0 ou S et qui peut être substitué par (C₁-C₄)-alkyle, hydroxy, (C₁-C₄)-alcoxy ou oxo,
(C₁-C₄)-alkyle pouvant à son tour être substitué par hydroxy ou (C₃-C₄)-alcoxy, et
R¹⁴ signifie (C₁-C₄)-alkyle, (C₃-C₆)-cycloalkyle ou phényle,
phényle pouvant être jusqu'à disubstitué, de manière identique ou différente, par fluor, chlore, cyano, méthyle, trifluorométhyle, méthoxy et trifluorométhoxy,
R³ représente (C₁-C₄)-alkyle, qui peut être substitué par hydroxy, (C₁-C₄)-alcoxy, hydroxycarbonyle, (C₁-C₄)-alcoxycarbonyle, aminocarbonyle, mono-(C₁-C₄)-alkylaminocarbonyle ou di-(C₁-C₄)-alkylaminocarbonyle ou hétéroaryle de 5 ou 6 chaînons ; représente (C₂-C₄)-alcényle ou représente un groupe de formule -L³-R¹⁵, où
L³ signifie une liaison ou (C₁-C₄)-alcanediyle et
R¹⁵ signifie (C₃-C₇)-cycloalkyle, hétérocyclyle de 4 à 6 chaînons ou phényle,
hétérocyclyle de 4 à 6 chaînons pouvant à son tour être substitué par oxo et phényle pouvant à son tour être jusqu'à disubstitué, de manière identique ou différente, par fluor, chlore, cyano, méthyle, trifluorométhyle, méthoxy et trifluorométhoxy,
R⁴ représente trifluorométhyle et
R⁵ représente hydrogène ou fluor,
ainsi que ses sels, solvates et les solvates des sels.

3. Composé de formule (I) selon la revendication 1 ou 2, dans laquelle
A représente CH,
R¹ représente hydrogène, fluor, chlore, nitro, méthyle, difluorométhyle, trifluorométhyle, méthoxy, trifluorométhoxy ou un groupe de formule -SO₂-R⁸, dans laquelle
R⁸ signifie (C₁-C₄)-alkyle, qui peut être substitué par hydroxy, méthoxy ou éthoxy,
R² représente hydrogène, (C₁-C₄)-alkyle ou un groupe de formule -CH₂-C(=O)-O-R¹⁰ ou -CH₂-C(=O)-NR¹¹R¹², dans laquelle
R¹⁰ signifie (C₁-C₄) -alkyle;
R¹¹ signifie hydrogène ou méthyle,
R¹² signifie hydrogène ou (C₁-C₄)-alkyle, qui peut être substitué par hydroxy, méthoxy ou éthoxy, ou
R¹¹ et R¹² forment ensemble, avec l'atome d'azote auquel ils sont liés, un cycle pyrrolidino, pipéridino ou morpholino,
R³ représente (C₁-C₄)-alkyle, qui peut être substitué par hydroxy, pyrrolidino, pipéridino, morpholino ou pyridyle ; représente allyle ou un groupe de formule -L³-R¹⁵, dans laquelle
L³ signifie une liaison, méthylène ou éthane-1,2-diyle et
R¹⁵ signifie (C₃-C₇) -cycloalkyle ou phényle, phényle pouvant à son tour être jusqu'à disubstitué, de manière identique ou différente, par fluor, chlore, cyano, méthyle ou trifluorométhyle,
R⁴ représente trifluorométhyle et
R⁵ représente hydrogène,
ainsi que ses sels, solvates et les solvates des sels.

4. Composé de formule (I) selon la revendication 1, 2 ou 3, dans laquelle
A représente CH,
R¹ représente hydrogène, trifluorométhyle ou méthylsulfonyle,
R² représente hydrogène ou un groupe de formule -CH₂-C(=O) -NR¹¹R¹², dans laquelle
R¹¹ et R¹² signifient, indépendamment l'un de l'autre hydrogène ou méthyle, ou
R¹¹ et R¹² forment ensemble avec l'atome d'azote auquel ils sont liés un cycle pyrrolidino,
R³ représente méthyle, éthyle, 2-hydroxyéthyle ou 2-(morpholin-4-yl)éthyle,
R⁴ représente trifluorométhyle et
R⁵ représente hydrogène,
ainsi que ses sels, solvates et les solvates des sels.

5. Procédé pour la préparation de composés de formule (I) tels que définis dans les revendications 1 à 4, **caractérisé en ce qu'**on condense d'abord un composé de formule (II) 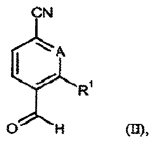 dans laquelle A et R¹ présentent les significations indiquées dans les revendications 1 à 4,
en présence d'un acide ou d'un anhydride d'acide dans une réaction dans un pot à 3 composants ou séquentiellement avec un ester de l'acide acétoacétique de formule (III) 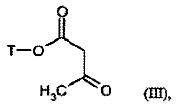 dans laquelle
T représente méthyle ou éthyle,
et d'un dérivé de phénylurée de formule (IV) 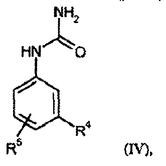 dans laquelle R⁴ et R⁵ présentent les significations indiquées dans les revendications 1 à 4 en un composé de formule (V-A) 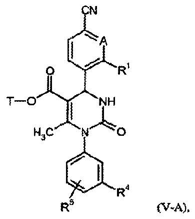 dans laquelle A, T, R¹, R⁴ et R⁵ présentent à chaque fois les significations indiquées ci-dessus, puis
[A] dans le cas où R² dans la formule (I) représente hydrogène, on le brome dans un solvant inerte en un composé de formule (VI-A) 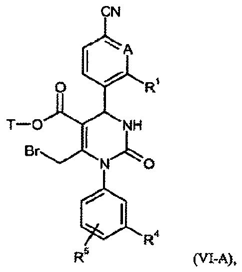 dans laquelle A, T, R¹, R⁴ et R⁵ présentent à chaque fois les significations indiquées ci-dessus, puis on le transforme avec un dérivé d'hydrazine de formule (VII)
R³-NH-NH₂ (VII),
dans laquelle R³ présente la signification indiquée dans les revendications 1 à 4,
en formant un cycle hexagonal, en un composé de formule (I-A) 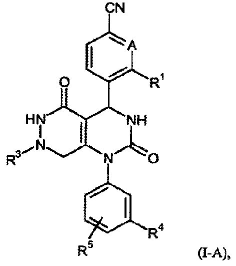 dans laquelle A, R¹, R³, R⁴ et R⁵ présentent à chaque fois les significations indiquées ci-dessus, où
[B] dans le cas où R² dans la formule (I) est différent d'hydrogène, on le fait d'abord réagir avec un composé de formule (VIII)
R^{2A}-X (VIII),
dans laquelle
R^{2A} présente la signification de R² indiquée dans les revendications 1 à 4, mais ne représente pas hydrogène, et
X représente un groupe partant tel que par exemple halogène, mésylate, tosylate ou triflate,
en présence d'une base en un composé de formule (V-B) 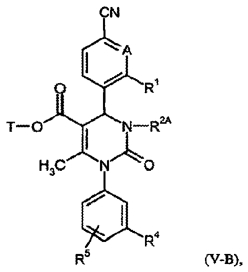 dans laquelle A, T, R¹, R^{2A}, R⁴ et R⁵ présentent à chaque fois les significations indiquées ci-dessus,
puis on le brome dans un solvant inerte en un composé de formule (VI-B) 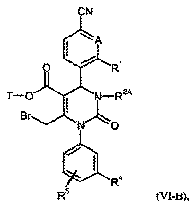 dans laquelle A, T, R¹, R², R⁴ et R⁵ présentent à chaque fois les significations indiquées ci-dessus,
puis on le transforme avec un dérivé d'hydrazine de formule (VII)
R³-NH-NH₂ (VII),
dans laquelle R³ présente la signification indiquée dans les revendications 1 à 4, en cyclisant en un composé de formule (I-B) 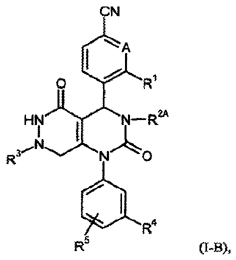 dans laquelle A, R¹, R^{2A}, R³, R⁴ et R⁵ présentent à chaque fois les significations indiquées ci-dessus,
et on sépare le cas échéant les composés de formule (I-A) ou, selon le cas, (I-B) ainsi obtenus selon des procédés connus par l'homme du métier en leurs énantiomères et/ou diastéréo-isomères et/ou on les transforme avec (i) des solvants et/ou (ii) des bases ou acides correspondants en leurs solvates, sels et/ou solvates des sels.

6. Composé, tel que défini dans l'une quelconque des revendications 1 à 4, destiné au traitement et/ou à la prévention de maladies.

7. Composé, tel que défini dans l'une quelconque des revendications 1 à 4, destiné à une utilisation dans un procédé pour le traitement et/ou la prévention de l'hypertonie artérielle pulmonaire (HAP) et d'autres formes de l'hypertonie pulmonaire (HP), des maladies pulmonaires obstructives chroniques (MPOC), du syndrome respiratoire aigu (ALI - acute lung injury), du syndrome de détresse respiratoire aiguë (ARDS), de l'emphysème pulmonaire, de la déficience en alpha-1-antitrypsine (AATD) et de la fibrose kystique (CF).

8. Utilisation d'un composé, tel que défini dans l'une quelconque des revendications 1 à 4, pour la préparation d'un médicament destiné au traitement et/ou à la prévention de l'hypertonie artérielle pulmonaire (HAP) et d'autres formes de l'hypertonie pulmonaire (HP), des maladies pulmonaires obstructives chroniques (MPOC), du syndrome respiratoire aigu (ALI - acute lung injury), du syndrome de détresse respiratoire aiguë (ARDS), de l'emphysème pulmonaire, de la déficience en alpha-1-antitrypsine (AATD) et de la fibrose kystique (CF) .

9. Médicament contenant un composé, tel que défini dans l'une quelconque des revendications 1 à 4, en combinaison avec un ou plusieurs adjuvants inertes, non toxiques, pharmaceutiquement appropriés.

10. Médicament contenant un composé tel que défini dans l'une quelconque des revendications 1 à 4 en combinaison avec une ou plusieurs autres substances actives choisies dans le groupe constitué par les inhibiteurs de kinase, les stimulateurs et les activateurs de la guanylate cyclase soluble, les analogues de prostacycline, les antagonistes du récepteur de l'endothéline, les inhibiteurs de la phosphodiestérase, les agonistes du récepteur bêta-adrénergique, les inhibiteurs des métalloprotéases de matrice, les antagonistes du récepteur de la sérotonine, les anticholinergiques et les glucocorticoïdes.

11. Médicament selon la revendication 9 ou 10 destiné au traitement et/ou à la prévention de l'hypertonie artérielle pulmonaire (HAP) et d'autres formes de l'hypertonie pulmonaire (HP), des maladies pulmonaires obstructives chroniques (MPOC), du syndrome respiratoire aigu (ALI acute lung injury), du syndrome de détresse respiratoire aiguë (ARDS), de l'emphysème pulmonaire, de la déficience en alpha-1-antitrypsine (AATD) et de la fibrose kystique (CF).

12. Composé ou médicament destiné à être utilisé dans un procédé pour le traitement et/ou la prévention de l'hypertonie artérielle pulmonaire (HAP) et d'autres formes de l'hypertonie pulmonaire (HP), des maladies pulmonaires obstructives chroniques (MPOC), du syndrome respiratoire aigu (ALI - acute lung injury), du syndrome de détresse respiratoire aiguë (ARDS), de l'emphysème pulmonaire, de la déficience en alpha-1-antitrypsine (AATD) et de la fibrose kystique (CF) chez les hommes et les animaux, en utilisant une quantité active d'au moins un composé, tel que défini dans l'une quelconque des revendications 1 à 4, ou d'un médicament tel que défini dans l'une quelconque des revendications 9 à 11.
